# EUROPEAN PATENT APPLICATION

(11) **EP 3 521 828 A1**
(43) Date of publication of application: **07.08.2019**
(21) Application number: 18154450.3
(22) Date of filing: 31.01.2018
(51) Int. Cl.: G01N 33/68

(54) **METHOD FOR THE DIAGNOSIS OF HEREDITARY ANGIOEDEMA**

(71) Applicant: Centogene AG, 18055 Rostock (DE)
(72) Inventor: COZMA, Claudia, 18059 Rostock (DE)
(74) Representative: Bohmann, Armin K.

(57) **Abstract**

The present invention is related to a method for differential diagnosis of hereditary angioedema, wherein the method comprises determining the level of C4 protein, C1-INH protein and Clq protein in a sample from a subject, wherein the sample is a dried blood spot sample and wherein the level is determined by mass spectrometry.

SEQUENCE LISTING

<110>  Centogene AG

<120>  Method for the diagnosis of hereditary angioedema

<130>  C 10076 EP

<140>  EP 18 154 450.3
<141>  2018-01-31

<160>  290

<170>  PatentIn version 3.5

<210>  1
<211>  245
<212>  PRT
<213>  Homo sapiens

<220>
<221>  MISC_FEATURE
<223>  Chain A of C1q

<400>  1

<210>  2
<211>  253
<212>  PRT
<213>  Homo sapiens

<220>
<221>  MISC_FEATURE
<223>  Chain B of C1q

<400>  2

Met Met Met Lys Ile Pro Trp Gly Ser Ile Pro Val Leu Met Leu Leu
1               5                   10                  15

Leu Leu Leu Gly Leu Ile Asp Ile Ser Gln Ala Gln Leu Ser Cys Thr
            20                  25                  30

Gly Pro Pro Ala Ile Pro Gly Ile Pro Gly Ile Pro Gly Thr Pro Gly
        35                  40                  45

Pro Asp Gly Gln Pro Gly Thr Pro Gly Ile Lys Gly Glu Lys Gly Leu
    50                  55                  60

Pro Gly Leu Ala Gly Asp His Gly Glu Phe Gly Glu Lys Gly Asp Pro
65                  70                  75                  80

Gly Ile Pro Gly Asn Pro Gly Lys Val Gly Pro Lys Gly Pro Met Gly
                85                  90                  95

Pro Lys Gly Gly Pro Gly Ala Pro Gly Ala Pro Gly Pro Lys Gly Glu
            100                 105                 110

Ser Gly Asp Tyr Lys Ala Thr Gln Lys Ile Ala Phe Ser Ala Thr Arg
        115                 120                 125

Thr Ile Asn Val Pro Leu Arg Arg Asp Gln Thr Ile Arg Phe Asp His
    130                 135                 140

Val Ile Thr Asn Met Asn Asn Asn Tyr Glu Pro Arg Ser Gly Lys Phe
145                 150                 155                 160

Thr Cys Lys Val Pro Gly Leu Tyr Tyr Phe Thr Tyr His Ala Ser Ser
                165                 170                 175

Arg Gly Asn Leu Cys Val Asn Leu Met Arg Gly Arg Glu Arg Ala Gln
            180                 185                 190

Lys Val Val Thr Phe Cys Asp Tyr Ala Tyr Asn Thr Phe Gln Val Thr
        195                 200                 205

Thr Gly Gly Met Val Leu Lys Leu Glu Gln Gly Glu Asn Val Phe Leu
    210                 215                 220

Gln Ala Thr Asp Lys Asn Ser Leu Leu Gly Met Glu Gly Ala Asn Ser
225                 230                 235                 240

Ile Phe Ser Gly Phe Leu Leu Phe Pro Asp Met Glu Ala
                245                 250

<210> 3
<211> 245
<212> PRT
<213> Homo sapiens

<220>
<221> MISC_FEATURE
<223> chain C of C1q

<220>
<221> MISC_FEATURE
<223> Chain C of C1q

<400> 3

<210>  4
<211>  7
<212>  PRT
<213>  Homo sapiens

<220>
<221>  MISC_FEATURE
<223>  synthetic

<400>  4

Gly Ser Pro Gly Asn Ile Lys
1               5


<210>  5
<211>  11
<212>  PRT
<213>  Homo sapiens


<220>
<221>  MISC_FEATURE
<223>  synthetic

<400>  5

Asp Gln Pro Arg Pro Ala Phe Ser Ala Ile Arg
1               5                   10


<210>  6
<211>  28
<212>  PRT
<213>  Homo sapiens


<220>
<221>  MISC_FEATURE
<223>  synthetic

<400>  6

Asn Pro Pro Met Gly Gly Asn Val Val Ile Phe Asp Thr Val Ile Thr
1               5                   10                  15


Asn Gln Glu Glu Pro Tyr Gln Asn His Ser Gly Arg
            20                  25


<210>  7
<211>  30
<212>  PRT
<213>  Homo sapiens


<220>
<221>  MISC_FEATURE
<223>  synthetic

<400>  7

Phe Val Cys Thr Val Pro Gly Tyr Tyr Tyr Phe Thr Phe Gln Val Leu
1               5                   10                  15

Ser Gln Trp Glu Ile Cys Leu Ser Ile Val Ser Ser Ser Arg
            20                  25                  30

<210>  8
<211>  30
<212>  PRT
<213>  Homo sapiens

<220>
<221>  MISC_FEATURE
<223>  synthetic

<400>  8

Phe Val Cys Thr Val Pro Gly Tyr Tyr Tyr Phe Thr Phe Gln Val Leu
1               5                   10                  15

Ser Gln Trp Glu Ile Cys Leu Ser Ile Val Ser Ser Ser Arg
            20                  25                  30

<210>  9
<211>  10
<212>  PRT
<213>  Homo sapiens

<220>
<221>  MISC_FEATURE
<223>  synthetic

<400>  9

Ser Leu Gly Phe Cys Asp Thr Thr Asn Lys
1               5                   10

<210>  10
<211>  10
<212>  PRT
<213>  Homo sapiens

<220>
<221>  MISC_FEATURE
<223>  synthetic

<400>  10

Ser Leu Gly Phe Cys Asp Thr Thr Asn Lys
1               5                   10

<210>  11
<211>  24
<212>  PRT
<213>  Homo sapiens

<220>

<221>  MISC_FEATURE
<223>  synthetic

<400>  11

Gly Leu Phe Gln Val Val Ser Gly Gly Met Val Leu Gln Leu Gln Gln
1               5                   10                  15

Gly Asp Gln Val Trp Val Glu Lys
            20

<210>  12
<211>  22
<212>  PRT
<213>  Homo sapiens

<220>
<221>  MISC_FEATURE
<223>  synthetic

<400>  12

Gly His Ile Tyr Gln Gly Ser Glu Ala Asp Ser Val Phe Ser Gly Phe
1               5                   10                  15

Leu Ile Phe Pro Ser Ala
            20

<210>  13
<211>  5
<212>  PRT
<213>  Homo sapiens

<220>
<221>  MISC_FEATURE
<223>  synthetic

<400>  13

Glu Asp Leu Cys Arg
1               5

<210>  14
<211>  5
<212>  PRT
<213>  Homo sapiens

<220>
<221>  MISC_FEATURE
<223>  synthetic

<400>  14

Ala Pro Asp Gly Lys
1               5

<210>  15
<211>  8
<212>  PRT
<213>  Homo sapiens

<220>
<221>  MISC_FEATURE
<223>  synthetic

<400>  15

Gly Glu Ala Gly Arg Pro Gly Arg
1               5

<210>  16
<211>  12
<212>  PRT
<213>  Homo sapiens

<220>
<221>  MISC_FEATURE
<223>  synthetic

<400>  16

Gly Glu Gln Gly Glu Pro Gly Ala Pro Gly Ile Arg
1               5                   10

<210>  17
<211>  13
<212>  PRT
<213>  Homo sapiens

<220>
<221>  MISC_FEATURE
<223>  synthetic

<400>  17

Val Gly Tyr Pro Gly Pro Ser Gly Pro Leu Gly Ala Arg
1               5                   10

<210>  18
<211>  4
<212>  PRT
<213>  Homo sapiens

<220>
<221>  MISC_FEATURE
<223>  synthetic

<400>  18

Ala Thr Gln Lys

1

<210>  19
<211>  5
<212>  PRT
<213>  Homo sapiens

<220>
<221>  MISC_FEATURE
<223>  synthetic

<400>  19

Asp Gln Thr Ile Arg
1               5

<210>  20
<211>  4
<212>  PRT
<213>  Homo sapiens

<220>
<221>  MISC_FEATURE
<223>  synthetic

<400>  20

Phe Thr Cys Lys
1

<210>  21
<211>  14
<212>  PRT
<213>  Homo sapiens

<220>
<221>  MISC_FEATURE
<223>  synthetic

<400>  21

Val Pro Gly Leu Tyr Tyr Phe Thr Tyr His Ala Ser Ser Arg
1               5                   10

<210>  22
<211>  9
<212>  PRT
<213>  Homo sapiens

<220>
<221>  MISC_FEATURE
<223>  synthetic

<400>  22

Gly Asn Leu Cys Val Asn Leu Met Arg
1               5

<210>  23
<211>  9
<212>  PRT
<213>  Homo sapiens

<220>
<221>  MISC_FEATURE
<223>  synthetic

<400>  23

Gly Asn Leu Cys Val Asn Leu Met Arg
1               5

<210>  24
<211>  22
<212>  PRT
<213>  Homo sapiens

<220>
<221>  MISC_FEATURE
<223>  synthetic

<400>  24

Val Val Thr Phe Cys Asp Tyr Ala Tyr Asn Thr Phe Gln Val Thr Thr
1               5                   10                  15

Gly Gly Met Val Leu Lys
            20

<210>  25
<211>  22
<212>  PRT
<213>  Homo sapiens

<220>
<221>  MISC_FEATURE
<223>  synthetic

<400>  25

Val Val Thr Phe Cys Asp Tyr Ala Tyr Asn Thr Phe Gln Val Thr Thr
1               5                   10                  15

Gly Gly Met Val Leu Lys
            20

<210>  26
<211>  14
<212>  PRT

<213>  Homo sapiens

<220>
<221>  MISC_FEATURE
<223>  synthetic

<400>  26

Leu Glu Gln Gly Glu Asn Val Phe Leu Gln Ala Thr Asp Lys
1               5                   10

<210>  27
<211>  24
<212>  PRT
<213>  Homo sapiens

<220>
<221>  MISC_FEATURE
<223>  synthetic

<400>  27

Asn Ser Leu Leu Gly Met Glu Gly Ala Asn Ser Ile Phe Ser Gly Phe
1               5                   10                  15

Leu Leu Phe Pro Asp Met Glu Ala
            20

<210>  28
<211>  32
<212>  PRT
<213>  Homo sapiens

<220>
<221>  MISC_FEATURE
<223>  synthetic

<400>  28

Gln Leu Ser Cys Thr Gly Pro Pro Ala Ile Pro Gly Ile Pro Gly Ile
1               5                   10                  15

Pro Gly Thr Pro Gly Pro Asp Gly Gln Pro Gly Thr Pro Gly Ile Lys
            20                  25                  30

<210>  29
<211>  32
<212>  PRT
<213>  Homo sapiens

<220>
<221>  MISC_FEATURE
<223>  synthetic

<400>  29

Gln Leu Ser Cys Thr Gly Pro Pro Ala Ile Pro Gly Ile Pro Gly Ile
1               5                   10                  15

Pro Gly Thr Pro Gly Pro Asp Gly Gln Pro Gly Thr Pro Gly Ile Lys
            20                  25                  30

<210>  30
<211>  15
<212>  PRT
<213>  Homo sapiens

<220>
<221>  MISC_FEATURE
<223>  synthetic

<400>  30

Gly Leu Pro Gly Leu Ala Gly Asp His Gly Glu Phe Gly Glu Lys
1               5                   10                  15

<210>  31
<211>  11
<212>  PRT
<213>  Homo sapiens

<220>
<221>  MISC_FEATURE
<223>  synthetic

<400>  31

Gly Asp Pro Gly Ile Pro Gly Asn Pro Gly Lys
1               5                   10

<210>  32
<211>  6
<212>  PRT
<213>  Homo sapiens

<220>
<221>  MISC_FEATURE
<223>  synthetic

<400>  32

Gly Pro Met Gly Pro Lys
1               5

<210>  33
<211>  12
<212>  PRT
<213>  Homo sapiens

<220>
<221>  MISC_FEATURE
<223>  synthetic

<400>  33

Gly Gly Pro Gly Ala Pro Gly Ala Pro Gly Pro Lys
1               5                   10


<210>  34
<211>  9
<212>  PRT
<213>  Homo sapiens


<220>
<221>  MISC_FEATURE
<223>  synthetic

<400>  34

Phe Gln Ser Val Phe Thr Val Thr Arg
1               5


<210>  35
<211>  13
<212>  PRT
<213>  Homo sapiens


<220>
<221>  MISC_FEATURE
<223>  synthetic

<400>  35

Gln Thr His Gln Pro Pro Ala Pro Asn Ser Leu Ile Arg
1               5                   10


<210>  36
<211>  18
<212>  PRT
<213>  Homo sapiens


<220>
<221>  MISC_FEATURE
<223>  synthetic

<400>  36

Phe Asn Ala Val Leu Thr Asn Pro Gln Gly Asp Tyr Asp Thr Ser Thr
1               5                   10                  15


Gly Lys

<210>  37
<211>  23
<212>  PRT
<213>  Homo sapiens

<220>
<221>  MISC_FEATURE
<223>  synthetic

<400>  37

Val Pro Gly Leu Tyr Tyr Phe Val Tyr His Ala Ser His Thr Ala Asn
1               5                   10                  15

Leu Cys Val Leu Leu Tyr Arg
            20

<210>  38
<211>  23
<212>  PRT
<213>  Homo sapiens

<220>
<221>  MISC_FEATURE
<223>  synthetic

<400>  38

Val Pro Gly Leu Tyr Tyr Phe Val Tyr His Ala Ser His Thr Ala Asn
1               5                   10                  15

Leu Cys Val Leu Leu Tyr Arg
            20

<210>  39
<211>  10
<212>  PRT
<213>  Homo sapiens

<220>
<221>  MISC_FEATURE
<223>  synthetic

<400>  39

Val Val Thr Phe Cys Gly His Thr Ser Lys
1               5                   10

<210>  40
<211>  10
<212>  PRT
<213>  Homo sapiens

<220>

<221>  MISC_FEATURE
<223>  synthetic

<400>  40

Val Val Thr Phe Cys Gly His Thr Ser Lys
1               5                   10


<210>  41
<211>  12
<212>  PRT
<213>  Homo sapiens


<220>
<221>  MISC_FEATURE
<223>  synthetic

<400>  41

Thr Asn Gln Val Asn Ser Gly Gly Val Leu Leu Arg
1               5                   10


<210>  42
<211>  35
<212>  PRT
<213>  Homo sapiens


<220>
<221>  MISC_FEATURE
<223>  syynthetic

<400>  42

Leu Gln Val Gly Glu Glu Val Trp Leu Ala Val Asn Asp Tyr Tyr Asp
1               5                   10                  15


Met Val Gly Ile Gln Gly Ser Asp Ser Val Phe Ser Gly Phe Leu Leu
            20                  25                  30


Phe Pro Asp
        35


<210>  43
<211>  19
<212>  PRT
<213>  Homo sapiens


<220>
<221>  MISC_FEATURE
<223>  synthetic

<400>  43

Asn Thr Gly Cys Tyr Gly Ile Pro Gly Met Pro Gly Leu Pro Gly Ala
1               5                   10                  15

Pro Gly Lys

<210> 44
<211> 19
<212> PRT
<213> Homo sapiens

<220>
<221> MISC_FEATURE
<223> synthetic

<400> 44

Asn Thr Gly Cys Tyr Gly Ile Pro Gly Met Pro Gly Leu Pro Gly Ala
1               5                   10                  15

Pro Gly Lys

<210> 45
<211> 10
<212> PRT
<213> Homo sapiens

<220>
<221> MISC_FEATURE
<223> synthetic

<400> 45

Asp Gly Tyr Asp Gly Leu Pro Gly Pro Lys
1               5                   10

<210> 46
<211> 12
<212> PRT
<213> Homo sapiens

<220>
<221> MISC_FEATURE
<223> synthetic

<400> 46

Gly Glu Pro Gly Ile Pro Ala Ile Pro Gly Ile Arg
1               5                   10

<210> 47
<211> 11
<212> PRT
<213> Homo sapiens

<220>
<221>  MISC_FEATURE
<223>  synthetic

<400>  47

Gly Glu Pro Gly Leu Pro Gly His Pro Gly Lys
1               5                   10


<210>  48
<211>  27
<212>  PRT
<213>  Homo sapiens


<220>
<221>  MISC_FEATURE
<223>  synthetic

<400>  48

Asn Gly Pro Met Gly Pro Pro Gly Met Pro Gly Val Pro Gly Pro Met
1               5                   10                  15


Gly Ile Pro Gly Glu Pro Gly Glu Glu Gly Arg
            20                  25


<210>  49
<211>  500
<212>  PRT
<213>  Homo sapiens


<220>
<221>  MISC_FEATURE
<223>  synthetic

<400>  49

<210>  50
<211>  10
<212>  PRT
<213>  Homo sapiens

<220>
<221>  MISC_FEATURE
<223>  synthetic

<400>  50

Asp Phe Thr Cys Val His Gln Ala Leu Lys
1               5                   10

<210>  51
<211>  10

<212>  PRT
<213>  Homo sapiens



<220>
<221>  MISC_FEATURE
<223>  synthetic

<400>  51

Asp Phe Thr Cys Val His Gln Ala Leu Lys
1               5                   10



<210>  52
<211>  5
<212>  PRT
<213>  Homo sapiens



<220>
<221>  MISC_FEATURE
<223>  synthetic

<400>  52

Gly Phe Thr Thr Lys
1               5



<210>  53
<211>  17
<212>  PRT
<213>  Homo sapiens



<220>
<221>  MISC_FEATURE
<223>  synthetic

<400>  53

Gly Val Thr Ser Val Ser Gln Ile Phe His Ser Pro Asp Leu Ala Ile
1               5                   10                  15



Arg




<210>  54
<211>  18
<212>  PRT
<213>  Homo sapiens



<220>
<221>  MISC_FEATURE
<223>  synthetic

<400>  54

Asn Pro Asn Ala Thr Ser Ser Ser Ser Gln Asp Pro Glu Ser Leu Gln

1               5                   10                  15

Asp Arg

<210>  55
<211>  8
<212>  PRT
<213>  Homo sapiens

<220>
<221>  MISC_FEATURE
<223>  synthetic

<400>  55

Asp Thr Phe Val Asn Ala Ser Arg
1               5

<210>  56
<211>  8
<212>  PRT
<213>  Homo sapiens

<220>
<221>  MISC_FEATURE
<223>  synthetic

<400>  56

Thr Leu Tyr Ser Ser Ser Pro Arg
1               5

<210>  57
<211>  19
<212>  PRT
<213>  Homo sapiens

<220>
<221>  MISC_FEATURE
<223>  synthetic

<400>  57

Val Leu Ser Asn Asn Ser Asp Ala Asn Leu Glu Leu Ile Asn Thr Trp
1               5                   10                  15

Val Ala Lys

<210>  58
<211>  5
<212>  PRT
<213>  Homo sapiens

<220>
<221>  MISC_FEATURE
<223>  synthetic

<400>  58

Asn Thr Asn Asn Lys
1               5


<210>  59
<211>  10
<212>  PRT
<213>  Homo sapiens


<220>
<221>  MISC_FEATURE
<223>  synthetic

<400>  59

Leu Leu Asp Ser Leu Pro Ser Asp Thr Arg
1               5                   10


<210>  60
<211>  6
<212>  PRT
<213>  Homo sapiens


<220>
<221>  MISC_FEATURE
<223>  synthetic

<400>  60

Thr Thr Phe Asp Pro Lys
1               5


<210>  61
<211>  7
<212>  PRT
<213>  Homo sapiens


<220>
<221>  MISC_FEATURE
<223>  synthetic

<400>  61

Met Glu Pro Phe His Phe Lys
1               5


<210>  62
<211>  7
<212>  PRT

<213>  Homo sapiens

<220>
<221>  MISC_FEATURE
<223>  synthetic

<400>  62

Val Pro Met Met Asn Ser Lys
1               5

<210>  63
<211>  12
<212>  PRT
<213>  Homo sapiens

<220>
<221>  MISC_FEATURE
<223>  synthetic

<400>  63

Tyr Pro Val Ala His Phe Ile Asp Gln Thr Leu Lys
1               5                   10

<210>  64
<211>  21
<212>  PRT
<213>  Homo sapiens

<220>
<221>  MISC_FEATURE
<223>  synthetic

<400>  64

Val Gly Gln Leu Gln Leu Ser His Asn Leu Ser Leu Val Ile Leu Val
1               5                   10                  15

Pro Gln Asn Leu Lys
            20

<210>  65
<211>  14
<212>  PRT
<213>  Homo sapiens

<220>
<221>  MISC_FEATURE
<223>  synthetic

<400>  65

Leu Glu Asp Met Glu Gln Ala Leu Ser Pro Ser Val Phe Lys
1               5                   10

<210>  66
<211>  5
<212>  PRT
<213>  Homo sapiens

<220>
<221>  MISC_FEATURE
<223>  synthetic

<400>  66

Ala Ile Met Glu Lys
1               5

<210>  67
<211>  5
<212>  PRT
<213>  Homo sapiens

<220>
<221>  MISC_FEATURE
<223>  synthetic

<400>  67

Leu Glu Met Ser Lys
1               5

<210>  68
<211>  10
<212>  PRT
<213>  Homo sapiens

<220>
<221>  MISC_FEATURE
<223>  synthetic

<400>  68

Phe Gln Pro Thr Leu Leu Thr Leu Pro Arg
1               5                   10

<210>  69
<211>  13
<212>  PRT
<213>  Homo sapiens

<220>
<221>  MISC_FEATURE
<223>  synthetic

<400>  69

Val Thr Thr Ser Gln Asp Met Leu Ser Ile Met Glu Lys

1               5                   10


<210>  70
<211>  4
<212>  PRT
<213>  Homo sapiens


<220>
<221>  MISC_FEATURE
<223>  synthetic

<400>  70

Gly Glu Gly Lys
1


<210>  71
<211>  51
<212>  PRT
<213>  Homo sapiens


<220>
<221>  MISC_FEATURE
<223>  synthetic

<400>  71

Leu Glu Phe Phe Asp Phe Ser Tyr Asp Leu Asn Leu Cys Gly Leu Thr
1               5                   10                  15


Glu Asp Pro Asp Leu Gln Val Ser Ala Met Gln His Gln Thr Val Leu
            20                  25                  30


Glu Leu Thr Glu Thr Gly Val Glu Ala Ala Ala Ala Ser Ala Ile Ser
        35                  40                  45


Val Ala Arg
    50


<210>  72
<211>  21
<212>  PRT
<213>  Homo sapiens


<220>
<221>  MISC_FEATURE
<223>  synthetic

<400>  72

Thr Leu Leu Val Phe Glu Val Gln Gln Pro Phe Leu Phe Val Leu Trp
1               5                   10                  15

Asp Gln Gln His Lys
            20

<210>  73
<211>  7
<212>  PRT
<213>  Homo sapiens

<220>
<221>  MISC_FEATURE
<223>  synthetic

<400>  73

Phe Pro Val Phe Met Gly Arg
1               5

<210>  74
<211>  5
<212>  PRT
<213>  Homo sapiens

<220>
<221>  MISC_FEATURE
<223>  synthetic

<400>  74

Val Tyr Asp Pro Arg
1               5

<210>  75
<211>  25
<212>  PRT
<213>  Homo sapiens

<220>
<221>  MISC_FEATURE
<223>  synthetic

<400>  75

Met Leu Phe Val Glu Pro Ile Leu Glu Val Ser Ser Leu Pro Thr Thr
1               5                   10                  15

Asn Ser Thr Thr Asn Ser Ala Thr Lys
            20                  25

<210>  76
<211>  1698
<212>  PRT
<213>  Homo sapiens

<220>

<221>  MISC_FEATURE
<223>  synthetic

<400>  76

Met Arg Leu Leu Trp Gly Leu Ile Trp Ala Ser Ser Phe Phe Thr Leu
1               5                   10                  15

Ser Leu Gln Lys Pro Arg Leu Leu Leu Phe Ser Pro Ser Val Val His
            20                  25                  30

Leu Gly Val Pro Leu Ser Val Gly Val Gln Leu Gln Asp Val Pro Arg
        35                  40                  45

Gly Gln Val Val Lys Gly Ser Val Phe Leu Arg Asn Pro Ser Arg Asn
    50                  55                  60

Asn Val Pro Cys Ser Pro Lys Val Asp Phe Thr Leu Ser Ser Glu Arg
65                  70                  75                  80

Asp Phe Ala Leu Leu Ser Leu Gln Val Pro Leu Lys Asp Ala Lys Ser
                85                  90                  95

Cys Gly Leu His Gln Leu Leu Arg Gly Pro Glu Val Gln Leu Val Ala
            100                 105                 110

His Ser Pro Trp Leu Lys Asp Ser Leu Ser Arg Thr Thr Asn Ile Gln
        115                 120                 125

Gly Ile Asn Leu Leu Phe Ser Ser Arg Arg Gly His Leu Phe Leu Gln
    130                 135                 140

Thr Asp Gln Pro Ile Tyr Asn Pro Gly Gln Arg Val Arg Tyr Arg Val
145                 150                 155                 160

Phe Ala Leu Asp Gln Lys Met Arg Pro Ser Thr Asp Thr Ile Thr Val
                165                 170                 175

Met Val Glu Asn Ser His Gly Leu Arg Val Arg Lys Lys Glu Val Tyr
            180                 185                 190

Met Pro Ser Ser Ile Phe Gln Asp Asp Phe Val Ile Pro Asp Ile Ser
        195                 200                 205

Glu Pro Gly Thr Trp Lys Ile Ser Ala Arg Phe Ser Asp Gly Leu Glu
    210                 215                 220

Ser Asn Ser Ser Thr Gln Phe Glu Val Lys Lys Tyr Val Leu Pro Asn
225                 230                 235                 240

Phe Glu Val Lys Ile Thr Pro Gly Lys Pro Tyr Ile Leu Thr Val Pro
                245                 250                 255

Gly His Leu Asp Glu Met Gln Leu Asp Ile Gln Ala Arg Tyr Ile Tyr
            260                 265                 270

Gly Lys Pro Val Gln Gly Val Ala Tyr Val Arg Phe Gly Leu Leu Asp
        275                 280                 285

Glu Asp Gly Lys Lys Thr Phe Phe Arg Gly Leu Glu Ser Gln Thr Lys
    290                 295                 300

Leu Val Asn Gly Gln Ser His Ile Ser Leu Ser Lys Ala Glu Phe Gln
305                 310                 315                 320

Asp Ala Leu Glu Lys Leu Asn Met Gly Ile Thr Asp Leu Gln Gly Leu
                325                 330                 335

Arg Leu Tyr Val Ala Ala Ala Ile Ile Glu Ser Pro Gly Gly Glu Met
            340                 345                 350

Glu Glu Ala Glu Leu Thr Ser Trp Tyr Phe Val Ser Ser Pro Phe Ser
        355                 360                 365

Leu Asp Leu Ser Lys Thr Lys Arg His Leu Val Pro Gly Ala Pro Phe
    370                 375                 380

Leu Leu Gln Ala Leu Val Arg Glu Met Ser Gly Ser Pro Ala Ser Gly
385                 390                 395                 400

Ile Pro Val Lys Val Ser Ala Thr Val Ser Ser Pro Gly Ser Val Pro
                405                 410                 415

Glu Val Gln Asp Ile Gln Gln Asn Thr Asp Gly Ser Gly Gln Val Ser
            420                 425                 430

Ile Pro Ile Ile Ile Pro Gln Thr Ile Ser Glu Leu Gln Leu Ser Val
        435                 440                 445

Ser Ala Gly Ser Pro His Pro Ala Ile Ala Arg Leu Thr Val Ala Ala
    450                 455                 460

Pro Pro Ser Gly Gly Pro Gly Phe Leu Ser Ile Glu Arg Pro Asp Ser
465                 470                 475                 480

Arg Pro Pro Arg Val Gly Asp Thr Leu Asn Leu Asn Leu Arg Ala Val

                485                 490                 495

Gly Ser Gly Ala Thr Phe Ser His Tyr Tyr Tyr Met Ile Leu Ser Arg
            500                 505                 510

Gly Gln Ile Val Phe Met Asn Arg Glu Pro Lys Arg Thr Leu Thr Ser
        515                 520                 525

Val Ser Val Phe Val Asp His His Leu Ala Pro Ser Phe Tyr Phe Val
    530                 535                 540

Ala Phe Tyr Tyr His Gly Asp His Pro Val Ala Asn Ser Leu Arg Val
545                 550                 555                 560

Asp Val Gln Ala Gly Ala Cys Glu Gly Lys Leu Glu Leu Ser Val Asp
                565                 570                 575

Gly Ala Lys Gln Tyr Arg Asn Gly Glu Ser Val Lys Leu His Leu Glu
            580                 585                 590

Thr Asp Ser Leu Ala Leu Val Ala Leu Gly Ala Leu Asp Thr Ala Leu
        595                 600                 605

Tyr Ala Ala Gly Ser Lys Ser His Lys Pro Leu Asn Met Gly Lys Val
    610                 615                 620

Phe Glu Ala Met Asn Ser Tyr Asp Leu Gly Cys Gly Pro Gly Gly Gly
625                 630                 635                 640

Asp Ser Ala Leu Gln Val Phe Gln Ala Ala Gly Leu Ala Phe Ser Asp
                645                 650                 655

Gly Asp Gln Trp Thr Leu Ser Arg Lys Arg Leu Ser Cys Pro Lys Glu
            660                 665                 670

Lys Thr Thr Arg Lys Lys Arg Asn Val Asn Phe Gln Lys Ala Ile Asn
        675                 680                 685

Glu Lys Leu Gly Gln Tyr Ala Ser Pro Thr Ala Lys Arg Cys Cys Gln
    690                 695                 700

Asp Gly Val Thr Arg Leu Pro Met Met Arg Ser Cys Glu Gln Arg Ala
705                 710                 715                 720

Ala Arg Val Gln Gln Pro Asp Cys Arg Glu Pro Phe Leu Ser Cys Cys
                725                 730                 735

Gln Phe Ala Glu Ser Leu Arg Lys Lys Ser Arg Asp Lys Gly Gln Ala
        740                 745                 750

Gly Leu Gln Arg Ala Leu Glu Ile Leu Gln Glu Glu Asp Leu Ile Asp
    755                 760                 765

Glu Asp Asp Ile Pro Val Arg Ser Phe Phe Pro Glu Asn Trp Leu Trp
    770                 775                 780

Arg Val Glu Thr Val Asp Arg Phe Gln Ile Leu Thr Leu Trp Leu Pro
785                 790                 795                 800

Asp Ser Leu Thr Thr Trp Glu Ile His Gly Leu Ser Leu Ser Lys Thr
                805                 810                 815

Lys Gly Leu Cys Val Ala Thr Pro Val Gln Leu Arg Val Phe Arg Glu
            820                 825                 830

Phe His Leu His Leu Arg Leu Pro Met Ser Val Arg Arg Phe Glu Gln
        835                 840                 845

Leu Glu Leu Arg Pro Val Leu Tyr Asn Tyr Leu Asp Lys Asn Leu Thr
    850                 855                 860

Val Ser Val His Val Ser Pro Val Glu Gly Leu Cys Leu Ala Gly Gly
865                 870                 875                 880

Gly Gly Leu Ala Gln Gln Val Leu Val Pro Ala Gly Ser Ala Arg Pro
                885                 890                 895

Val Ala Phe Ser Val Val Pro Thr Ala Ala Ala Ala Val Ser Leu Lys
            900                 905                 910

Val Val Ala Arg Gly Ser Phe Glu Phe Pro Val Gly Asp Ala Val Ser
        915                 920                 925

Lys Val Leu Gln Ile Glu Lys Glu Gly Ala Ile His Arg Glu Glu Leu
    930                 935                 940

Val Tyr Glu Leu Asn Pro Leu Asp His Arg Gly Arg Thr Leu Glu Ile
945                 950                 955                 960

Pro Gly Asn Ser Asp Pro Asn Met Ile Pro Asp Gly Asp Phe Asn Ser
                965                 970                 975

Tyr Val Arg Val Thr Ala Ser Asp Pro Leu Asp Thr Leu Gly Ser Glu
            980                 985                 990

Gly Ala Leu Ser Pro Gly Gly Val Ala Ser Leu Leu Arg Leu Pro Arg
        995                 1000                1005

Gly Cys Gly Glu Gln Thr Met Ile Tyr Leu Ala Pro Thr Leu Ala
    1010                1015                1020

Ala Ser Arg Tyr Leu Asp Lys Thr Glu Gln Trp Ser Thr Leu Pro
    1025                1030                1035

Pro Glu Thr Lys Asp His Ala Val Asp Leu Ile Gln Lys Gly Tyr
    1040                1045                1050

Met Arg Ile Gln Gln Phe Arg Lys Ala Asp Gly Ser Tyr Ala Ala
    1055                1060                1065

Trp Leu Ser Arg Asp Ser Ser Thr Trp Leu Thr Ala Phe Val Leu
    1070                1075                1080

Lys Val Leu Ser Leu Ala Gln Glu Gln Val Gly Gly Ser Pro Glu
    1085                1090                1095

Lys Leu Gln Glu Thr Ser Asn Trp Leu Leu Ser Gln Gln Gln Ala
    1100                1105                1110

Asp Gly Ser Phe Gln Asp Pro Cys Pro Val Leu Asp Arg Ser Met
    1115                1120                1125

Gln Gly Gly Leu Val Gly Asn Asp Glu Thr Val Ala Leu Thr Ala
    1130                1135                1140

Phe Val Thr Ile Ala Leu His His Gly Leu Ala Val Phe Gln Asp
    1145                1150                1155

Glu Gly Ala Glu Pro Leu Lys Gln Arg Val Glu Ala Ser Ile Ser
    1160                1165                1170

Lys Ala Asn Ser Phe Leu Gly Glu Lys Ala Ser Ala Gly Leu Leu
    1175                1180                1185

Gly Ala His Ala Ala Ala Ile Thr Ala Tyr Ala Leu Thr Leu Thr
    1190                1195                1200

Lys Ala Pro Val Asp Leu Leu Gly Val Ala His Asn Asn Leu Met
    1205                1210                1215

Ala Met Ala Gln Glu Thr Gly Asp Asn Leu Tyr Trp Gly Ser Val
    1220                1225                1230

Thr Gly Ser Gln Ser Asn Ala Val Ser Pro Thr Pro Ala Pro Arg
    1235                1240                1245

Asn Pro Ser Asp Pro Met Pro Gln Ala Pro Ala Leu Trp Ile Glu
    1250                1255                1260

Thr Thr Ala Tyr Ala Leu Leu His Leu Leu Leu His Glu Gly Lys
    1265                1270                1275

Ala Glu Met Ala Asp Gln Ala Ser Ala Trp Leu Thr Arg Gln Gly
    1280                1285                1290

Ser Phe Gln Gly Gly Phe Arg Ser Thr Gln Asp Thr Val Ile Ala
    1295                1300                1305

Leu Asp Ala Leu Ser Ala Tyr Trp Ile Ala Ser His Thr Thr Glu
    1310                1315                1320

Glu Arg Gly Leu Asn Val Thr Leu Ser Ser Thr Gly Arg Asn Gly
    1325                1330                1335

Phe Lys Ser His Ala Leu Gln Leu Asn Asn Arg Gln Ile Arg Gly
    1340                1345                1350

Leu Glu Glu Glu Leu Gln Phe Ser Leu Gly Ser Lys Ile Asn Val
    1355                1360                1365

Lys Val Gly Gly Asn Ser Lys Gly Thr Leu Lys Val Leu Arg Thr
    1370                1375                1380

Tyr Asn Val Leu Asp Met Lys Asn Thr Thr Cys Gln Asp Leu Gln
    1385                1390                1395

Ile Glu Val Thr Val Lys Gly His Val Glu Tyr Thr Met Glu Ala
    1400                1405                1410

Asn Glu Asp Tyr Glu Asp Tyr Glu Tyr Asp Glu Leu Pro Ala Lys
    1415                1420                1425

Asp Asp Pro Asp Ala Pro Leu Gln Pro Val Thr Pro Leu Gln Leu
    1430                1435                1440

Phe Glu Gly Arg Arg Asn Arg Arg Arg Arg Glu Ala Pro Lys Leu
    1445                1450                1455

Thr Ser Leu Ser Asp Arg Tyr Val Ser His Phe Glu Thr Glu Gly

1460                1465                1470

Pro His Val Leu Leu Tyr Phe Asp Ser Val Pro Thr Ser Arg Glu
    1475                1480                1485

Cys Val Gly Phe Glu Ala Val Gln Glu Val Pro Val Gly Leu Val
    1490                1495                1500

Gln Pro Ala Ser Ala Thr Leu Tyr Asp Tyr Tyr Asn Pro Glu Arg
    1505                1510                1515

Arg Cys Ser Val Phe Tyr Gly Ala Pro Ser Lys Ser Arg Leu Leu
    1520                1525                1530

Ala Thr Leu Cys Ser Ala Glu Val Cys Gln Cys Ala Glu Gly Lys
    1535                1540                1545

Cys Pro Arg Gln Arg Arg Ala Leu Glu Arg Gly Leu Gln Asp Glu
    1550                1555                1560

Asp Gly Tyr Arg Met Lys Phe Ala Cys Tyr Tyr Pro Arg Val Glu
    1565                1570                1575

Tyr Gly Phe Gln Val Lys Val Leu Arg Glu Asp Ser Arg Ala Ala
    1580                1585                1590

Phe Arg Leu Phe Glu Thr Lys Ile Thr Gln Val Leu His Phe Thr
    1595                1600                1605

Lys Asp Val Lys Ala Ala Ala Asn Gln Met Arg Asn Phe Leu Val
    1610                1615                1620

Arg Ala Ser Cys Arg Leu Arg Leu Glu Pro Gly Lys Glu Tyr Leu
    1625                1630                1635

Ile Met Gly Leu Asp Gly Ala Thr Tyr Asp Leu Glu Gly His Pro
    1640                1645                1650

Gln Tyr Leu Leu Asp Ser Asn Ser Trp Ile Glu Glu Met Pro Ser
    1655                1660                1665

Glu Arg Leu Cys Arg Ser Thr Arg Gln Arg Ala Ala Cys Ala Gln
    1670                1675                1680

Leu Asn Asp Phe Leu Gln Glu Tyr Gly Thr Gln Gly Cys Gln Val
    1685                1690                1695

<210>  77
<211>  18
<212>  PRT
<213>  Homo sapiens

<220>
<221>  MISC_FEATURE
<223>  synthetic

<400>  77

Gly Cys Gly Glu Gln Thr Met Ile Tyr Leu Ala Pro Thr Leu Ala Ala
1               5                   10                  15

Ser Arg

<210>  78
<211>  18
<212>  PRT
<213>  Homo sapiens

<220>
<221>  MISC_FEATURE
<223>  synthetic

<400>  78

Gly Cys Gly Glu Gln Thr Met Ile Tyr Leu Ala Pro Thr Leu Ala Ala
1               5                   10                  15

Ser Arg

<210>  79
<211>  4
<212>  PRT
<213>  Homo sapiens

<220>
<221>  MISC_FEATURE
<223>  synthetic

<400>  79

Tyr Leu Asp Lys
1

<210>  80
<211>  12
<212>  PRT
<213>  Homo sapiens

<220>

<221>  MISC_FEATURE
<223>  synthetic

<400>  80

Thr Glu Gln Trp Ser Thr Leu Pro Pro Glu Thr Lys
1               5                   10


<210>  81
<211>  9
<212>  PRT
<213>  Homo sapiens


<220>
<221>  MISC_FEATURE
<223>  synthetic

<400>  81

Asp His Ala Val Asp Leu Ile Gln Lys
1               5


<210>  82
<211>  4
<212>  PRT
<213>  Homo sapiens


<220>
<221>  MISC_FEATURE
<223>  synthetic

<400>  82

Gly Tyr Met Arg
1


<210>  83
<211>  11
<212>  PRT
<213>  Homo sapiens


<220>
<221>  MISC_FEATURE
<223>  synthetic

<400>  83

Ala Asp Gly Ser Tyr Ala Ala Trp Leu Ser Arg
1               5                   10


<210>  84
<211>  12
<212>  PRT
<213>  Homo sapiens

<220>
<221>  MISC_FEATURE
<223>  synthetic

<400>  84

Gly Ser Ser Thr Trp Leu Thr Ala Phe Val Leu Lys
1               5                   10


<210>  85
<211>  15
<212>  PRT
<213>  Homo sapiens


<220>
<221>  MISC_FEATURE
<223>  synthetic

<400>  85

Val Leu Ser Leu Ala Gln Glu Gln Val Gly Gly Ser Pro Glu Lys
1               5                   10                  15


<210>  86
<211>  27
<212>  PRT
<213>  Homo sapiens


<220>
<221>  MISC_FEATURE
<223>  synthetic

<400>  86

Leu Gln Glu Thr Ser Asn Trp Leu Leu Ser Gln Gln Gln Ala Asp Gly
1               5                   10                  15


Ser Phe Gln Asp Leu Ser Pro Val Ile His Arg
            20                  25


<210>  87
<211>  7
<212>  PRT
<213>  Homo sapiens


<220>
<221>  MISC_FEATURE
<223>  synthetic

<400>  87

Val Glu Ala Ser Ile Ser Lys
1               5


<210>  88

<211>  8
<212>  PRT
<213>  Homo sapiens

<220>
<221>  MISC_FEATURE
<223>  synthetic

<400>  88

Ala Ser Ser Phe Leu Gly Glu Lys
1               5

<210>  89
<211>  22
<212>  PRT
<213>  Homo sapiens

<220>
<221>  MISC_FEATURE
<223>  synthetic

<400>  89

Ala Ser Ala Gly Leu Leu Gly Ala His Ala Ala Ala Ile Thr Ala Tyr
1               5                   10                  15

Ala Leu Thr Leu Thr Lys
            20

<210>  90
<211>  38
<212>  PRT
<213>  Homo sapiens

<220>
<221>  MISC_FEATURE
<223>  synthetic

<400>  90

Gly Val Ala His Asn Asn Leu Met Ala Met Ala Gln Glu Thr Gly Asp
1               5                   10                  15

Asn Leu Tyr Trp Gly Ser Val Thr Gly Ser Gln Ser Asn Ala Val Ser
            20                  25                  30

Pro Thr Pro Ala Pro Arg
        35

<210>  91
<211>  30
<212>  PRT
<213>  Homo sapiens

<220>
<221>  MISC_FEATURE
<223>  synthetic

<400>  91

Asn Pro Ser Asp Pro Met Pro Gln Ala Pro Ala Leu Trp Ile Glu Thr
1               5                   10                  15

Thr Ala Tyr Ala Leu Leu His Leu Leu Leu His Glu Gly Lys
            20                  25                  30

<210>  92
<211>  13
<212>  PRT
<213>  Homo sapiens

<220>
<221>  MISC_FEATURE
<223>  synthetic

<400>  92

Ala Glu Met Ala Asp Gln Ala Ala Ala Trp Leu Thr Arg
1               5                   10

<210>  93
<211>  9
<212>  PRT
<213>  Homo sapiens

<220>
<221>  MISC_FEATURE
<223>  synthetic

<400>  93

Gln Gly Ser Phe Gln Gly Gly Phe Arg
1               5

<210>  94
<211>  25
<212>  PRT
<213>  Homo sapiens

<220>
<221>  MISC_FEATURE
<223>  synthetic

<400>  94

Ser Thr Gln Asp Thr Val Ile Ala Leu Asp Ala Leu Ser Ala Tyr Trp
1               5                   10                  15

Ile Ala Ser His Thr Thr Glu Glu Arg
            20                  25

<210>  95
<211>  11
<212>  PRT
<213>  Homo sapiens

<220>
<221>  MISC_FEATURE
<223>  synthetic

<400>  95

Gly Leu Asn Val Thr Leu Ser Ser Thr Gly Arg
1               5                   10

<210>  96
<211>  4
<212>  PRT
<213>  Homo sapiens

<220>
<221>  MISC_FEATURE
<223>  synthetic

<400>  96

Asn Gly Phe Lys
1

<210>  97
<211>  9
<212>  PRT
<213>  Homo sapiens

<220>
<221>  MISC_FEATURE
<223>  synthetic

<400>  97

Ser His Ala Leu Gln Leu Asn Asn Arg
1               5

<210>  98
<211>  13
<212>  PRT
<213>  Homo sapiens

<220>
<221>  MISC_FEATURE
<223>  synthetic

<400>  98

Gly Leu Glu Glu Glu Leu Gln Phe Ser Leu Gly Ser Lys
1               5                   10


<210>  99
<211>  6
<212>  PRT
<213>  Homo sapiens


<220>
<221>  MISC_FEATURE
<223>  synthetic

<400>  99

Val Gly Gly Asn Ser Lys
1               5


<210>  100
<211>  8
<212>  PRT
<213>  Homo sapiens


<220>
<221>  MISC_FEATURE
<223>  synthetic

<400>  100

Thr Tyr Asn Val Leu Asp Met Lys
1               5


<210>  101
<211>  14
<212>  PRT
<213>  Homo sapiens


<220>
<221>  MISC_FEATURE
<223>  synthetic

<400>  101

Asn Thr Thr Cys Gln Asp Leu Gln Ile Glu Val Thr Val Lys
1               5                   10


<210>  102
<211>  14
<212>  PRT
<213>  Homo sapiens


<220>
<221>  MISC_FEATURE
<223>  synthetic

<400>  102

Asn Thr Thr Cys Gln Asp Leu Gln Ile Glu Val Thr Val Lys
1               5                   10


<210>  103
<211>  24
<212>  PRT
<213>  Homo sapiens


<220>
<221>  MISC_FEATURE
<223>  synthetic

<400>  103

Gly His Val Glu Tyr Thr Met Glu Ala Asn Glu Asp Tyr Glu Asp Tyr
1               5                   10                  15


Glu Tyr Asp Glu Leu Pro Ala Lys
            20


<210>  104
<211>  18
<212>  PRT
<213>  Homo sapiens


<220>
<221>  MISC_FEATURE
<223>  synthetic

<400>  104

Asp Asp Pro Asp Ala Pro Leu Gln Pro Val Thr Pro Leu Gln Leu Phe
1               5                   10                  15


Glu Gly



<210>  105
<211>  6
<212>  PRT
<213>  Homo sapiens


<220>
<221>  MISC_FEATURE
<223>  synthetic

<400>  105

Asn Val Asn Phe Gln Lys
1               5

<210>  106
<211>  5
<212>  PRT
<213>  Homo sapiens

<220>
<221>  MISC_FEATURE
<223>  synthetic

<400>  106

Ala Ile Asn Glu Lys
1               5

<210>  107
<211>  10
<212>  PRT
<213>  Homo sapiens

<220>
<221>  MISC_FEATURE
<223>  synthetic

<400>  107

Leu Gly Gln Tyr Ala Ser Pro Thr Ala Lys
1               5                   10

<210>  108
<211>  8
<212>  PRT
<213>  Homo sapiens

<220>
<221>  MISC_FEATURE
<223>  synthetic

<400>  108

Cys Cys Gln Asp Gly Val Thr Arg
1               5

<210>  109
<211>  5
<212>  PRT
<213>  Homo sapiens

<220>
<221>  MISC_FEATURE
<223>  synthetic

<400>  109

Leu Pro Met Met Arg
1               5

<210>  110
<211>  5
<212>  PRT
<213>  Homo sapiens

<220>
<221>  MISC_FEATURE
<223>  synthetic

<400>  110

Ser Cys Glu Gln Arg
1               5

<210>  111
<211>  7
<212>  PRT
<213>  Homo sapiens

<220>
<221>  MISC_FEATURE
<223>  synthetic

<400>  111

Val Gln Gln Pro Asp Cys Arg
1               5

<210>  112
<211>  7
<212>  PRT
<213>  Homo sapiens

<220>
<221>  MISC_FEATURE
<223>  synthetic

<400>  112

Val Gln Gln Pro Asp Cys Arg
1               5

<210>  113
<211>  14
<212>  PRT
<213>  Homo sapiens

<220>
<221>  MISC_FEATURE
<223>  synthetic

<400>  113

Glu Pro Phe Leu Ser Cys Cys Gln Phe Ala Glu Ser Leu Arg
1               5                   10

<210>  114
<211>  7
<212>  PRT
<213>  Homo sapiens

<220>
<221>  MISC_FEATURE
<223>  synthetic

<400>  114

Gly Gln Ala Gly Leu Gln Arg
1               5

<210>  115
<211>  19
<212>  PRT
<213>  Homo sapiens

<220>
<221>  MISC_FEATURE
<223>  synthetic

<400>  115

Ala Leu Glu Ile Leu Gln Glu Glu Asp Leu Ile Asp Glu Asp Asp Ile
1               5                   10                  15

Pro Val Arg

<210>  116
<211>  10
<212>  PRT
<213>  Homo sapiens

<220>
<221>  MISC_FEATURE
<223>  synthetic

<400>  116

Ser Phe Phe Pro Glu Asn Trp Leu Trp Arg
1               5                   10

<210>  117
<211>  6
<212>  PRT
<213>  Homo sapiens

<220>
<221>  MISC_FEATURE
<223>  syynthetic

<400>  117

Val Glu Thr Val Asp Arg
1               5


<210>  118
<211>  24
<212>  PRT
<213>  Homo sapiens


<220>
<221>  MISC_FEATURE
<223>  synthetic

<400>  118

Phe Gln Ile Leu Thr Leu Trp Leu Pro Asp Ser Leu Thr Thr Trp Glu
1               5                   10                  15


Ile His Gly Leu Ser Leu Ser Lys
            20


<210>  119
<211>  11
<212>  PRT
<213>  Homo sapiens


<220>
<221>  MISC_FEATURE
<223>  synthetic

<400>  119

Gly Leu Cys Val Ala Thr Pro Val Gln Leu Arg
1               5                   10


<210>  120
<211>  11
<212>  PRT
<213>  Homo sapiens


<220>
<221>  MISC_FEATURE
<223>  synthetic

<400>  120

Gly Leu Cys Val Ala Thr Pro Val Gln Leu Arg
1               5                   10


<210>  121
<211>  7
<212>  PRT
<213>  Homo sapiens

<220>
<221>  MISC_FEATURE
<223>  synthetic

<400>  121

Glu Phe His Leu His Leu Arg
1               5


<210>  122
<211>  16
<212>  PRT
<213>  Homo sapiens


<220>
<221>  MISC_FEATURE
<223>  synthetic

<400>  122

Phe Glu Gln Leu Glu Leu Arg Pro Val Leu Tyr Asn Tyr Leu Asp Lys
1               5                   10                  15


<210>  123
<211>  51
<212>  PRT
<213>  Homo sapiens


<220>
<221>  MISC_FEATURE
<223>  synthetic

<400>  123

Asn Leu Thr Val Ser Val His Val Ser Pro Val Glu Gly Leu Cys Leu
1               5                   10                  15


Ala Gly Gly Gly Gly Leu Ala Gln Gln Val Leu Val Pro Ala Gly Ser
            20                  25                  30


Ala Arg Pro Val Ala Phe Ser Val Val Pro Thr Ala Ala Thr Ala Val
        35                  40                  45


Ser Leu Lys
    50


<210>  124
<211>  51
<212>  PRT
<213>  Homo sapiens


<220>

<221>  MISC_FEATURE
<223>  synthetic

<400>  124

Asn Leu Thr Val Ser Val His Val Ser Pro Val Glu Gly Leu Cys Leu
1               5                   10                  15

Ala Gly Gly Gly Gly Leu Ala Gln Gln Val Leu Val Pro Ala Gly Ser
            20                  25                  30

Ala Arg Pro Val Ala Phe Ser Val Val Pro Thr Ala Ala Thr Ala Val
        35                  40                  45

Ser Leu Lys
    50

<210>  125
<211>  4
<212>  PRT
<213>  Homo sapiens

<220>
<221>  MISC_FEATURE
<223>  synthetic

<400>  125

Val Val Ala Arg
1

<210>  126
<211>  13
<212>  PRT
<213>  Homo sapiens

<220>
<221>  MISC_FEATURE
<223>  synthetic

<400>  126

Gly Ser Phe Glu Phe Pro Val Gly Asp Ala Val Ser Lys
1               5                   10

<210>  127
<211>  6
<212>  PRT
<213>  Homo sapiens

<220>
<221>  MISC_FEATURE
<223>  synthetic

<400>  127

Glu Gly Ala Ile His Arg
1               5


<210>  128
<211>  13
<212>  PRT
<213>  Homo sapiens


<220>
<221>  MISC_FEATURE
<223>  synthetic

<400>  128

Glu Glu Leu Val Tyr Glu Leu Asn Pro Leu Asp His Arg
1               5                   10


<210>  129
<211>  23
<212>  PRT
<213>  Homo sapiens


<220>
<221>  MISC_FEATURE
<223>  synthetic

<400>  129

Thr Leu Glu Ile Pro Gly Asn Ser Asp Pro Asn Met Ile Pro Asp Gly
1               5                   10                  15


Asp Phe Asn Ser Tyr Val Arg
            20


<210>  130
<211>  26
<212>  PRT
<213>  Homo sapiens


<220>
<221>  MISC_FEATURE
<223>  synthetic

<400>  130

Val Thr Ala Ser Asp Pro Leu Asp Thr Leu Gly Ser Glu Gly Ala Leu
1               5                   10                  15


Ser Pro Gly Gly Val Ala Ser Leu Leu Arg
            20                  25


<210>  131

<211>  14
<212>  PRT
<213>  Homo sapiens

<220>
<221>  MISC_FEATURE
<223>  synthetic

<400>  131

Gly Pro Glu Val Gln Leu Val Ala His Ser Pro Trp Leu Lys
1               5                   10

<210>  132
<211>  5
<212>  PRT
<213>  Homo sapiens

<220>
<221>  MISC_FEATURE
<223>  synthetic

<400>  132

Asp Ser Leu Ser Arg
1               5

<210>  133
<211>  14
<212>  PRT
<213>  Homo sapiens

<220>
<221>  MISC_FEATURE
<223>  synthetic

<400>  133

Thr Thr Asn Ile Gln Gly Ile Asn Leu Leu Phe Ser Ser Arg
1               5                   10

<210>  134
<211>  17
<212>  PRT
<213>  Homo sapiens

<220>
<221>  MISC_FEATURE
<223>  synthetic

<400>  134

Gly His Leu Phe Leu Gln Thr Asp Gln Pro Ile Tyr Asn Pro Gly Gln
1               5                   10                  15

Arg

<210>  135
<211>  7
<212>  PRT
<213>  Homo sapiens

<220>
<221>  MISC_FEATURE
<223>  synthetic

<400>  135

Val Phe Ala Leu Asp Gln Lys
1               5

<210>  136
<211>  19
<212>  PRT
<213>  Homo sapiens

<220>
<221>  MISC_FEATURE
<223>  synthetic

<400>  136

Met Arg Pro Ser Thr Asp Thr Ile Thr Val Met Val Glu Asn Ser His
1               5                   10                  15

Gly Leu Arg

<210>  137
<211>  25
<212>  PRT
<213>  Homo sapiens

<220>
<221>  MISC_FEATURE
<223>  synthetic

<400>  137

Glu Val Tyr Met Pro Ser Ser Ile Phe Gln Asp Asp Phe Val Ile Pro
1               5                   10                  15

Asp Ile Ser Glu Pro Gly Thr Trp Lys
            20                  25

<210>  138
<211>  16
<212>  PRT

<213>  Homo sapiens

<220>
<221>  MISC_FEATURE
<223>  synthetic

<400>  138

Phe Ser Asp Gly Leu Glu Ser Asn Ser Ser Thr Gln Phe Glu Val Lys
1               5                   10                  15

<210>  139
<211>  26
<212>  PRT
<213>  Homo sapiens

<220>
<221>  MISC_FEATURE
<223>  synthetic

<400>  139

Leu Leu Leu Phe Ser Pro Ser Val Val His Leu Gly Val Pro Leu Ser
1               5                   10                  15

Val Gly Val Gln Leu Gln Asp Val Pro Arg
            20                  25

<210>  140
<211>  9
<212>  PRT
<213>  Homo sapiens

<220>
<221>  MISC_FEATURE
<223>  synthetic

<400>  140

Tyr Val Leu Pro Asn Phe Glu Val Lys
1               5

<210>  141
<211>  25
<212>  PRT
<213>  Homo sapiens

<220>
<221>  MISC_FEATURE
<223>  synthetic

<400>  141

Ile Thr Pro Gly Lys Pro Tyr Ile Leu Thr Val Pro Gly His Leu Asp
1               5                   10                  15

Glu Met Gln Leu Asp Ile Gln Ala Arg
            20                  25

<210>  142
<211>  14
<212>  PRT
<213>  Homo sapiens

<220>
<221>  MISC_FEATURE
<223>  synthetic

<400>  142

Tyr Ile Tyr Gly Lys Pro Val Gln Gly Val Ala Tyr Val Arg
1               5                   10

<210>  143
<211>  9
<212>  PRT
<213>  Homo sapiens

<220>
<221>  MISC_FEATURE
<223>  synthetic

<400>  143

Phe Gly Leu Leu Asp Glu Asp Gly Lys
1               5

<210>  144
<211>  4
<212>  PRT
<213>  Homo sapiens

<220>
<221>  MISC_FEATURE
<223>  synthetic

<400>  144

Thr Phe Phe Arg
1

<210>  145
<211>  7
<212>  PRT
<213>  Homo sapiens

<220>
<221>  MISC_FEATURE
<223>  synthetic

<400>  145

Gly Leu Glu Ser Gln Thr Lys
1               5


<210>  146
<211>  12
<212>  PRT
<213>  Homo sapiens

<400>  146

Leu Val Asn Gly Gln Ser His Ile Ser Leu Ser Lys
1               5                   10


<210>  147
<211>  12
<212>  PRT
<213>  Homo sapiens


<220>
<221>  MISC_FEATURE
<223>  synthetic

<400>  147

Leu Asn Met Gly Ile Thr Asp Leu Gln Gly Leu Arg
1               5                   10


<210>  148
<211>  36
<212>  PRT
<213>  Homo sapiens


<220>
<221>  MISC_FEATURE
<223>  synthetic

<400>  148

Leu Tyr Val Ala Ala Ala Ile Ile Glu Ser Pro Gly Gly Glu Met Glu
1               5                   10                  15


Glu Ala Glu Leu Thr Ser Trp Tyr Phe Val Ser Ser Pro Phe Ser Leu
            20                  25                  30


Asp Leu Ser Lys
        35


<210>  149
<211>  13
<212>  PRT
<213>  Homo sapiens

<220>
<221>  MISC_FEATURE
<223>  synthetic

<400>  149

Glu Met Ser Gly Ser Pro Ala Ser Gly Ile Pro Val Lys
1               5                   10


<210>  150
<211>  55
<212>  PRT
<213>  Homo sapiens


<220>
<221>  MISC_FEATURE
<223>  synthetic

<400>  150

Val Ser Ala Thr Val Ser Ser Pro Gly Ser Val Pro Glu Val Gln Asp
1               5                   10                  15


Ile Gln Gln Asn Thr Asp Gly Ser Gly Gln Val Ser Ile Pro Ile Ile
            20                  25                  30


Ile Pro Gln Thr Ile Ser Glu Leu Gln Leu Ser Val Ser Ala Gly Ser
        35                  40                  45


Pro His Pro Ala Ile Ala Arg
    50                  55


<210>  151
<211>  25
<212>  PRT
<213>  Homo sapiens


<220>
<221>  MISC_FEATURE
<223>  synthetic

<400>  151

Leu Thr Val Ala Ala Pro Pro Ser Gly Gly Pro Gly Phe Leu Ser Ile
1               5                   10                  15


Glu Arg Pro Asp Ser Arg Pro Pro Arg
            20                  25


<210>  152
<211>  10
<212>  PRT
<213>  Homo sapiens

<220>
<221>  MISC_FEATURE
<223>  synthetic

<400>  152

Val Gly Asp Thr Leu Asn Leu Asn Leu Arg
1               5                   10


<210>  153
<211>  5
<212>  PRT
<213>  Homo sapiens


<220>
<221>  MISC_FEATURE
<223>  synthetic

<400>  153

Gly Gln Val Val Lys
1               5


<210>  154
<211>  18
<212>  PRT
<213>  Homo sapiens


<220>
<221>  MISC_FEATURE
<223>  synthetic

<400>  154

Ala Val Gly Ser Gly Ala Thr Phe Ser His Tyr Tyr Tyr Met Ile Leu
1               5                   10                  15


Ser Arg




<210>  155
<211>  8
<212>  PRT
<213>  Homo sapiens


<220>
<221>  MISC_FEATURE
<223>  synthetic

<400>  155

Gly Gln Ile Val Phe Met Asn Arg
1               5

<210>  156
<211>  35
<212>  PRT
<213>  Homo sapiens

<220>
<221>  MISC_FEATURE
<223>  synthetic

<400>  156

Thr Leu Thr Ser Val Ser Val Phe Val Asp His His Leu Ala Pro Ser
1               5                   10                  15

Phe Tyr Phe Val Ala Phe Tyr Tyr His Gly Asp His Pro Val Ala Asn
            20                  25                  30

Ser Leu Arg
        35

<210>  157
<211>  11
<212>  PRT
<213>  Homo sapiens

<220>
<221>  MISC_FEATURE
<223>  synthetic

<400>  157

Val Asp Val Gln Ala Gly Ala Cys Glu Gly Lys
1               5                   10

<210>  158
<211>  11
<212>  PRT
<213>  Homo sapiens

<220>
<221>  MISC_FEATURE
<223>  synthetic

<400>  158

Val Asp Val Gln Ala Gly Ala Cys Glu Gly Lys
1               5                   10

<210>  159
<211>  9
<212>  PRT
<213>  Homo sapiens

<220>
<221>  MISC_FEATURE
<223>  synthetic

<400>  159

Leu Glu Leu Ser Val Asp Gly Ala Lys
1               5


<210>  160
<211>  6
<212>  PRT
<213>  Homo sapiens


<220>
<221>  MISC_FEATURE
<223>  synthetic

<400>  160

Asn Gly Glu Ser Val Lys
1               5


<210>  161
<211>  26
<212>  PRT
<213>  Homo sapiens


<220>
<221>  MISC_FEATURE
<223>  synthetic

<400>  161

Leu His Leu Glu Thr Asp Ser Leu Ala Leu Val Ala Leu Gly Ala Leu
1               5                   10                  15


Asp Thr Ala Leu Tyr Ala Ala Gly Ser Lys
            20                  25


<210>  162
<211>  4
<212>  PRT
<213>  Homo sapiens


<220>
<221>  MISC_FEATURE
<223>  synthetic

<400>  162

Asn Pro Ser Arg
1


<210>  163

<211>  9
<212>  PRT
<213>  Homo sapiens

<220>
<221>  MISC_FEATURE
<223>  synthetic

<400>  163

Ser His Lys Pro Leu Asn Met Gly Lys
1               5

<210>  164
<211>  41
<212>  PRT
<213>  Homo sapiens

<220>
<221>  MISC_FEATURE
<223>  synthetic

<400>  164

Val Phe Glu Ala Met Asn Ser Tyr Asp Leu Gly Cys Gly Pro Gly Gly
1               5                   10                  15

Gly Asp Ser Ala Leu Gln Val Phe Gln Ala Ala Gly Leu Ala Phe Ser
            20                  25                  30

Asp Gly Asp Gln Trp Thr Leu Ser Arg
        35                  40

<210>  165
<211>  41
<212>  PRT
<213>  Homo sapiens

<220>
<221>  MISC_FEATURE
<223>  synthetic

<400>  165

Val Phe Glu Ala Met Asn Ser Tyr Asp Leu Gly Cys Gly Pro Gly Gly
1               5                   10                  15

Gly Asp Ser Ala Leu Gln Val Phe Gln Ala Ala Gly Leu Ala Phe Ser
            20                  25                  30

Asp Gly Asp Gln Trp Thr Leu Ser Arg
        35                  40

<210>  166
<211>  8
<212>  PRT
<213>  Homo sapiens

<220>
<221>  MISC_FEATURE
<223>  synthetic

<400>  166

Asn Asn Val Pro Cys Ser Pro Lys
1               5

<210>  167
<211>  8
<212>  PRT
<213>  Homo sapiens

<220>
<221>  MISC_FEATURE
<223>  synthetic

<400>  167

Asn Asn Val Pro Cys Ser Pro Lys
1               5

<210>  168
<211>  5
<212>  PRT
<213>  Homo sapiens

<220>
<221>  MISC_FEATURE
<223>  synthetic

<400>  168

Leu Ser Cys Pro Lys
1               5

<210>  169
<211>  5
<212>  PRT
<213>  Homo sapiens

<220>
<221>  MISC_FEATURE
<223>  synthetic

<400>  169

Leu Ser Cys Pro Lys
1               5

<210>  170
<211>  9
<212>  PRT
<213>  Homo sapiens

<220>
<221>  MISC_FEATURE
<223>  synthetic

<400>  170

Val Asp Phe Thr Leu Ser Ser Glu Arg
1               5

<210>  171
<211>  12
<212>  PRT
<213>  Homo sapiens

<220>
<221>  MISC_FEATURE
<223>  synthetic

<400>  171

Asp Phe Ala Leu Leu Ser Leu Gln Val Pro Leu Lys
1               5                   10

<210>  172
<211>  9
<212>  PRT
<213>  Homo sapiens

<220>
<221>  MISC_FEATURE
<223>  synthetic

<400>  172

Ser Cys Gly Leu His Gln Leu Leu Arg
1               5

<210>  173
<211>  9
<212>  PRT
<213>  Homo sapiens

<220>
<221>  MISC_FEATURE
<223>  synthetic

<400>  173

Ser Cys Gly Leu His Gln Leu Leu Arg
1               5

<210>  174
<211>  8
<212>  PRT
<213>  Homo sapiens

<220>
<221>  MISC_FEATURE
<223>  synthetic

<400>  174

Val Val Glu Glu Gln Glu Ser Arg
1               5

<210>  175
<211>  9
<212>  PRT
<213>  Homo sapiens

<220>
<221>  MISC_FEATURE
<223>  synthetic

<400>  175

Val His Tyr Thr Val Cys Ile Trp Arg
1               5

<210>  176
<211>  9
<212>  PRT
<213>  Homo sapiens

<220>
<221>  MISC_FEATURE
<223>  synthetic

<400>  176

Val His Tyr Thr Val Cys Ile Trp Arg
1               5

<210>  177
<211>  21
<212>  PRT
<213>  Homo sapiens

<220>
<221>  MISC_FEATURE
<223>  synthetic

<400>  177

Val Gly Leu Ser Gly Met Ala Ile Ala Asp Val Thr Leu Leu Ser Gly

1               5                   10                  15

Phe His Ala Leu Arg
            20

<210>  178
<211>  5
<212>  PRT
<213>  Homo sapiens

<220>
<221>  MISC_FEATURE
<223>  synthetic

<400>  178

Ala Asp Leu Glu Lys
1               5

<210>  179
<211>  7
<212>  PRT
<213>  Homo sapiens

<220>
<221>  MISC_FEATURE
<223>  synthetic

<400>  179

Leu Thr Ser Leu Ser Asp Arg
1               5

<210>  180
<211>  23
<212>  PRT
<213>  Homo sapiens

<220>
<221>  MISC_FEATURE
<223>  synthetic

<400>  180

Tyr Val Ser His Phe Glu Thr Glu Gly Pro His Val Leu Leu Tyr Phe
1               5                   10                  15

Asp Ser Val Pro Thr Ser Arg
            20

<210>  181
<211>  31
<212>  PRT
<213>  Homo sapiens

<220>
<221>  MISC_FEATURE
<223>  synthetic

<400>  181

Glu Cys Val Gly Phe Glu Ala Val Gln Glu Val Pro Val Gly Leu Val
1               5                   10                  15

Gln Pro Ala Ser Ala Thr Leu Tyr Asp Tyr Tyr Asn Pro Glu Arg
            20                  25                  30

<210>  182
<211>  31
<212>  PRT
<213>  Homo sapiens

<220>
<221>  MISC_FEATURE
<223>  synthetic

<400>  182

Glu Cys Val Gly Phe Glu Ala Val Gln Glu Val Pro Val Gly Leu Val
1               5                   10                  15

Gln Pro Ala Ser Ala Thr Leu Tyr Asp Tyr Tyr Asn Pro Glu Arg
            20                  25                  30

<210>  183
<211>  10
<212>  PRT
<213>  Homo sapiens

<220>
<221>  MISC_FEATURE
<223>  synthetic

<400>  183

Cys Ser Val Phe Tyr Gly Ala Pro Ser Lys
1               5                   10

<210>  184
<211>  10
<212>  PRT
<213>  Homo sapiens

<220>
<221>  MISC_FEATURE
<223>  synthetic

<400>  184

Cys Ser Val Phe Tyr Gly Ala Pro Ser Lys
1               5                   10

<210>  185
<211>  17
<212>  PRT
<213>  Homo sapiens

<220>
<221>  MISC_FEATURE
<223>  synthetic

<400>  185

Leu Leu Ala Thr Leu Cys Ser Ala Glu Val Cys Gln Cys Ala Glu Gly
1               5                   10                  15

Lys

<210>  186
<211>  17
<212>  PRT
<213>  Homo sapiens

<220>
<221>  MISC_FEATURE
<223>  synthetic

<400>  186

Leu Leu Ala Thr Leu Cys Ser Ala Glu Val Cys Gln Cys Ala Glu Gly
1               5                   10                  15

Lys

<210>  187
<211>  4
<212>  PRT
<213>  Homo sapiens

<220>
<221>  MISC_FEATURE
<223>  synthetic

<400>  187

Ala Leu Glu Arg
1

<210>  188
<211>  7

<212>  PRT
<213>  Homo sapiens

<220>
<221>  MISC_FEATURE
<223>  synthetic

<400>  188

Phe Ala Cys Tyr Tyr Pro Arg
1               5

<210>  189
<211>  7
<212>  PRT
<213>  Homo sapiens

<220>
<221>  MISC_FEATURE
<223>  synthetic

<400>  189

Phe Ala Cys Tyr Tyr Pro Arg
1               5

<210>  190
<211>  8
<212>  PRT
<213>  Homo sapiens

<220>
<221>  MISC_FEATURE
<223>  synthetic

<400>  190

Val Glu Tyr Gly Phe Gln Val Lys
1               5

<210>  191
<211>  4
<212>  PRT
<213>  Homo sapiens

<220>
<221>  MISC_FEATURE
<223>  synthetic

<400>  191

Ala Ala Phe Arg
1

<210>  192

<211>  5
<212>  PRT
<213>  Homo sapiens

<220>
<221>  MISC_FEATURE
<223>  synthetic

<400>  192

Leu Phe Glu Thr Lys
1               5

<210>  193
<211>  7
<212>  PRT
<213>  Homo sapiens

<220>
<221>  MISC_FEATURE
<223>  synthetic

<400>  193

Ala Ala Ala Asn Gln Met Arg
1               5

<210>  194
<211>  4
<212>  PRT
<213>  Homo sapiens

<220>
<221>  MISC_FEATURE
<223>  synthetic

<400>  194

Ala Ser Cys Arg
1

<210>  195
<211>  5
<212>  PRT
<213>  Homo sapiens

<220>
<221>  MISC_FEATURE
<223>  synthetic

<400>  195

Leu Glu Pro Gly Lys
1               5

<210>  196
<211>  35
<212>  PRT
<213>  Homo sapiens

<220>
<221>  MISC_FEATURE
<223>  synthetic

<400>  196

Glu Tyr Leu Ile Met Gly Leu Asp Gly Ala Thr Tyr Asp Leu Glu Gly
1               5                   10                  15

His Pro Gln Tyr Leu Leu Asp Ser Asn Ser Trp Ile Glu Glu Met Pro
            20                  25                  30

Ser Glu Arg
        35

<210>  197
<211>  20
<212>  PRT
<213>  Homo sapiens

<220>
<221>  MISC_FEATURE
<223>  synthetic

<400>  197

Ala Ala Cys Ala Gln Leu Asn Asp Phe Leu Gln Glu Tyr Gly Thr Gln
1               5                   10                  15

Gly Cys Gln Val
            20

<210>  198
<211>  20
<212>  PRT
<213>  Homo sapiens

<220>
<221>  MISC_FEATURE
<223>  synthetic

<400>  198

Ala Ala Cys Ala Gln Leu Asn Asp Phe Leu Gln Glu Tyr Gly Thr Gln
1               5                   10                  15

Gly Cys Gln Val
            20

<210>  199
<211>  1663
<212>  PRT
<213>  Homo sapiens

<220>
<221>  MISC_FEATURE
<223>  C3

<400>  199

<210>  200
<211>  15
<212>  PRT
<213>  Homo sapiens

<220>
<221>  MISC_FEATURE
<223>  synthetic

<400>  200

Phe Val Thr Val Gln Ala Thr Phe Gly Thr Gln Val Val Glu Lys
1               5                   10                  15

<210>  201
<211>  17
<212>  PRT
<213>  Homo sapiens

<220>
<221>  MISC_FEATURE
<223>  synthetic

<400>  201

Val Val Leu Val Ser Leu Gln Ser Gly Tyr Leu Phe Ile Gln Thr Asp
1               5                   10                  15

Lys

<210>  202
<211>  12
<212>  PRT
<213>  Homo sapiens

<220>
<221>  MISC_FEATURE
<223>  synthetic

<400>  202

Thr Ile Tyr Thr Pro Gly Ser Thr Val Leu Tyr Arg
1               5                   10

<210>  203
<211>  7
<212>  PRT
<213>  Homo sapiens

<220>
<221>  MISC_FEATURE
<223>  synthetic

<400>  203

Ile Phe Thr Val Asn His Lys
1               5

<210>  204
<211>  6
<212>  PRT
<213>  Homo sapiens

<220>
<221>  MISC_FEATURE
<223>  synthetic

<400>  204

Leu Leu Pro Val Gly Arg
1               5

<210>  205
<211>  15
<212>  PRT
<213>  Homo sapiens

<220>
<221>  MISC_FEATURE
<223>  synthetic

<400>  205

Thr Val Met Val Asn Ile Glu Asn Pro Glu Gly Ile Pro Val Lys
1               5                   10                  15

<210>  206
<211>  29
<212>  PRT
<213>  Homo sapiens

<220>
<221>  MISC_FEATURE
<223>  synthetic

<400>  206

Gln Asp Ser Leu Ser Ser Gln Asn Gln Leu Gly Val Leu Pro Leu Ser
1               5                   10                  15

Trp Asp Ile Pro Glu Leu Val Asn Met Gly Gln Trp Lys
            20                  25

<210>  207
<211>  18
<212>  PRT
<213>  Homo sapiens

<220>
<221>  MISC_FEATURE
<223>  synthetic

<400>  207

Ala Tyr Tyr Glu Asn Ser Pro Gln Gln Val Phe Ser Thr Glu Phe Glu
1               5                   10                  15

Val Lys

<210>  208
<211>  16
<212>  PRT
<213>  Homo sapiens

<220>
<221>  MISC_FEATURE
<223>  synthetic

<400>  208

Glu Tyr Val Leu Pro Ser Phe Glu Val Ile Val Glu Pro Thr Glu Lys
1               5                   10                  15

<210>  209
<211>  13
<212>  PRT
<213>  Homo sapiens

<220>
<221>  MISC_FEATURE
<223>  synthetic

<400>  209

Ser Pro Met Tyr Ser Ile Ile Thr Pro Asn Ile Leu Arg
1               5                   10

<210>  210
<211>  8
<212>  PRT
<213>  Homo sapiens

<220>
<221>  MISC_FEATURE
<223>  synthetic

<400>  210

Phe Tyr Tyr Ile Tyr Asn Glu Lys
1               5

<210>  211

<211>  9
<212>  PRT
<213>  Homo sapiens

<220>
<221>  MISC_FEATURE
<223>  synthetic

<400>  211

Gly Leu Glu Val Thr Ile Thr Ala Arg
1               5

<210>  212
<211>  5
<212>  PRT
<213>  Homo sapiens

<220>
<221>  MISC_FEATURE
<223>  synthetic

<400>  212

Phe Leu Tyr Gly Lys
1               5

<210>  213
<211>  17
<212>  PRT
<213>  Homo sapiens

<220>
<221>  MISC_FEATURE
<223>  synthetic

<400>  213

Val Glu Gly Thr Ala Phe Val Ile Phe Gly Ile Gln Asp Gly Glu Gln
1               5                   10                  15

Arg

<210>  214
<211>  14
<212>  PRT
<213>  Homo sapiens

<220>
<221>  MISC_FEATURE
<223>  synthetic

<400>  214

Ile Pro Ile Glu Asp Gly Ser Gly Glu Val Val Leu Ser Arg
1               5                   10

<210>  215
<211>  10
<212>  PRT
<213>  Homo sapiens

<220>
<221>  MISC_FEATURE
<223>  synthetic

<400>  215

Val Leu Leu Asp Gly Val Gln Asn Pro Arg
1               5                   10

<210>  216
<211>  7
<212>  PRT
<213>  Homo sapiens

<220>
<221>  MISC_FEATURE
<223>  synthetic

<400>  216

Ala Glu Asp Leu Val Gly Lys
1               5

<210>  217
<211>  21
<212>  PRT
<213>  Homo sapiens

<220>
<221>  MISC_FEATURE
<223>  synthetic

<400>  217

Ser Leu Tyr Val Ser Ala Thr Val Ile Leu His Ser Gly Ser Asp Met
1               5                   10                  15

Val Gln Ala Glu Arg
            20

<210>  218
<211>  16
<212>  PRT
<213>  Homo sapiens

<220>

<221>  MISC_FEATURE
<223>  synthetic

<400>  218

Ser Gly Ile Pro Ile Val Thr Ser Pro Tyr Gln Ile His Phe Thr Lys
1               5                   10                  15


<210>  219
<211>  24
<212>  PRT
<213>  Homo sapiens


<220>
<221>  MISC_FEATURE
<223>  synthetic

<400>  219

Tyr Phe Lys Pro Gly Met Pro Phe Asp Leu Met Val Phe Val Thr Asn
1               5                   10                  15


Pro Asp Gly Ser Pro Ala Tyr Arg
            20


<210>  220
<211>  30
<212>  PRT
<213>  Homo sapiens


<220>
<221>  MISC_FEATURE
<223>  synthetic

<400>  220

Leu Glu Ser Glu Glu Thr Met Val Leu Glu Ala His Asp Ala Gln Gly
1               5                   10                  15


Asp Val Pro Val Thr Val Thr Val His Asp Phe Pro Gly Lys
            20                  25                  30


<210>  221
<211>  22
<212>  PRT
<213>  Homo sapiens


<220>
<221>  MISC_FEATURE
<223>  synthetic

<400>  221

Val Pro Val Ala Val Gln Gly Glu Asp Thr Val Gln Ser Leu Thr Gln
1               5                   10                  15

Gly Asp Gly Val Ala Lys
                20

<210>  222
<211>  17
<212>  PRT
<213>  Homo sapiens

<220>
<221>  MISC_FEATURE
<223>  synthetic

<400>  222

Leu Ser Ile Asn Thr His Pro Ser Gln Lys Pro Leu Ser Ile Thr Val
1               5                   10                  15

Arg

<210>  223
<211>  11
<212>  PRT
<213>  Homo sapiens

<220>
<221>  MISC_FEATURE
<223>  synthetic

<400>  223

Gln Glu Leu Ser Glu Ala Glu Gln Ala Thr Arg
1               5                   10

<210>  224
<211>  23
<212>  PRT
<213>  Homo sapiens

<220>
<221>  MISC_FEATURE
<223>  synthetic

<400>  224

Thr Met Gln Ala Leu Pro Tyr Ser Thr Val Gly Asn Ser Asn Asn Tyr
1               5                   10                  15

Leu His Leu Ser Val Leu Arg
                20

<210>  225

<211>  16
<212>  PRT
<213>  Homo sapiens

<220>
<221>  MISC_FEATURE
<223>  synthetic

<400>  225

Thr Glu Leu Arg Pro Gly Glu Thr Leu Asn Val Asn Phe Leu Leu Arg
1               5                   10                  15

<210>  226
<211>  5
<212>  PRT
<213>  Homo sapiens

<220>
<221>  MISC_FEATURE
<223>  synthetic

<400>  226

Ala His Glu Ala Lys
1               5

<210>  227
<211>  9
<212>  PRT
<213>  Homo sapiens

<220>
<221>  MISC_FEATURE
<223>  synthetic

<400>  227

Tyr Tyr Thr Tyr Leu Ile Met Asn Lys
1               5

<210>  228
<211>  22
<212>  PRT
<213>  Homo sapiens

<220>
<221>  MISC_FEATURE
<223>  synthetic

<400>  228

Glu Pro Gly Gln Asp Leu Val Val Leu Pro Leu Ser Ile Thr Thr Asp
1               5                   10                  15

Phe Ile Pro Ser Phe Arg
            20

<210>  229
<211>  14
<212>  PRT
<213>  Homo sapiens

<220>
<221>  MISC_FEATURE
<223>  synthetic

<400>  229

Leu Val Ala Tyr Tyr Thr Leu Ile Gly Ala Ser Gly Gln Arg
1               5                   10

<210>  230
<211>  12
<212>  PRT
<213>  Homo sapiens

<220>
<221>  MISC_FEATURE
<223>  synthetic

<400>  230

Glu Val Val Ala Asp Ser Val Trp Val Asp Val Lys
1               5                   10

<210>  231
<211>  10
<212>  PRT
<213>  Homo sapiens

<220>
<221>  MISC_FEATURE
<223>  synthetic

<400>  231

Asp Ser Cys Val Gly Ser Leu Val Val Lys
1               5                   10

<210>  232
<211>  10
<212>  PRT
<213>  Homo sapiens

<220>
<221>  MISC_FEATURE
<223>  synthetic

<400>  232

Asp Ser Cys Val Gly Ser Leu Val Val Lys
1               5                   10

<210>  233
<211>  11
<212>  PRT
<213>  Homo sapiens

<220>
<221>  MISC_FEATURE
<223>  synthetic

<400>  233

Gln Pro Val Pro Gly Gln Gln Met Thr Leu Lys
1               5                   10

<210>  234
<211>  8
<212>  PRT
<213>  Homo sapiens

<220>
<221>  MISC_FEATURE
<223>  synthetic

<400>  234

Ile Glu Gly Asp His Gly Ala Arg
1               5

<210>  235
<211>  7
<212>  PRT
<213>  Homo sapiens

<220>
<221>  MISC_FEATURE
<223>  synthetic

<400>  235

Ile Trp Asp Val Val Glu Lys
1               5

<210>  236
<211>  11
<212>  PRT
<213>  Homo sapiens

<220>
<221>  MISC_FEATURE
<223>  synthetic

<400>  236

Ala Asp Ile Gly Cys Thr Pro Gly Ser Gly Lys
1               5                   10


<210>  237
<211>  24
<212>  PRT
<213>  Homo sapiens


<220>
<221>  MISC_FEATURE
<223>  synthetic

<400>  237

Asp Tyr Ala Gly Val Phe Ser Asp Ala Gly Leu Thr Phe Thr Ser Ser
1               5                   10                  15


Ser Gly Gln Gln Thr Ala Gln Arg
            20


<210>  238
<211>  10
<212>  PRT
<213>  Homo sapiens


<220>
<221>  MISC_FEATURE
<223>  synthetic

<400>  238

Ala Glu Leu Gln Cys Pro Gln Pro Ala Ala
1               5                   10


<210>  239
<211>  10
<212>  PRT
<213>  Homo sapiens


<220>
<221>  MISC_FEATURE
<223>  synthetic

<400>  239

Ala Glu Leu Gln Cys Pro Gln Pro Ala Ala
1               5                   10


<210>  240
<211>  7
<212>  PRT
<213>  Homo sapiens

<220>
<221>  MISC_FEATURE
<223>  synthetic

<400>  240

Leu Val Leu Ser Ser Glu Lys
1               5


<210>  241
<211>  21
<212>  PRT
<213>  Homo sapiens


<220>
<221>  MISC_FEATURE
<223>  synthetic

<400>  241

Thr Val Leu Thr Pro Ala Thr Asn His Met Gly Asn Val Thr Phe Thr
1               5                   10                  15


Ile Pro Ala Asn Arg
            20


<210>  242
<211>  16
<212>  PRT
<213>  Homo sapiens


<220>
<221>  MISC_FEATURE
<223>  synthetic

<400>  242

Ser Asn Leu Asp Glu Asp Ile Ile Ala Glu Glu Asn Ile Val Ser Arg
1               5                   10                  15


<210>  243
<211>  15
<212>  PRT
<213>  Homo sapiens


<220>
<221>  MISC_FEATURE
<223>  synthetic

<400>  243

Ser Glu Phe Pro Glu Ser Trp Leu Trp Asn Val Glu Asp Leu Lys
1               5                   10                  15

<210>  244
<211>  4
<212>  PRT
<213>  Homo sapiens

<220>
<221>  MISC_FEATURE
<223>  synthetic

<400>  244

Glu Pro Pro Lys
1

<210>  245
<211>  6
<212>  PRT
<213>  Homo sapiens

<220>
<221>  MISC_FEATURE
<223>  synthetic

<400>  245

Asn Gly Ile Ser Thr Lys
1               5

<210>  246
<211>  16
<212>  PRT
<213>  Homo sapiens

<220>
<221>  MISC_FEATURE
<223>  synthetic

<400>  246

Asp Ser Ile Thr Thr Trp Glu Ile Leu Ala Val Ser Met Ser Asp Lys
1               5                   10                  15

<210>  247
<211>  21
<212>  PRT
<213>  Homo sapiens

<220>
<221>  MISC_FEATURE
<223>  synthetic

<400>  247

Gly Ile Cys Val Ala Asp Pro Phe Glu Val Thr Val Met Gln Asp Phe
1               5                   10                  15

Phe Ile Asp Leu Arg
            20

<210>  248
<211>  21
<212>  PRT
<213>  Homo sapiens

<220>
<221>  MISC_FEATURE
<223>  synthetic

<400>  248

Gly Ile Cys Val Ala Asp Pro Phe Glu Val Thr Val Met Gln Asp Phe
1               5                   10                  15

Phe Ile Asp Leu Arg
            20

<210>  249
<211>  7
<212>  PRT
<213>  Homo sapiens

<220>
<221>  MISC_FEATURE
<223>  synthetic

<400>  249

Leu Pro Tyr Ser Val Val Arg
1               5

<210>  250
<211>  7
<212>  PRT
<213>  Homo sapiens

<220>
<221>  MISC_FEATURE
<223>  synthetic

<400>  250

Asn Glu Gln Val Glu Ile Arg
1               5

<210>  251
<211>  7
<212>  PRT
<213>  Homo sapiens

<220>
<221>  MISC_FEATURE
<223>  synthetic

<400>  251

Ala Val Leu Tyr Asn Tyr Arg
1               5


<210>  252
<211>  6
<212>  PRT
<213>  Homo sapiens


<220>
<221>  MISC_FEATURE
<223>  synthetic

<400>  252

Gln Asn Gln Glu Leu Lys
1               5


<210>  253
<211>  16
<212>  PRT
<213>  Homo sapiens


<220>
<221>  MISC_FEATURE
<223>  synthetic

<400>  253

Val Glu Leu Leu His Asn Pro Ala Phe Cys Ser Leu Ala Thr Thr Lys
1               5                   10                  15


<210>  254
<211>  16
<212>  PRT
<213>  Homo sapiens


<220>
<221>  MISC_FEATURE
<223>  synthetic

<400>  254

Val Glu Leu Leu His Asn Pro Ala Phe Cys Ser Leu Ala Thr Thr Lys
1               5                   10                  15


<210>  255
<211>  9
<212>  PRT
<213>  Homo sapiens

<220>
<221>  MISC_FEATURE
<223>  synthetic

<400>  255

Thr Gly Leu Gln Glu Val Glu Val Lys
1               5


<210>  256
<211>  13
<212>  PRT
<213>  Homo sapiens


<220>
<221>  MISC_FEATURE
<223>  synthetic

<400>  256

Ala Ala Val Tyr His His Phe Ile Ser Asp Gly Val Arg
1               5                   10


<210>  257
<211>  5
<212>  PRT
<213>  Homo sapiens


<220>
<221>  MISC_FEATURE
<223>  synthetic

<400>  257

Thr Val Ala Val Arg
1               5


<210>  258
<211>  6
<212>  PRT
<213>  Homo sapiens


<220>
<221>  MISC_FEATURE
<223>  synthetic

<400>  258

Thr Leu Asp Pro Glu Arg
1               5


<210>  259
<211>  5
<212>  PRT
<213>  Homo sapiens

<220>
<221>  MISC_FEATURE
<223>  synthetic

<400>  259

Ser Glu Glu Thr Lys
1               5


<210>  260
<211>  12
<212>  PRT
<213>  Homo sapiens


<220>
<221>  MISC_FEATURE
<223>  synthetic

<400>  260

Glu Asn Glu Gly Phe Thr Val Thr Ala Glu Gly Lys
1               5                   10


<210>  261
<211>  14
<212>  PRT
<213>  Homo sapiens


<220>
<221>  MISC_FEATURE
<223>  synthetic

<400>  261

Gly Gln Gly Thr Leu Ser Val Val Thr Met Tyr His Ala Lys
1               5                   10


<210>  262
<211>  7
<212>  PRT
<213>  Homo sapiens


<220>
<221>  MISC_FEATURE
<223>  synthetic

<400>  262

Asp Gln Leu Thr Cys Asn Lys
1               5


<210>  263
<211>  7
<212>  PRT

<213>  Homo sapiens

<220>
<221>  MISC_FEATURE
<223>  synthetic

<400>  263

Asp Gln Leu Thr Cys Asn Lys
1               5

<210>  264
<211>  4
<212>  PRT
<213>  Homo sapiens

<220>
<221>  MISC_FEATURE
<223>  synthetic

<400>  264

Phe Asp Leu Lys
1

<210>  265
<211>  11
<212>  PRT
<213>  Homo sapiens

<220>
<221>  MISC_FEATURE
<223>  synthetic

<400>  265

Val Thr Ile Lys Pro Ala Pro Glu Thr Glu Lys
1               5                   10

<210>  266
<211>  6
<212>  PRT
<213>  Homo sapiens

<220>
<221>  MISC_FEATURE
<223>  synthetic

<400>  266

Arg Pro Gln Asp Ala Lys
1               5

<210>  267
<211>  10

<212>  PRT
<213>  Homo sapiens

<220>
<221>  MISC_FEATURE
<223>  synthetic

<400>  267

Asn Thr Met Ile Leu Glu Ile Cys Thr Arg
1               5                   10

<210>  268
<211>  10
<212>  PRT
<213>  Homo sapiens

<220>
<221>  MISC_FEATURE
<223>  synthetic

<400>  268

Asn Thr Met Ile Leu Glu Ile Cys Thr Arg
1               5                   10

<210>  269
<211>  26
<212>  PRT
<213>  Homo sapiens

<220>
<221>  MISC_FEATURE
<223>  synthetic

<400>  269

Gly Asp Gln Asp Ala Thr Met Ser Ile Leu Asp Ile Ser Met Met Thr
1               5                   10                  15

Gly Phe Ala Pro Asp Thr Asp Asp Leu Lys
            20                  25

<210>  270
<211>  8
<212>  PRT
<213>  Homo sapiens

<220>
<221>  MISC_FEATURE
<223>  synthetic

<400>  270

Gln Leu Ala Asn Gly Val Asp Arg

1                   5

<210>  271
<211>  4
<212>  PRT
<213>  Homo sapiens

<220>
<221>  MISC_FEATURE
<223>  synthetic

<400>  271

Tyr Ile Ser Lys
1

<210>  272
<211>  5
<212>  PRT
<213>  Homo sapiens

<220>
<221>  MISC_FEATURE
<223>  synthetic

<400>  272

Tyr Glu Leu Asp Lys
1                   5

<210>  273
<211>  5
<212>  PRT
<213>  Homo sapiens

<220>
<221>  MISC_FEATURE
<223>  synthetic

<400>  273

Ala Phe Ser Asp Arg
1                   5

<210>  274
<211>  9
<212>  PRT
<213>  Homo sapiens

<220>
<221>  MISC_FEATURE
<223>  synthetic

<400>  274

Asn Thr Leu Ile Ile Tyr Leu Asp Lys
1               5

<210>  275
<211>  12
<212>  PRT
<213>  Homo sapiens

<220>
<221>  MISC_FEATURE
<223>  synthetic

<400>  275

Val Ser His Ser Glu Asp Asp Cys Leu Ala Phe Lys
1               5                   10

<210>  276
<211>  12
<212>  PRT
<213>  Homo sapiens

<220>
<221>  MISC_FEATURE
<223>  synthetic

<400>  276

Val Ser His Ser Glu Asp Asp Cys Leu Ala Phe Lys
1               5                   10

<210>  277
<211>  16
<212>  PRT
<213>  Homo sapiens

<220>
<221>  MISC_FEATURE
<223>  synthetic

<400>  277

Val His Gln Tyr Phe Asn Val Glu Leu Ile Gln Pro Gly Ala Val Lys
1               5                   10                  15

<210>  278
<211>  13
<212>  PRT
<213>  Homo sapiens

<220>
<221>  MISC_FEATURE
<223>  synthetic

<400>  278

Val Tyr Ala Tyr Tyr Asn Leu Glu Glu Ser Cys Thr Arg
1               5                   10

<210>  279
<211>  13
<212>  PRT
<213>  Homo sapiens

<220>
<221>  MISC_FEATURE
<223>  synthetic

<400>  279

Val Tyr Ala Tyr Tyr Asn Leu Glu Glu Ser Cys Thr Arg
1               5                   10

<210>  280
<211>  6
<212>  PRT
<213>  Homo sapiens

<220>
<221>  MISC_FEATURE
<223>  synthetic

<400>  280

Phe Tyr His Pro Glu Lys
1               5

<210>  281
<211>  6
<212>  PRT
<213>  Homo sapiens

<220>
<221>  MISC_FEATURE
<223>  synthetic

<400>  281

Val Thr Leu Glu Glu Arg
1               5

<210>  282
<211>  11
<212>  PRT
<213>  Homo sapiens

<220>
<221>  MISC_FEATURE
<223>  synthetic

<400>  282

Ala Cys Glu Pro Gly Val Asp Tyr Val Tyr Lys
1               5                   10

<210>  283
<211>  19
<212>  PRT
<213>  Homo sapiens

<220>
<221>  MISC_FEATURE
<223>  synthetic

<400>  283

Val Gln Leu Ser Asn Asp Phe Asp Glu Tyr Ile Met Ala Ile Glu Gln
1               5                   10                  15

Thr Ile Lys

<210>  284
<211>  12
<212>  PRT
<213>  Homo sapiens

<220>
<221>  MISC_FEATURE
<223>  synthetic

<400>  284

Ser Gly Ser Asp Glu Val Gln Val Gly Gln Gln Arg
1               5                   10

<210>  285
<211>  7
<212>  PRT
<213>  Homo sapiens

<220>
<221>  MISC_FEATURE
<223>  synthetic

<400>  285

Thr Phe Ile Ser Pro Ile Lys
1               5

<210>  286
<211>  4
<212>  PRT
<213>  Homo sapiens

<220>
<221>  MISC_FEATURE
<223>  synthetic

<400>  286

Glu Ala Leu Lys
1


<210>  287
<211>  4
<212>  PRT
<213>  Homo sapiens


<220>
<221>  MISC_FEATURE
<223>  synthetic

<400>  287

Leu Glu Glu Lys
1


<210>  288
<211>  24
<212>  PRT
<213>  Homo sapiens


<220>
<221>  MISC_FEATURE
<223>  synthetic

<400>  288

His Tyr Leu Met Trp Gly Leu Ser Ser Asp Phe Trp Gly Glu Lys Pro
1               5                   10                  15


Asn Leu Ser Tyr Ile Ile Gly Lys
            20


<210>  289
<211>  20
<212>  PRT
<213>  Homo sapiens


<220>
<221>  MISC_FEATURE
<223>  synthetic

<400>  289

Asp Thr Trp Val Glu His Trp Pro Glu Glu Asp Glu Cys Gln Asp Glu
1               5                   10                  15

Glu Asn Gln Lys
            20

<210>  290
<211>  20
<212>  PRT
<213>  Homo sapiens

<220>
<221>  MISC_FEATURE
<223>  synthetic

<400>  290

Asp Thr Trp Val Glu His Trp Pro Glu Glu Asp Glu Cys Gln Asp Glu
1               5                   10                  15

Glu Asn Gln Lys
            20

## Description

The present invention is related to a method for differential diagnoses of hereditary angioedema in a subject, and a kit suitable for use in differential diagnoses of hereditary angioedema.

Hereditary angioedema is a rare inherited disorder characterized by recurrent episodes of the accumulation of fluids outside of the blood vessels, blocking the normal flow of blood or lymphatic fluid and causing rapid swelling of tissues in the hands, feet, limbs, face, intestinal tract, or airway. Usually, this swelling is not accompanied by itching, as it might be with an allergic reaction. Swelling of the gastrointestinal tract leads to cramping. Swelling of the airway may lead to obstruction, a potentially very serious complication. These symptoms develop as the result of deficiency or improper functioning of certain proteins that help to maintain the normal flow of fluids through very small blood vessels, i.e. capillaries. In some cases, fluid may accumulate in other internal organs.

The severity of the disease varies greatly among affected individuals. There are three main types of hereditary angioedema, namely Type I, Type II and Type III, with type I being the most common form. Both hereditary angioedema Type I and II are caused by a mutation in the SERPING1 gene that makes the C1 inhibitor protein, which normally suppresses activation of the complement system, while type III is often due to a mutation of the factor XII gene.

Hereditary angioedema is inherited as an autosomal dominant trait. The mutant gene can be inherited from either parent, or can be the result of a spontaneous new mutation in the affected individual.

Treatment of patients with acute attacks includes administration of a plasma derived C1 esterase inhibitor ("Berinert®", CSL Behring), a kallikrein inhibitor ("Kalbitor®", Dyax Corporation) or a bradykinin antagonist ("Firazyr®", Shire). Treatment of patients for long-term prophylaxis include administration of a C1 esterase inhibitor such as "Cinryze®" (Viropharma) and or a 17-α-alkylated androgen such as Danazol (available under the brand names "Danatrol", "Danocrine", "Danol", and "Danoval")

Hereditary angioedema may be diagnosed by measuring C1-INH levels using either a chromogenic assay or a complex ELISA. Such laboratory tests, i.e. chromogenic assay or a complex ELISA, are nonspecific, time consuming and demand high levels or resources such a biological sample, time and laboratory materials.

The problem underlying the present invention is the provision of a method and means for differential diagnosis of hereditary angioedema.

These and other problems are solved by the subject matter of the attached independent claims. Preferred embodiments may be taken from the attached dependent claims.

More specifically, the problem underlying the present invention is solved in a first aspect by a method for differential diagnosis of hereditary angioedema, wherein the method comprises determining the level of C4 protein, C1-INH protein and C1q protein in a sample from a subject, wherein the sample is a dried blood spot sample and wherein the level is determined by mass spectrometry.

The problem underlying the present invention is solved in a second aspect by a kit suitable for use in a method for differential diagnosis of hereditary angioedema, preferably a method for differential diagnosis of hereditary angioedema according to the first aspect, wherein the kit comprises at least one element selected from the group comprising an interaction partner of one biomarker, one biomarker, instructions of use for the kit, and one or more container, wherein the biomarker is selected from the group comprising C4 protein, a fragment peptide of C4 protein, C1-INH protein, a fragment peptide of C1-INH protein, C1q protein and a fragment peptide of C1q.

The present inventor has surprisingly identified a set of biomarkers which is useful in differently diagnosis of hereditary angioedema. Such set of biomarkers comprises (a) C4 protein or a C4 fragment peptide, (b) C1-INH protein or a C1-INH fragment peptide and (c) C1q protein or a C1q fragment peptide.

In accordance with each and any aspect of the present invention, the biomarker is selected from the group comprising C4 protein, a peptide derived from C4 protein which is, in an embodiment, a C4 fragment peptide, C1-INH protein, a peptide derived from C1-INH protein which is, in an embodiment, a C1-INH fragment peptide, a C1q protein, and a peptide derived from C1q protein which is, in an embodiment, a C1q fragment peptide. It is thus within the present invention that in the practicing of the various methods of the present invention including any aspect and embodiment thereof, the level of C4 protein, C1-INH protein and/or C1q protein is determined. More specifically, it is within the present invention that in the practicing of the various methods of the present invention including any aspect and embodiment thereof, the level of C4 protein, C1-INH protein and C1q protein is determined.

In an embodiment of each and any aspect of the present invention, a peptide derived from C1q protein is a peptide obtained or obtainable upon enzymatic digestion of C1q protein, preferably digestion of C1q protein by tryptic digestion of C1q protein. In an embodiment such peptide is not chemically converted, transformed or derivatized.

Subcomponent C1q of the complement system binds to immunoglobulin complexes with resulting serial activation of C1r (enzyme), C1s (proenzyme), and the other 8 components of complement. C1q is composed of 3 different species of chains, called A, B and C. Preferably any reference herein to C1q or protein C1q refers to both the subcomponent C1q and each and any of its individual chains A, B and C, unless indicated differently.

The amino acid sequence of chain A of C1q is as follows:

The amino acid sequence of chain B of C1q is as follows:

MMMKIPWGSIPVLMLLLLLGLIDISQAQLSCTGPPAIPGIPGIPGTPGPDGQPGTPGIKG

EKGLPGLAGDHGEFGEKGDPGIPGNPGKVGPKGPMGPKGGPGAPGAPGPKGESGDYKATQ

KIAFSATRTINVPLRRDQTIRFDHVITNMNNNYEPRSGKFTCKVPGLYYFTYHASSRGNL

CVNLMRGRERAQKVVTFCDYAYNTFQVTTGGMVLKLEQGENVFLQATDKNSLLGMEGANS

IFSGFLLFPDMEA

The amino acid sequence of chain C of C1q is as follows:

MDVGPSSLPHLGLKLLLLLLLLLPLRGQANTGCYGIPGMPGLPGAPGKDGYDGLPGPKGEP

GIPAIPGIRGPKGQKGEPGLPGHPGKNGPMGPPGMPGVPGPMGIPGEPGEEGRYKQKFQS

VFTVTRQTHQPPAPNSLIRFNAVLTNPQGDYDTSTGKFTCKVPGLYYFVYHASHTANLCV

LLYRSGVKVVTFCGHTSKTNQVNSGGVLLRLQVGEEVWLAVNDYYDMVGIQGSDSVFSGF

LLFPD

In an embodiment of each and any aspect of the present invention, the fragment peptide derived from C1q is one selected from the following table.

| **Peptide** | **Sequence (N-terminus -> C-terminus)** |
|---|---|
| C1q-A_[104-110] | GSPGNIK |
| C1q-A_[111-121] | DQPRP AFSAIR |
| C1q-A_[123-150] | NPPMGGNVVIFDTVITNQEEPYQNHSGR |
| C1q-A_[151-180] | FVCTVPGYYYFTFQVLSQWEICLSIVSSSR |
| C1q-A_[151-180]_Cys_CAM: 153,172 | FVCTVPGYYYFTFQVLSQWEICLSIVSSSR |
| C1q-A_[186-195] | SLGFCDTTNK |
| C1q-A_[186-195]_Cys_CAM: 190 | SLGFCDTTNK |
| C1q-A_[196-219] | GLFQVVSGGMVLQLQQGDQVWVEK |
| C1q-A_[224-245] | GHIYQGSEADSVFSGFLIFPSA |
| C1q-A_[23-27] | EDLCR |
| C1q-A_[28-32] | APDGK |
| C1q-A_[34-41] | GEAGRPGR |
| C1q-A_[49-60] | GEQGEPGAPGIR |
| C1q-A_[82-94] | VGYPGPSGPLGAR |
| C1q-B_[118-121] | ATQK |
| C1q-B_[137-141] | DQTIR |
| C1q-B_[160-163] | FTCK |
| C1q-B_[164-177] | VPGLYYFTYHASSR |
| C1q-B_[1 78-186] | GNLCVNLMR |
| C1q-B_[178-186]_Cys_CAM: 181 | GNLCVNLMR |
| C1q-B_[194-215] | VVTFCDYAYNTFQVTTGGMVLK |
| C1q-B_[194-215]_Cys_CAM: 198 | VVTFCDYAYNTFQVTTGGMVLK |
| C1q-B_[216-229] | LEQGENVFLQATDK |
| C1q-B_[230-253] | NSLLGMEGANSIFSGFLLFPDMEA |
| C1q-B_[28-59] | QLSCTGPP AIPGIPGIPGTPGPDGQPGTPGIK |
| C1q-B_[28-59]_Cys_CAM: 31 | QLSCTGPPAIPGIPGIPGTPGPDGQPGTPGIK |
| C1q-B_[63-77] | GLPGLAGDHGEFGEK |
| C1q-B_[78-88] | GDPGIPGNPGK |
| C1q-B_[93-98] | GPMGPK |
| C1q-B_[99-110] | GGPGAPGAPGPK |
| C1q-C_[118-126] | FQSVFTVTR |
| C1q-C_[127-139] | QTHQPPAPNSLIR |
| C1q-C_[140-157] | FNAVLTNPQGDYDTSTGK |
| C1q-C_[162-184] | VPGLYYFVYHASHTANLCVLLYR |
| C1q-C_[162-184]_Cys_CAM: 179 | VPGLYYFVYHASHTANLCVLLYR |
| C1q-C_[189-198] | WTFCGHTSK |
| C1q-C_[189-198]_Cys_CAM: 193 | WTFCGHTSK |
| C1q-C_[199-210] | TNQVNSGGVLLR |
| C1q-C_[211-245] | LQVGEEVWLAVNDYYDMVGIQGSDSVFSGFLLFPD |
| C1q-C_[29-47] | NTGCYGIPGMPGLPGAPGK |
| C1q-C_[29-47]_Cys_CAM: 32 | NTGCYGIPGMPGLPGAPGK |
| C1q-C_[48-57] | DGYDGLPGPK |
| C1q-C_[58-69] | GEPGIPAIPGIR |
| C1q-C_[76-86] | GEPGLPGHPGK |
| C1q-C_[87-113] | NGPMGPPGMPGVPGPMGIPGEPGEEGR |

A particularly preferred peptide derived from C1q is C1qB_[178-186] which is a compound having a molecular mass m/z of 510.26 as measured with a high resolution ion mobility mass spectrometer and can be measured with MRM-MS, and the amino acid sequence of which is as follows: GNLCVNLMR. This peptide is preferably used as a control and/or for distinguishing between HAE and AAE.

Another particularly preferred peptide derived from C1q is C1qB_[63-77] which is a compound having a molecular mass m/z of 742.36 or 495.24 as measured with a high resolution ion mobility mass spectrometer and can be measured with MRM-MS, and the amino acid sequence of which is as follows: GLPGLAGDHGEFGEK. This peptide is preferably used as a control and/or for distinguishing between HAE and AAE.

In accordance with each and any aspect of the present invention, in an embodiment of such each and any aspect of the present invention, apart from the biomarker the methods comprise the step of determining the presence and/or level of another biomarker, wherein the other biomarker C1-INH protein, a peptide derived from C1-INH, C4 protein and/or a peptide derived from C4 protein.

In an embodiment of each and any aspect of the present invention, a peptide derived from C1-INH protein is a peptide obtained or obtainable upon enzymatic digestion of C1-INH protein, preferably digestion of C1-INH protein by tryptic digestion of C1-INH protein. In an embodiment such peptide is not chemically converted, transformed or derivatized.

In an embodiment of each and any aspect of the present invention, a peptide derived from C4 protein is a peptide obtained or obtainable upon enzymatic digestion of C4 protein, preferably digestion of C4 protein by tryptic digestion of C4 protein. In an embodiment such peptide is not chemically converted, transformed or derivatized.

The amino acid sequence of C1-INH is as follows:

In an embodiment of each and any aspect of the present invention, the fragment peptide derived from C1-INH is one selected from the following table.

| **Peptide** | **Sequence (N-terminus -> C-terminus)** |
|---|---|
| SerpinG1_[202-211] | DFTCVHQALK |
| SerpinG1_[202-211]_Cys_CAM: 205 | DFTCVHQALK |
| SerpinG1_[212-216] | GFTTK |
| SerpinG1_[217-233] | GVTSVSQIFHSPDLAIR |
| SerpinG1_[23-40] | NPNATSSSSQDPESLQDR |
| SerpinG1_[234-241] | DTFVNASR |
| SerpinG1_[242-249] | TLYSSSPR |
| SerpinG1_[250-268] | VLSNNSDANLELINTWVAK |
| SerpinG1_[269-273] | NTNNK |
| SerpinG1_[274-276] | ISR |
| SerpinG1_[277-286] | LLDSLPSDTR |
| SerpinG1_[301-306] | TTFDPK |
| SerpinG1_[310-316] | MEPFHFK |
| SerpinG1_[322-328] | VPMMNSK |
| SerpinG1_[330-341] | YPVAHFIDQTLK |
| SerpinG1_[344-364] | VGQLQLSHNLSLVILVPQNLK |
| SerpinG1_[367-380] | LEDMEQALSPSVFK |
| SerpinG1_[381-385] | AIMEK |
| SerpinG1_[386-390] | LEMSK |
| SerpinG1_[391-400] | FQPTLLTLPR |
| SerpinG1_[403-415] | VTTSQDMLSIMEK |
| SerpinG1_[41-44] | GEGK |
| SerpinG1_[416-466] | LEFFDFSYDLNLCGLTEDPDLQVSAMQHQTVLELTETG VEAAAASAISVAR |
| SerpinG1_[467-487] | TLLVFEVQQPFLFVLWDQQHK |
| SerpinG1_[488-494] | FPVFMGR |
| SerpinG1_[495-499] | VYDPR |
| SerpinG1_[53-77] | MLFVEPILEVSSLPTTNSTTNSATK |

Particularly preferred fragment peptides derived from C1-INH are SerpinG1_[242-249] and SerpinG1_[391-400], whereby SerpinG1_[242-249] with MRM transition 455.74 → 696.33 is particularly preferred.

The amino acid sequence of C4 is as follows:

MRLLWGLIWASSFFTLSLQKPRLLLFSPSVVHLGVPLSVGVQLQDVPRGQVVKGSVFLRN
PSRNNVPCSPKVDFTLSSERDFALLSLQVPLKDAKSCGLHQLLRGPEVQLVAHSPWLKDS
LSRTTNIQGINLLFSSRRGHLFLQTDQPIYNPGQRVRYRVFALDQKMRPSTDTITVMVEN
SHGLRVRKKEVYMPSSIFQDDFVIPDISEPGTWKISARFSDGLESNSSTQFEVKKYVLPN
FEVKITPGKPYILTVPGHLDEMQLDIQARYIYGKPVQGVAYVRFGLLDEDGKKTFFRGLE
SQTKLVNGQSHISLSKAEFQDALEKLNMGITDLQGLRLYVAAAIIESPGGEMEEAELTSW
YFVSSPFSLDLSKTKRHLVPGAPFLLQALVREMSGSPASGIPVKVSATVSSPGSVPEVQD

IQQNTDGSGQVSIPIIIPQTISELQLSVSAGSPHPAIARLTVAAPPSGGPGFLSIERPDS
RPPRVGDTLNLNLRAVGSGATFSHYYYMILSRGQIVFMNREPKRTLTSVSVFVDHHLAPS
FYFVAFYYHGDHPVANSLRVDVQAGACEGKLELSVDGAKQYRNGESVKLHLETDSLALVA
LGALDTALYAAGSKSHKPLNMGKVFEAMNSYDLGCGPGGGDSALQVFQAAGLAFSDGDQW
TLSRKRLSCPKEKTTRKKRNVNFQKAINEKLGQYASPTAKRCCQDGVTRLPMMRSCEQRA
ARVQQPDCREPFLSCCQFAESLRKKSRDKGQAGLQRALEILQEEDLIDEDDIPVRSFFPE
NWLWRVETVDRFQILTLWLPDSLTTWEIHGLSLSKTKGLCVATPVQLRVFREFHLHLRLP
MSVRRFEQLELRPVLYNYLDKNLTVSVHVSPVEGLCLAGGGGLAQQVLVPAGSARPVAFS
VVPTAAAAVSLKVVARGSFEFPVGDAVSKVLQIEKEGAIHREELVYELNPLDHRGRTLEI
PGNSDPNMIPDGDFNSYVRVTASDPLDTLGSEGALSPGGVASLLRLPRGCGEQTMIYLAP
TLAASRYLDKTEQWSTLPPETKDHAVDLIQKGYMRIQQFRKADGSYAAWLSRDSSTWLTA
FVLKVLSLAQEQVGGSPEKLQETSNWLLSQQQADGSFQDPCPVLDRSMQGGLVGNDETVA
LTAFVTIALHHGLAVFQDEGAEPLKQRVEASISKANSFLGEKASAGLLGAHAAAITAYAL
TLTKAPVDLLGVAHNNLMAMAQETGDNLYWGSVTGSQSNAVSPTPAPRNPSDPMPQAPAL
WIETTAYALLHLLLHEGKAEMADQASAWLTRQGSFQGGFRSTQDTVIALDALSAYWIASH
TTEERGLNVTLSSTGRNGFKSHALQLNNRQIRGLEEELQFSLGSKINVKVGGNSKGTLKV
LRTYNVLDMKNTTCQDLQIEVTVKGHVEYTMEANEDYEDYEYDELPAKDDPDAPLQPVTP
LQLFEGRRNRRRREAPKLTSLSDRYVSHFETEGPHVLLYFDSVPTSRECVGFEAVQEVPV
GLVQPASATLYDYYNPERRCSVFYGAPSKSRLLATLCSAEVCQCAEGKCPRQRRALERGL
QDEDGYRMKFACYYPRVEYGFQVKVLREDSRAAFRLFETKITQVLHFTKDVKAAANQMRN
FLVRASCRLRLEPGKEYLIMGLDGATYDLEGHPQYLLDSNSWIEEMPSERLCRSTRQRAA
CAQLNDFLQEYGTQGCQV

In an embodiment of each and any aspect of the present invention, the fragment peptide derived from C4 is one selected from the following table.

| **Peptide** | **Sequence (N-terminus -> C-terminus)** |
|---|---|
| C4Alpha_[1006-1008] | LPR |
| C4Alpha_[1009-1026] | GCGEQTMIYLAPTLAASR |
| C4Alpha_[1009-1026]_Cys_CAM: 1010 | GCGEQTMIYLAPTLAASR |
| C4Alpha_[1027-1030] | YLDK |
| C4Alpha_[1031-1042] | TEQWSTLPPETK |
| C4Alpha_[1043-1051] | DHAVDLIQK |
| C4Alpha_[1052-1055] | GYMR |
| C4Alpha_[1062-1072] | ADGSYAAWLSR |
| C4Alpha_[1073-1084] | GSSTWLTAFVLK |
| C4Alpha_[1085-1099] | VLSLAQEQVGGSPEK |
| C4Alpha_[1100-1126] | LQETSNWLLSQQQADGSFQDLSPVIHR |
| C4Alpha_[1168-1174] | VEASISK |
| C4Alpha_[1175-1182] | ASSFLGEK |
| C4Alpha_[1183-1204] | ASAGLLGAHAAAITAYALTLTK |
| C4Alpha_[1211-1248] | GVAHNNLMAMAQETGDNLYWGSVTGSQSNAV SPTPAPR |
| C4Alpha_[1249-1278] | NPSDPMPQAPALWIETTAYALLHLLLHEGK |
| C4Alpha_[1279-1291] | AEMADQAAAWLTR |
| C4Alpha_[1292-1300] | QGSFQGGFR |
| C4Alpha_[1301-1325] | STQDTVIALDALSAYWIASHTTEER |
| C4Alpha_[1326-1336] | GLNVTLSSTGR |
| C4Alpha_[1337-1340] | NGFK |
| C4Alpha_[1341-1349] | SHALQLNNR |
| C4Alpha_[1350-1352] | QIR |
| C4Alpha_[1353-1365] | GLEEELQFSLGSK |
| C4Alpha_[1370-1375] | VGGNSK |
| C4Alpha_[1383-1390] | TYNVLDMK |
| C4Alpha_[1391-1404] | NTTCQDLQIEVTVK |
| C4Alpha_[1391-1404]_Cys_CAM: 1394 | NTTCQDLQIEVTVK |
| C4Alpha_[1405-1428] | GHVEYTMEANEDYEDYEYDELPAK |
| C4Alpha_[1429-1446] | DDPDAPLQPVTPLQLFEG |
| C4Alpha_[680-685] | NVNFQK |
| C4Alpha_[686-690] | AINEK |
| C4Alpha_[691-700] | LGQYASPTAK |
| C4Alpha_[702-709]_Cys_CAM: 702, 703 | CCQDGVTR |
| C4Alpha_[710-714] | LPMMR |
| C4Alpha_[715-719]_Cys_CAM: 716 | SCEQR |
| C4Alpha_[723-729] | VQQPDCR |
| C4Alpha_[723-729]_Cys_CAM: 728 | VQQPDCR |
| C4Alpha_[730-743]_Cys_CAM: 735, 736 | EPFLSCCQFAESLR |
| C4Alpha_[750-756] | GQAGLQR |
| C4Alpha_[757-775] | ALEILQEEDLIDEDDIPVR |
| C4Alpha_[776-785] | SFFPENWLWR |
| C4Alpha_[786-791] | VETVDR |
| C4Alpha_[792-815] | FQILTLWLPDSLTTWEIHGLSLSK |
| C4Alpha_[818-828] | GLCVATPVQLR |
| C4Alpha_[818-828]_Cys_CAM: 820 | GLCVATPVQLR |
| C4Alpha_[832-838] | EFHLHLR |
| C4Alpha_[846-861] | FEQLELRPVLYNYLDK |
| C4Alpha_[862-912] | NLTVSVHVSPVEGLCLAGGGGLAQQVLVPAGSA RPVAFSVVPTAATAVSLK |
| C4Alpha_[862-912]_Cys_CAM: 876 | NLTVSVHVSPVEGLCLAGGGGLAQQVLVPAGSA RPVAFSVVPTAATAVSLK |
| C4Alpha_[913-916] | VVAR |
| C4Alpha_[917-929] | GSFEFPVGDAVSK |
| C4Alpha_[936-941] | EGAIHR |
| C4Alpha_[942-954] | EELVYELNPLDHR |
| C4Alpha_[957-979] | TLEIPGNSDPNMIPDGDFNSYVR |
| C4Alpha_[980-1005] | VTASDPLDTLGSEGALSPGGVASLLR |
| C4Beta_[105-118] | GPEVQLVAHSPWLK |
| C4Beta_[119-123] | DSLSR |
| C4Beta_[124-137] | TTNIQGINLLFSSR |
| C4Beta_[139-155] | GHLFLQTDQPIYNPGQR |
| C4Beta_[158-159] | YR |
| C4Beta_[160-166] | VFALDQK |
| C4Beta_[167-185] | MRPSTDTITVMVENSHGLR |
| C4Beta_[190-214] | EVYMPSSIFQDDFVIPDISEPGTWK |
| C4Beta_[219-234] | FSDGLESNSSTQFEVK |
| C4Beta_[23-48] | LLLFSPSVVHLGVPLSVGVQLQDVPR |
| C4Beta_[236-244] | YVLPNFEVK |
| C4Beta_[245-269] | ITPGKPYILTVPGHLDEMQLDIQAR |
| C4Beta_[270-283] | YIYGKPVQGVAYVR |
| C4Beta_[284-292] | FGLLDEDGK |
| C4Beta_[294-297] | TFFR |
| C4Beta_[298-304] | GLESQTK |
| C4Beta_[305-316] | LVNGQSHISLSK |
| C4Beta_[326-337] | LNMGITDLQGLR |
| C4Beta_[338-373] | LYVAAAIIESPGGEMEEAELTSWYFVSSPFSLDLS K |
| C4Beta_[392-404] | EMSGSPASGIPVK |
| C4Beta_[405-459] | VSATVSSPGSVPEVQDIQQNTDGSGQVSIPIIIPQTI SELQLSVSAGSPHPAIAR |
| C4Beta_[460-484] | LTVAAPPSGGPGFLSIERPDSRPPR |
| C4Beta_[485-494] | VGDTLNLNLR |
| C4Beta_[49-53] | GQVVK |
| C4Beta_[495-512] | AVGSGATFSHYYYMILSR |
| C4Beta_[513-520] | GQIVFMNR |
| C4Beta_[521-523] | EPK |
| C4Beta_[525-559] | TLTSVSVFVDHHLAPSFYFVAFYYHGDHPVANS LR |
| C4Beta_[560-570] | VDVQAGACEGK |
| C4Beta_[560-570]_Cys_CAM: 567 | VDVQAGACEGK |
| C4Beta_[571-579] | LELSVDGAK |
| C4Beta_[580-582] | QYR |
| C4Beta_[583-588] | NGESVK |
| C4Beta_[589-614] | LHLETDSLALVALGALDTALYAAGSK |
| C4Beta_[60-63] | NPSR |
| C4Beta_[615-623] | SHKPLNMGK |
| C4Beta_[624-664] | VFEAMNSYDLGCGPGGGDSALQVFQAAGLAFS |
| | DGDQWTLSR |
| C4Beta_[624-664]_Cys_CAM: 635 | VFEAMNSYDLGCGPGGGDSALQVFQAAGLAFS DGDQWTLSR |
| C4Beta_[64-71] | NNVPCSPK |
| C4Beta_[64-71]_Cys_CAM: 68 | NNVPCSPK |
| C4Beta_[667-671] | LSCPK |
| C4Beta_[667-671]_Cys_CAM: 669 | LSCPK |
| C4Beta_[72-80] | VDFTLSSER |
| C4Beta_[81-92] | DFALLSLQVPLK |
| C4Beta_[93-95] | DAK |
| C4Beta_[96-104] | SCGLHQLLR |
| C4Beta_[96-104]_Cys_CAM: 97 | SCGLHQLLR |
| C4Gamma_[1458-1465] | VVEEQESR |
| C4Gamma_[1466-1474] | VHYTVCIWR |
| C4Gamma_[1466-1474]_Cys_CAM: 1471 | VHYTVCIWR |
| C4Gamma_[1475-1477] | NGK |
| C4Gamma_[1478-1498] | VGLSGMAIADVTLLSGFHALR |
| C4Gamma_[1499-1503] | ADLEK |
| C4Gamma_[1504-1510] | LTSLSDR |
| C4Gamma_[1511-1533] | YVSHFETEGPHVLLYFDSVPTSR |
| C4Gamma_[1534-1564] | ECVGFEAVQEVPVGLVQPASATLYDYYNPER |
| C4Gamma_[1534-1564]_Cys_CAM: 1535 | ECVGFEAVQEVPVGLVQPASATLYDYYNPER |
| C4Gamma_[1566-1575] | CSVFYGAPSK |
| C4Gamma_[1566-1575]_Cys_CAM: 1566 | CSVFYGAPSK |
| C4Gamma_[1578-1594] | LLATLCSAEVCQCAEGK |
| C4Gamma_[1578-1594]_Cys_CAM: 1583, 1588, 159 0 | LLATLCSAEVCQCAEGK |
| C4Gamma_[1595-1597] | CPR |
| C4Gamma_[1595-1597]_Cys_CAM: 1595 | CPR |
| C4Gamma_[1601-1604] | ALER |
| C4Gamma_[1616-1622] | FACYYPR |
| C4Gamma_[1616-1622]_Cys_CAM: 1618 | FACYYPR |
| C4Gamma_[1623-1630] | VEYGFQVK |
| C4Gamma_[1631-1633] | VLR |
| C4Gamma_[638-1641] | AAFR |
| C4Gamma_[1642-1646] | LFETK |
| C4Gamma_[1656-1658] | DVK |
| C4Gamma_[1659-1665] | AAANQMR |
| C4Gamma_[1671-1674] | ASCR |
| C4Gamma_[1677-1681] | LEPGK |
| C4Gamma_[1682-1716] | EYLIMGLDGATYDLEGHPQYLLDSNSWIEEMPSE R |
| C4Gamma_[1720-1722] | STR |
| C4Gamma_[1725-1744] | AACAQLNDFLQEYGTQGCQV |
| C4Gamma_[1725-1744]_Cys_CAM: 1727, 1742 | AACAQLNDFLQEYGTQGCQV |

Particularly preferred fragment peptides derived from C4 are C4Beta[571-579], C4Alpha[680-685], C4Alpha[786-791], C4Beta[294-297], whereby C4Beta[571-579] with MRM transition 466.26 → 243.13 is particularly preferred.

In connection with each and any aspect of the present invention, C3 protein and/or a peptide derived from C3 protein may be used, preferably as an internal control for the proper functioning of the detection system, preferably of the analysis technique used for determining the level of the biomarker.

A peptide derived from C3 is a peptide obtained or obtainable upon enzymatic digestion of C3 protein, preferably digestion of C3 protein by tryptic digestion of C3 protein.

Component C3 of the complement system plays several important biologic roles in the classical, alternative, and lectin activation pathways, e.g., (1) formation of C3- and C5-convertases, both essential for the full activation of the system; (2) production of opsonins that enhance phagocytosis of microorganisms; (3) degranulation of mast cells and basophils medicated by the fragments C3a and C5a; (4) solubilization and clearance of C3b-bound immune complexes; (5) adjuvant function of fragments C3d and C3dg; and (6) clearance of apoptotic cells. Hereditary angioedema patients typically have normal C3 levels.

The amino acid sequence of C3 is as follows:

In an embodiment of each and any aspect of the present invention, the fragment peptide derived from C3 is one selected from the following table.

| **Peptide** | **Sequence (N-terminus -> C-terminus)** |
|---|---|
| C3Beta_[105-119] | FVTVQATFGTQVVEK |
| C3Beta_[120-136] | VVLVSLQSGYLFIQTDK |
| C3Beta_[137-148] | TIYTPGSTVLYR |
| C3Beta_[149-155] | IFTVNHK |
| C3Beta_[156-161] | LLPVGR |
| C3Beta_[162-176] | TVMVNIENPEGIPVK |
| C3Beta_[177-205] | QDSLSSQNQLGVLPLSWDIPELVNMGQWK |
| C3Beta_[208-225] | AYYENSPQQVFSTEFEVK |
| C3Beta_[226-241] | EYVLPSFEVIVEPTEK |
| C3Beta_[23-35] | SPMYSIITPNILR |
| C3Beta_[242-249] | FYYIYNEK |
| C3Beta_[250-258] | GLEVTITAR |
| C3Beta_[259-263] | FLYGK |
| C3Beta_[265-281] | VEGTAFVIFGIQDGEQR |
| C3Beta_[291-304] | IPIEDGSGEVVLSR |
| C3Beta_[306-315] | VLLDGVQNPR |
| C3Beta_[316-322] | AEDLVGK |
| C3Beta_[323-343] | SLYVSATVILHSGSDMVQAER |
| C3Beta_[344-359] | SGIPIVTSPYQIHFTK |
| C3Beta_[363-386] | YFKPGMPFDLMVFVTNPDGSPAYR |
| C3Beta_[36-65] | LESEETMVLEAHDAQGDVPVTVTVHDFPGK |
| C3Beta_[387-408] | VPVAVQGEDTVQSLTQGDGVAK |
| C3Beta_[409-425] | LSINTHPSQKPLSITVR |
| C3Beta_[429-439] | QELSEAEQATR |
| C3Beta_[440-462] | TMQALPYSTVGNSNNYLHLSVLR |
| C3Beta_[463-478] | TELRPGETLNVNFLLR |
| C3Beta_[479-481] | MDR |
| C3Beta_[482-486] | AHEAK |
| C3Beta_[489-497] | YYTYLIMNK |
| C3Beta_[500-502] | LLK |
| C3Beta_[503-505] | AGR |
| C3Beta_[506-508] | QVR |
| C3Beta_[509-530] | EPGQDLVVLPLSITTDFIPSFR |
| C3Beta_[531-544] | LVAYYTLIGASGQR |
| C3Beta_[545-556] | EVVADSVWVDVK |
| C3Beta_[557-566] | DSCVGSLVVK |
| C3Beta_[557-566]_Cys_CAM: 559 | DSCVGSLVVK |
| C3Beta_[574-584] | QPVPGQQMTLK |
| C3Beta_[585-592] | IEGDHGAR |
| C3Beta_[616-622] | IWDVVEK |
| C3Beta_[623-633] | ADIGCTPGSGK |
| C3Beta_[634-657] | DYAGVFSDAGLTFTSSSGQQTAQR |
| C3Beta_[658-667] | AELQCPQPAA |
| C3Beta_[658-667]_Cys_CAM: 662 | AELQCPQPAA |
| C3Beta_[67-73] | LVLSSEK |
| C3Beta_[74-94] | TVLTPATNHMGNVTFTIPANR |
| C3Beta_[95-97] | EFK |
| C3Beta_[98-100] | SEK |
| C3cAlpha1_[749-764] | SNLDEDIIAEENIVSR |
| C3cAlpha1_[765-779] | SEFPESWLWNVEDLK |
| C3eAlpha1_[780-783] | EPPK |
| C3cAlpha1_[784-789] | NGISTK |
| C3cAlpha1_[797-812] | DSITTWEILAVSMSDK |
| C3cAlpha1_[814-834] | GICVADPFEVTVMQDFFIDLR |
| C3cAlpha1_[814-834]_Cys_CAM: 816 | GICVADPFEVTVMQDFFIDLR |
| C3cAlpha1_[835-841] | LPYSVVR |
| C3cAlpha1_[842-848] | NEQVEIR |
| C3cAlpha1_[849-855] | AVLYNYR |
| C3cAlpha1_[856-861] | QNQELK |
| C3cAlpha1_[864-879] | VELLHNPAFCSLATTK |
| C3cAlpha1_[864-879]_Cys_CAM: 873 | VELLHNPAFCSLATTK |
| C3cAlpha1_[905-913] | TGLQEVEVK |
| C3cAlpha1_[914-926] | AAVYHHFISDGVR |
| C3cAlpha1_[938-940] | MNK |
| C3cAlpha1_[941-945] | TVAVR |
| C3cAlpha1_[946-951] | TLDPER |
| C3cAlpha1_[952-954] | LGR |
| C3cAlpha2_[1321-1325] | SEETK |
| C3cAlpha2_[1326-1337] | ENEGFTVTAEGK |
| C3cAlpha2_[1338-1351] | GQGTLSVVTMYHAK |
| C3cAlpha2_[1354-1360] | DQLTCNK |
| C3cAlpha2_[1354-1360]_Cys_CAM: 1358 | DQLTCNK |
| C3cAlpha2_[1361-1364] | FDLK |
| C3cAlpha2_[1365-1375] | VTIKPAPETEK |
| C3cAlpha2_[1376-1381] | RPQDAK |
| C3cAlpha2_[1382-1391] | NTMILEICTR |
| C3cAlpha2_[1382-1391]_Cys_CAM: 1389 | NTMILEICTR |
| C3cAlpha2_[1394-1419] | GDQDATMSILDISMMTGFAPDTDDLK |
| C3cAlpha2_[1420-1427] | QLANGVDR |
| C3cAlpha2_[1428-1431] | YISK |
| C3cAlpha2_[1432-1436] | YELDK |
| C3cAlpha2_[1437-1441] | AFSDR |
| C3cAlpha2_[1442-1450] | NTLIIYLDK |
| C3cAlpha2_[1451-1462] | VSHSEDDCLAFK |
| C3cAlpha2_[1451-1462]_Cys_CAM: 1458 | VSHSEDDCLAFK |
| C3cAlpha2_[1463-1478] | VHQYFNVELIQPGAVK |
| C3cAlpha2_[1479-1491] | VYAYYNLEESCTR |
| C3cAlpha2_[1479-1491LCys_CAM: 1489 | VYAYYNLEESCTR |
| C3cAlpha2_[1492-1497] | FYHPEK |
| C3cAlpha2_[1502-1504] | LNK |
| C3cAlpha2_[1505-1507] | LCR |
| C3cAlpha2_[1505-1507]_Cys_CAM: 1506 | LCR |
| C3cAlpha2_[1527-1532] | VTLEER |
| C3cAlpha2_[1533-1535] | LDK |
| C3cAlpha2_[1536-1546] | ACEPGVDYVYK |
| C3cAlpha2_[1536-1546]_Cys_CAM: 1537 | ACEPGVDYVYK |
| C3cAlpha2_[1552-1570] | VQLSNDFDEYIMAIEQTIK |
| C3cAlpha2_[1571-1582] | SGSDEVQVGQQR |
| C3cAlpha2_[1583-1589] | TFISPIK |
| C3cAlpha2_[1592-1595] | EALK |
| C3cAlpha2_[1596-1599] | LEEK |
| C3cAlpha2_[1601 -1624] | HYLMWGLSSDFWGEKPNLSYIIGK |
| C3cAlpha2_[1625-1644] | DTWVEHWPEEDECQDEENQK |
| C3cAlpha2_[1625-1644]_Cys_CAM: 1637 | DTWVEHWPEEDECQDEENQK |

A particularly preferred peptide derived from C3 is C3Beta_ [489-497] which is a compound having a molecular mass m/z of 604.81 as measured with a high resolution ion mobility mass spectrometer, MRM transition 604.8 → 327.22 and which can be measured with MRM-MS, and the amino acid sequence of which is as follows: YYTYLIMNK. Another preferred fragment peptide of C3 is C3cAlpha1_[814-834]_Cys_CAM: 816 with MRM transition 824.74 798.44.

Another particularly preferred peptide derived from C3 is C3cAlpha_[814-834]Cys_CAM816 which is a having a molecular mass m/z of 495.25 as measured with a high resolution ion mobility mass spectrometer and can be measured with MRM-MS, and the amino acid sequence of which is as follows: GICVADPFEVTVMQDFFIDLR. "CAM" refers to carbamidomethyl and is the result of the alkylation of the free SH-groups after cleavage of C3 into peptides.

As preferably used herein, a fragment peptide is a peptide of a protein generated by digestion, preferably complete digestion of the protein by a proteolytic enzyme.

It will be acknowledged that rather than using trypsin for the generation of a peptide from proteins C1q, C1-INH, C4 and C3 respectively, another proteolytic enzyme may be used, preferably the proteolytic enzyme is a protease or peptidase which, upon complete digestion of the protein, provides a mixture of peptides, wherein each species of the peptide is present only once. This ensures that there is a 1:1 stoichiometry between the protein and each and any peptide obtained by such complete digestion of the protein using the protease. In another embodiment, digestion reaction or protease is selected from the group comprising Arg-C, Asp-N, Asp-N (N-terminal Glu), BNPS or NCS/urea, Caspase-1, Caspase-10, Caspase-2, Caspase-3, Caspase-4, Caspase-5, Caspase-6, Caspase-7, Caspase-8, Caspase-9, Chymotrypsin, Chymotrypsin (low specificity), Clostripain, CNBr, CNBr (methyl-Cys), CNBr (with acids), Enterokinase, Factor Xa, Formic acid, Glu-C (AmAc buffer, Glu-C (Phos buffer), Granzyme B, HRV3C protease, Hydroxylamine, Iodosobenzoic acid, Lys-C, Lys-N, Lys-N (Cys modified), Mild acid hydrolysis, NBS (long exposure), NBS (short exposure), NTCB, Pancreatic elastase, Pepsin A, Pepsin A (low specificity), Prolyl endopeptidase, Proteinase K, TEV protease, Thermolysin, Thrombin

It will be further acknowledged by a person skilled in the art that although, in principle, all of the protein derived peptides are suitable for use in any method of any aspect of the present invention, the use of different peptides may be preferred depending on the technique used for the detection of the biomarker. Accordingly, in an embodiment of each any aspect of the invention, the biomarker is a peptide derived from any of proteins C1q, C1-INH and C4 which is particularly suitable for detection by means of mass spectrometry, particularly in case detection is made by mass spectrometry. Also accordingly, in an embodiment of each any aspect of the invention, the biomarker is a peptide derived from any of proteins C1q, C1-INH and C4 against which an antibody or a functional nucleic acid may be generated with the antibody and functional nucleic acid providing for a highly specific and/or highly selective detection and/or quantification of said protein, particularly in case detection is made by means of assay using such antibody or functional nucleic acid as an interaction partner of said peptide.

The term "hereditary angioedema" (HAE), to which it is also referred herein as "the disease", is a rare inherited disorder characterized by recurrent episodes of the accumulation of fluids outside of the blood vessels, blocking the normal flow of blood or lymphatic fluid and causing rapid swelling of tissues in the hands, feet, eyelids, lips, limbs, face, intestinal tract, airways and genitals. Usually, this swelling is not accompanied by itching, as it might be with an allergic reaction. Swelling of the gastrointestinal tract leads to cramping. Swelling of the airway may lead to obstruction, a potentially very serious complication. These symptoms develop as the result of deficiency or improper functioning of certain proteins that help to maintain the normal flow of fluids through very small blood vessels (capillaries).

In some cases, fluid may accumulate in other internal organs. The severity of the disease varies greatly among affected individuals. Edema may also occur in the mucous membranes that line the respiratory and digestive tracts, which is more common in people with hereditary angioedema than in those who have other forms of angioedema (i.e., acquired or traumatic). People with this disorder typically have areas of swelling that are hard and painful, not red and itchy (pruritic). A skin rash (urticaria) is rarely present.

The symptoms of hereditary angioedema may recur and can become more severe. Injury, severe pain, surgery, dental procedures, viral illness, and/or stress can trigger or worsen the recurring symptoms.

Symptoms associated with swelling in the digestive system (gastrointestinal tract) include nausea, vomiting, acute abdominal pain, and/or other signs of obstruction. Edema of the throat (pharynx) or voice-box (larynx) can result in pain, difficulty swallowing (dysphagia), difficulty speaking (dysphonia), noisy respiration (stridor), and potentially life-threatening asphyxiation.

There are three forms of hereditary angioedema, namely hereditary angioedema type I, hereditary angioedema type II and hereditary angioedema type III.

The most common form of the disorder is hereditary angioedema type I, which is the result of a deficiency of a protein known as complement component C1 esterase inhibitor. In hereditary angioedema type I, representing 85% of patients, serum levels of the C1 esterase inhibitor are less than 35% of normal. In type II, the levels are normal or elevated, but the protein is nonfunctional. The two types are clinically indistinguishable. Hereditary angioedema type III is caused by mutation in the gene encoding coagulation factor XII (F12; 610619) on chromosome 5q.

Hereditary angioedema is inherited as an autosomal dominant trait. The genetic defect underlying hereditary angioedema is a heterozygous mutation in the C1 esterase inhibitor gene (C1NH, SERPING1) on chromosome 11q. Patients with of hereditary angioedema type I appear to have a deletion of the C1 esterase inhibitor gene or a truncated transcript because of a stop codon, whereas patients with of hereditary angioedema type II have a single base substitution. The two forms are clinically indistinguishable. Mutations in the C1 esterase inhibitor gene associated with hereditary angioedema and of mutations in C1 esterase inhibitor gene tested in the diagnosis of hereditary angioedema are known to the person skilled in the art and can be retrieved from scientific papers using routine measures. Mutations in the C1 esterase inhibitor protein associated with hereditary angioedema and of mutations in C1 esterase inhibitor protein tested in the diagnosis of hereditary angioedema are known to the person skilled in the art and can be retrieved from scientific papers using routine measures. Known DNA changes in the C1 esterase inhibitor gene are c.550G>A, c.671T>A, c.551_685del, c.-191_51del / del of exon 1 and 2, c.1081C>T, c.106_107del and c.1397G>A.

In an embodiment of each and any aspect of the present invention, hereditary angioedema is hereditary angioedema type I.

The term "sample" as used herein means preferably a limited quantity of a subject's material, wherein said subject's material is part of or has been taken from a subject and/or a subject's body. Preferably, said material is selected from the group comprising body fluids such as blood, a blood product, urine, saliva, cerebrospinal fluid and lymph, as well as stool or any kind of tissue and or cell material being part of a subject and/or a subject's body. It will be acknowledged by a person skilled in the art that the presence of and/or a level of the biomarker of the invention in said sample is intended to be similar to and represent the presence and/or the level of the biomarker in a larger amount of that subject's material. More precisely and as an illustrative, non-limiting example, a level of the biomarker of the invention determined in a sample of, e.g., some ml of blood from a subject also represents a level of said biomarker in the blood of the subject's body. Furthermore, in an embodiment of the methods of each and any aspect of the invention, a sample from the subject comprises said subject's material in a form, for example processed, fixed and/or preserved such that said sample is suitable for use in the methods of each and any aspect of the invention, whereby such processing, fixing and/or preserving preferably does neither generate the biomarker, at least not unintentionally, which was not as such present in the blood of the patient. The subject's material in the sample may thus be diluted, for example with a solvent suitable for the method of each and any aspect of the invention such as methanol and/or water, may be dried, for example on a filter card, may be resolved after having been dried such, for example with a solvent suitable for the method of the invention such as methanol and/or water, or a substance may be added, wherein said substance prevents blood from coagulation such as for example EDTA or heparin.

A sample as preferably used in connection with each and any aspect of the present invention a sample as used in such methods is prepared from a primary source such as whole blood. Other samples include, but are not limited to serum samples and plasma samples.

In an embodiment of the various aspects of the invention the primary sample is whole blood which is, in an embodiment, processed such that it is collected on a dry blood filter card; preferably approximately 3µl of full blood are collected on a spot of said dry blood filter card having a diameter of 3 mm. A person skilled in the art will acknowledge that the exact volume thus collected may vary depending on the hematocrit of the specific patient.

In an embodiment of each and any aspect of the present invention where the sample is blood or dry blood spots or other liquids or tissues and wherein the biomarker is a peptide derived from C4 protein, C1q protein and/or from C1-INH protein, the sample may be processed as follows:
- extracting of blood components;
- subjecting the extract in situ to a reaction with reducing agent, preferably dithiothreitol (DDT), to reduce the disulfide bridges in the proteins and to an alkylation agent, preferably iodacetamide (IAA), to alkylate the free -SH groups;
- digesting the mixture into peptides, preferably by use of a protease, more preferably by the use of the protease trypsin; and
- analyzing the mixture containing peptide fragments of the proteins by mass spectrometry, preferably LC-mass spectrometry analysis, and more preferably in the presence of an internal standard.

In an embodiment of each and any aspect of the method of the invention wherein an internal standard is added to a or the sample, the internal standard may be added to the sample before or after the trypsin digestion step, i.e. the internal standard may be added into the sample immediately after the sample is taken from the subject, or may be added to the supernatant which is subjected to HPLC, as well as in between these points in time. It is within the skills of a person of the art to determine how and when an internal standard is to be added to the sample in order to achieve an accurate detection and determination of a level of the biomarker, wherein according to the present invention preferably the internal standard is added to a sample that contains the biomarker.

It will be acknowledged by a person skilled in the art that by said addition of internal standard, also referred to herein as IS, to the sample, i.e. spiking of the sample, to be subjected to such method according to the present invention, the concentration of IS in the sample is known and, e.g., by determining the area under the peak, i.e. the peak area, of the internal standard in, e.g., an HPLC-mass spectrometric chromatogram the relation between a peak area and a concentration of a substance, e.g. of IS, and/or the biomarker of the present invention is established and thus a means provided for determining the level of the biomarker in the sample. A person skilled in the art will further acknowledge that various molecules may be used as an IS. Nevertheless, an IS having a similar chemical structure compared to the molecule such as the biomarker is preferred. In a preferred embodiment, the molecule being the IS can be distinguished from the biomarker of the present invention. The latter applies in particular to those embodiments of each and any aspect of the present invention where the biomarker is a peptide derived C4 protein, C1-INH protein and/or C1q protein. In a further preferred embodiment of each and any aspect of the present invention, the IS is selected such that a molecule which is ideally not present or rare in nature, is bearing heavy isotopes (such as C13, N15 versions of the biomarker), comprising modified amino acids such as D-amino acids or + or - amino acids, or dextro peptides. In a preferred embodiment of each and any aspect of the present invention Leucine-Enkephaline is used as an internal standard which is not present as such in nature.

In an embodiment of the various aspects of the present invention where the internal standard is added to a sample from a subject, it is preferred that the IS is added such that it is dissolved in a solvent, e.g. water, prior to said addition to the sample.

According to the present invention, including any aspect and embodiment thereof, a biomarker is detected.

As preferably used herein, the term "detecting" means methods which include detecting the presence or absence of a substance in a sample and/or qualifying the type of said substance. In an embodiment the substance is a biomarker, a control and/or an internal standard. Detecting can be accomplished by methods known in the art and those further described herein. These methods include, without limitation, mass spectrometric analysis, biochip array, functional nucleic acids and/immunoassay. Preferably, the biomarker is detected and/or quantified by means of mass-spectrometric analysis. In a more preferred embodiment, mass spectrometric analysis is selected from the group comprising SELDI MS, MALDI MS, ESI MS, DESI MS and ion mobility MS. In an embodiment, mass spectrometric analysis uses an analyzer selected from the group comprising ToF, QToF, ion trap, Triple Quad, orbitrap, FT-ICR, ion mobility and any combination thereof. In an embodiment of the present invention, including any aspect and embodiment thereof, the level of the biomarker is determined by means of mass spectrometric analysis following HPLC separation.

In another embodiment of each and any aspect of the present invention, the biomarker is detected by means of an interaction partner. Such interaction partner is one selected from the group comprising an antibody, an anticaline and a functional nucleic acid. It is within the skills of a person of the art to generate an antibody binding to the biomarker. Antibodies may be generated as known to the one skilled in the art and described, e. g. by Harlow, E., and Lane, D., "Antibodies: A Laboratory Manual," Cold Spring Harbor Laboratory, Cold Spring Harbor, NY, (1988). It is within the skills of a person of the art to generate an anticaline binding to the biomarker. The generation of anticlines is, for example, described in German patent application DE 197 42 706. In an embodiment, the functional nucleic acid is an aptamer. It is within the skills of a person of the art the generate an aptamer. Aptamers are D-nucleic acids which are either single stranded or double stranded and which specifically interact with a target molecule. The generation of aptamers is, for example, described in European patent EP 0 533 838. In an embodiment, the functional nucleic acid is a spiegelmer. It is within the skills of a person of the art the generate a spiegelmer. Spiegelmers are L-nucleic acids which are either single stranded or double stranded and which specifically interact with a target molecule. The generation of aptamers is, for example, described in international patent application WO 98/08856.

It will be understood by a person skilled in the art that the above indicated techniques and methods for detecting the biomarker may be equally used for quantifying the biomarker.

In an embodiment of the present invention, including any aspect and embodiment thereof, the "level" or "level of a biomarker" as preferably used herein, means the concentration or concentration of a biomarker, preferably in a sample of a subject. The level may be an absolute level, expressed, for example, in ng/ml (ng of the compound and biomarker, respectively, in ml of a/the sample). The level may be a relative level. Such relative level is, in an embodiment, the ratio of a/the biomarker to an internal standard. In an embodiment of the present invention, including any aspect and embodiments thereof, is determined as follows, preferably after cleavage of protein C4, C1q and/or C1-INH peptide fragments, and more preferably after alkylation of the free SH-groups of the peptide. In the analytical set-up as described in the example part in more detail, an internal standard is added to the sample to be analyzed. In the course of such analysis a chromatogram is obtained indicating as individual peaks the various compounds detected in the sample. The various compounds include, among others, a fragment of C4 protein, C1q and/or C1-INH protein and the internal standard. In order to determine from such chromatogram and the peaks indicated therein, the concentration or level of a/the fragment peptide(s) the peak area of the peak corresponding to a/the peptide fragment(s) and the peak area of the peak corresponding to the internal standard is determined. Based on the peak area of the fragment peptide(s) and the peak area of the internal standard the ratio of the fragment peptide(s) to the internal standard can be determined. The concentration of a/the fragment peptide(s) is obtained using a standard curve of a/the fragment peptides at different concentrations in the presence of internal standard at known concentration(s).

In embodiment of the present invention, including any aspect and embodiment thereof, the level of a/the biomarker is compared to a level of the same or another biomarker of the present invention determined in another sample, e.g. from the same patient, from another patient, from a control and/or from the same or different points in time, and/or a level of a control and/or a level of an IS. In connection therewith "comparing" or "compared to" as used herein, preferably means the mathematical comparison of the two or more values of the levels or ratios of the biomarker(s). It will thus be immediately evident whether one of said values is higher, lower or identical if at least two of such values or ratios are compared with each other. In an embodiment, such comparison may be carried out using a/the absolute level. In an alternative embodiment, such comparison may be carried out using a/the relative level.

In an embodiment of the present invention, including any aspect and embodiments thereof, the level of the biomarker is also determined in a control. As used herein, a control is preferably a sample from a subject, wherein the hereditary angioedema status of said subject is known. In an embodiment a control is a sample of a healthy patient. In a further embodiment an amount of said biomarker is added to said sample of a healthy patient prior to determining the level of said biomarker in said sample of a healthy patient comprising said added biomarker, preferably in the practicing of a method of the present invention. In a further embodiment the control is a sample from at least one subject having a known hereditary angioedema status, e.g. a control patient, and in a still further preferred embodiment also comprises the genetic status with regard to mutations of the gene, affected in said disease, comprising C1 esterase inhibitor protein, i.e. comprising the subject having homozygous and/or compound heterozygous mutations, the subject being a carrier of a mutation. In a further preferred embodiment, the control is a sample from a subject not being treated for the disease. In a still further preferred embodiment the control is a sample from a single subject or a pool of samples from different subjects and/or samples taken from the subject(s) at different points in time.

In an embodiment of the present invention, including any aspect and embodiments thereof, a subject is considered to be a healthy subject with regard to the disease, if the subject does not suffer from symptoms associated with such disease. More specifically and in an embodiment of the present invention, including any aspect and embodiment thereof, a subject will be considered to be healthy regarding hereditary angioedema, if it has no mutation of the functional parts of the C1 esterase inhibitor gene resulting in a reduction of or deficiency of the respective protein or the activity thereof, resulting in symptoms associated with hereditary angioedema.

In connection with the present invention, including any aspect and embodiments thereof, a "patient" is a subject showing at least one symptom of the disease. More preferably, a patient is a subject presenting one homozygous mutation or multiple heterozygous mutations of the C1 esterase inhibitor gene resulting in reduction or deficiency of the respective protein and /or protein activity, resulting in symptoms associated with hereditary angioedema. Furthermore, in connection therewith a "carrier" is a subject presenting one heterozygous mutation of the C1 esterase inhibitor gene resulting or not resulting in reduction or deficiency of the respective protein and /or protein activity, usually or preferably not resulting in symptoms associated with hereditary angioedema.

In embodiment of the present invention, including any aspect and embodiment thereof, the level of a/the biomarker is compared to a cut-off (which term is synonymously used to the terms cut-off value or cut-off level). The term "cut-off value" as preferably used herein is a level (or concentration) which may be an absolute level or a relative level, which is indicative whether a person is suffering from a disease and/or is at risk of suffering from a disease. Depending on the biomarker, a subject is regarded as suffering the from the diseases or being at risk of suffering from the diseases if either the level of the biomarker detected and determined, respectively, is lower than the cut-off value, or the level of the biomarker detected and determined, respectively, is higher than the cut-off value. As preferably used herein, the cut-off value is set at the mean value of a cohort of healthy subject ± 2x standard deviation.

The cut-off value for some of the fragment peptides used in the method for differential diagnosis of hereditary angioedema is as follows.

| **Peptide** | **Cut-off** |
|---|---|
| C4Beta_[571-579] | 500 ng/mL |
| SerpinG1_[242-249] | 835 ng/mL |
| C1q-Beta_[178-186] | 800 ng/mL |
| C4Alpha_[680-685] | 260 ng/mL |
| C4Alpha_[786-791] | 100 ng/mL |
| C4Beta_[294-297] | 201 ng/mL |
| C4Gamma_[1638-1641] | 920 ng/mL |
| SerpinG1_[391-400] | 392 ng/mL |
| C1q-Beta_[63-77] | 1690 ng/mL |

It will be understood by a person skilled in the art that based on the above cut-off values, corresponding cut-off values may be calculated for any of the other fragment peptides based on the molecular weight of the above fragment peptides and said other fragment peptides. The same also applies to the cut-off value of any one of the C4 protein, C1-INH protein and C1q protein and the individual polypeptides forming the same. The cut-off values calculated in such way are also referred to herein as corresponding cut-off values, whereby, preferably reference is made to one or more of the above cut-off values for the indicated fragment peptides.

A "limit of detection" of a substance such as a biomarker of control, as preferably used herein, is a level of the substance determined by a method for determining a level of the substance, wherein a level less then or lower then said limit of detection cannot be determined by said method. It is thus immediately clear that a "cut-off value" and a "limit of detection", as used herein, are preferably not necessarily identical, although both reflect a certain level of a substance, e.g. of a biomarker of the present invention. Also, it will be immediately understood that a cut-off value will be selected preferably such that selectivity and sensitivity of the method are as high as possible. In contrast thereto, a limit of detection represents an absolute level of the biomarker of the present invention which reflects the minimum level of biomarker which can be detected with a method for determining the level of said biomarker. It is thus immediately clear that a limit of detection depends on the method for determining a level of a substance and on the substance the level of which is to be determined by the method. A skilled person will immediately understand that a high limit of detection, e.g. higher than an ideal cut-off value would possibly result in a low sensitivity of the method since the percentage of true positives that are predicted by a test to be positive also depends on whether a level of the biomarker may be determined for said true positives. In other words, if the limit of detection is higher than an ideal cut-off value, true positives having a level of the biomarker slightly higher than the cut-off value may not be distinguished from true negatives having a level of the biomarker lower than the cut-off value since no level of the biomarker may be determined for both true positives having a level of the biomarker slightly higher than the cut-off value and negatives having a level of the biomarker lower than the cut-off value. It is thus immediately clear that a low limit of detection is of advantage. Preferably, an "ideal cut-off value" as used herein is a cut-off value that has the highest selectivity and sensitivity.

It is within the present invention that the method for diagnosing the disease as subject to the first aspect of the present invention, in one embodiment, encompasses that the subject from whom the sample has been taken, is a subject from whom a sample had been subjected to said method earlier. In a preferred embodiment the time difference between said two samples is 2 weeks, one month, two months or three months; preferably the time difference between said two samples is one month. In accordance therewith, the method of the first aspect, including any embodiment thereof, comprises determining the level of a/the biomarker in a sample from as subject and as a further step determining the level of a/the biomarker in a second sample from the subject, wherein the second sample has been taken from the subject after said time difference.

It is within the present invention that the method for diagnosing the disease as subject to the first aspect of the present invention, in one embodiment, uses a sample taken from a subject to whom a therapy had been applied prior to the point in time when the sample was taken or to whom a therapy was applied at the point in time when the sample was taken.

It is within the present invention that the method for diagnosing the disease as subject to the first aspect of the present invention, in one embodiment, uses a sample taken from a subject to whom no therapy had been applied prior to the point in time when the sample was taken or to whom no therapy was applied at the point in time when the sample was taken.

In a second aspect, the present invention is related to a kit, wherein the kit comprises at least one element selected from the group comprising an interaction partner of a or the biomarker, a or the biomarker, instructions of use for the kit, and one or more containers. In an embodiment, the kit is for use in a method according to the first aspect of the present invention. In a preferred embodiment, the kit comprises an interaction partner of a or the biomarker, preferably an interaction partner for one fragment peptide of each of C4, C1q and C1-IHN or an interaction part for each of C4, C1q and C1-IHN, and instructions for use and, optionally, one or more containers. In another preferred embodiment, the kit comprises a or the biomarker, preferably an interaction partner for one fragment peptide of each of C4, C1q and C1-IHN or an interaction part for each of C4, C1q and C1-IHN, and instructions for use and, optionally, one or more containers.

In an embodiment of the second aspect, the interaction partner is one selected from the group comprising an antibody, an anticaline, an aptamer and a spiegelmer, wherein any one of the antibody, anticaline, aptamer and spiegelmer and spiegelmer is capable of binding to a or the biomarker, preferably the binding is such that a complex is formed between the biomarker and the interaction partner which allows detection and, respectively, quantification of the complex or the biomarker, preferably after dissolution of the complex.

The term "being at risk for developing a disease" as used herein preferably means that it is likely that a subject will suffer from said disease and/or will develop said disease or symptoms associated with said disease, particularly if no treatment is applied. In connection therewith, it has to be acknowledged that hereditary angioedema is a genetic disorder and thus the occurrence of relatives, particularly parents having said disease or having a mutation known to be the cause of said disease are indicative for a subject, e.g. the child of two hereditary angioedema patients or two hereditary angioedema carriers, to be at risk for developing said disease. It will furthermore be acknowledged that the progression of a disease is linked to the occurrence of symptoms as well as the severity of said symptoms. Accordingly, a person not suffering from symptoms at present, however, may be at risk for developing the disease, for example, because although genetically mutations of a gene, known to cause a disease are present, no symptoms or no severe symptoms occur. Nevertheless, it will be immediately understood that the methods and biomarkers of the present invention, particularly if the level of said biomarker according to the present invention is reduced or increased, depending on the biomarker, allow for diagnosing that such subject is at risk for developing the disease independent from the presence or absence of symptoms. Accordingly, the methods according to the present invention allow for determining whether a subject is at risk of suffering from the disease. It is also within the present invention that a therapy is applied, maintained, reduced, elevated or not applied based on whether the subject is at risk of suffering from the disease or not.

The term "qualifying hereditary angioedema status" in a subject as used herein, preferably means a classification of a subject's biomarker profile selected from the group comprising to identify or detect the presence or absence of hereditary angioedema in the subject, to predict the onset of or the risk for developing of hereditary angioedema in the subject, to determine the course of hereditary angioedema in a subject, to determine whether a subject suffers from an early status of hereditary angioedema or an advanced or progressed status of hereditary angioedema or to determine whether a level of a biomarker in a subject has significantly changed over time.

The term "managing subject treatment" or "subject management" as used herein, preferably refers to the behavior of the clinician or physician subsequent to the determination of hereditary angioedema status. For example, if the result of the methods according to the present invention is inconclusive or there is reason that confirmation of status is necessary, the physician may order new tests, such as testing for the function of the affected proteins and/or sequencing of the C1 esterase inhibitor gene. Alternatively, if the status indicates that treating for hereditary angioedema is appropriate, the physician may schedule the subject for treating for hereditary angioedema. Likewise, if the status is negative or if the results show that treatment has been successful, no further management may be necessary. Nevertheless, a person skilled in the art will immediately acknowledge that besides gene therapy any suitable and/or effective therapy may be applied, including the therapy discloses herein. Furthermore, it is an embodiment of the present invention that managing subject treatment comprises titrating of a dose of a drug applied as a treatment for hereditary angioedema, e.g. amount of an C1 esterase inhibitor, a kallikrein inhibitor or a bradykinin antagonist, applied or administered to a patient and/or subject. In some embodiments of the methods of the present invention wherein a level of a biomarker present in a sample from a subject is determined at several points in time, or is compared to other levels of the biomarker, a cut-off value and/or a level of said biomarker in a control and/or another value of a ratio of the levels of two biomarkers, a skilled person will apply or not apply a therapy, or amend a therapy already applied in order to treat or not to treat, or to continue treating hereditary angioedema.

In an embodiment of the present invention, the terms "being at risk of developing the disease" and "being at risk of suffering from the disease" are used interchangeably herein, unless indicated to the contrary.

The present invention is now further illustrated by the following figures and examples from which further features, embodiments and advantages may be taken.

### More specifically,

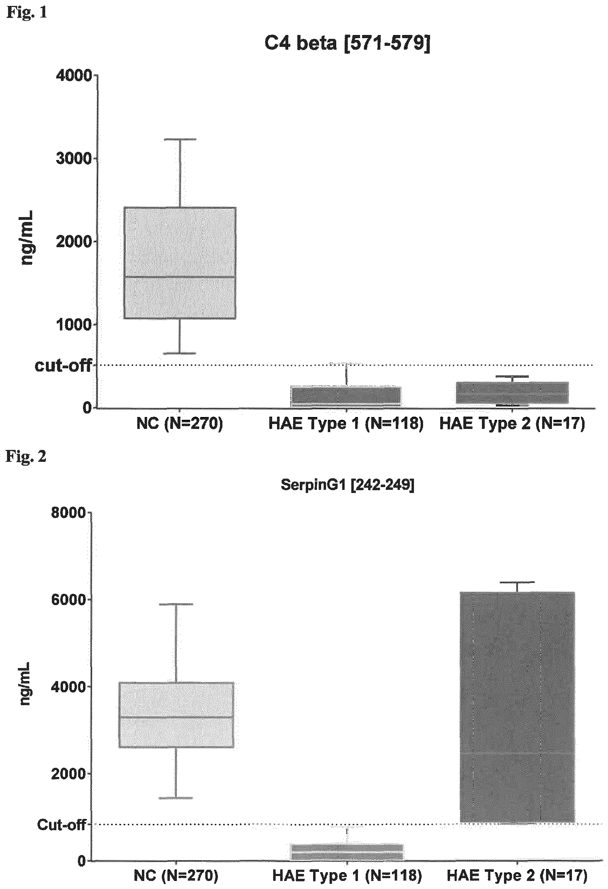

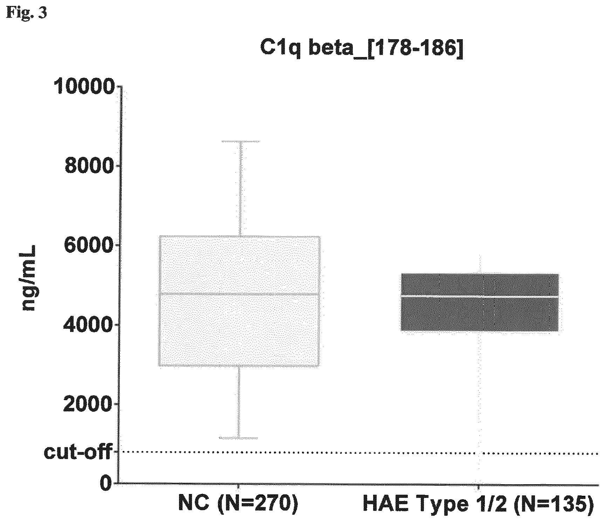

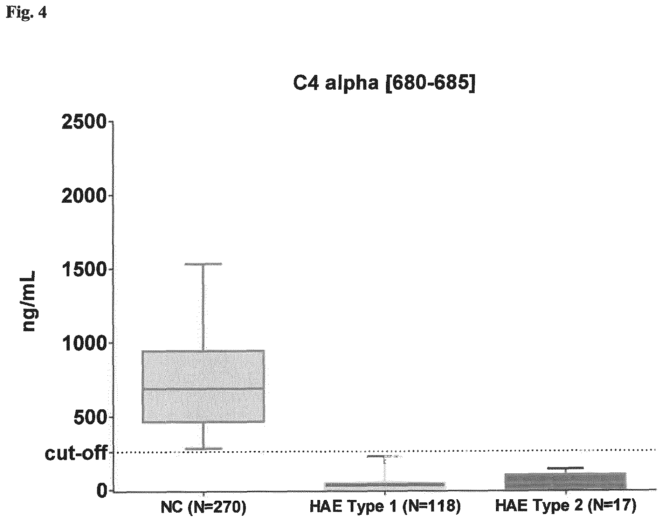

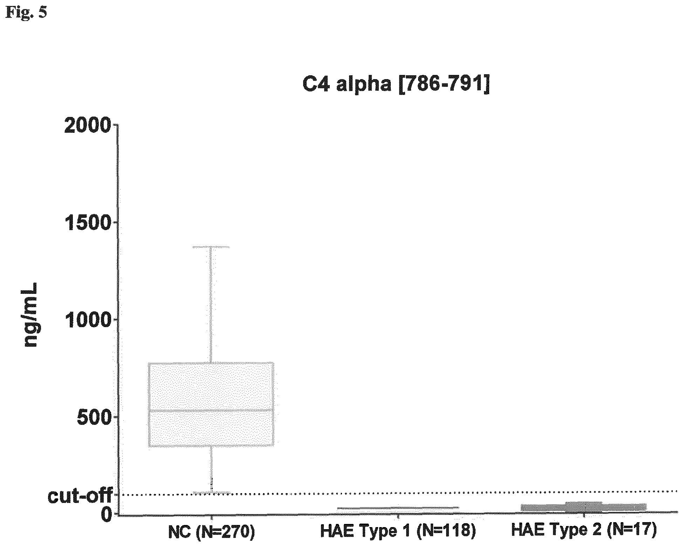

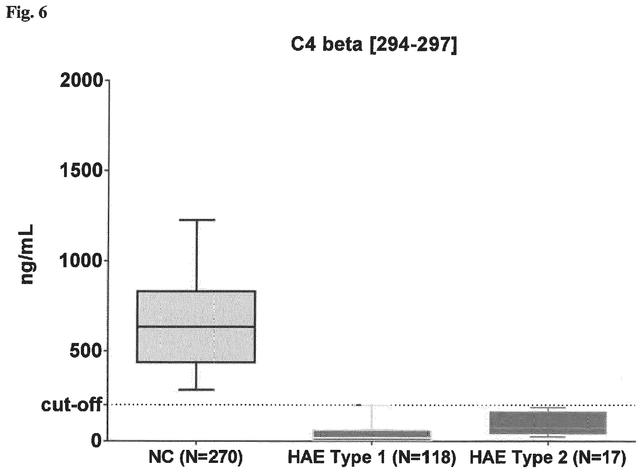

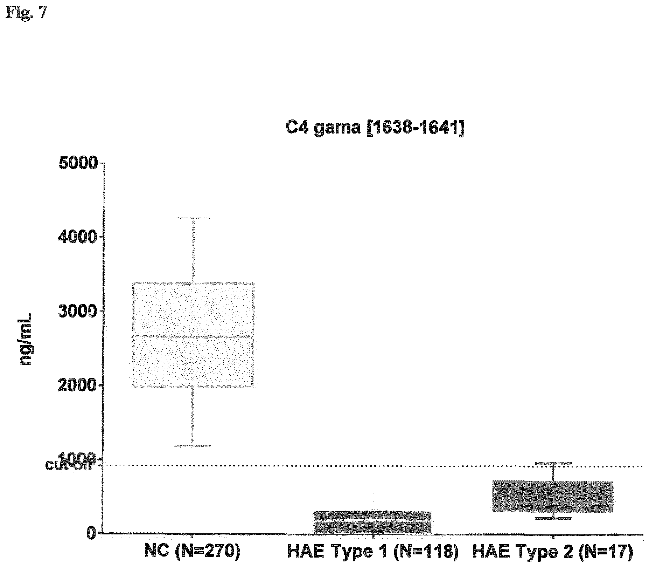

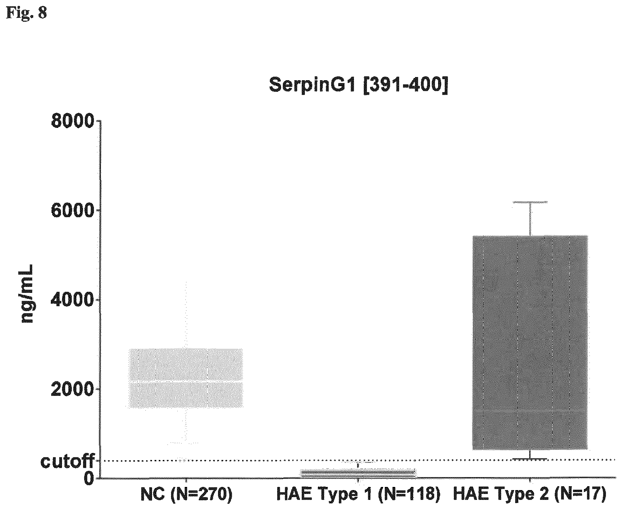

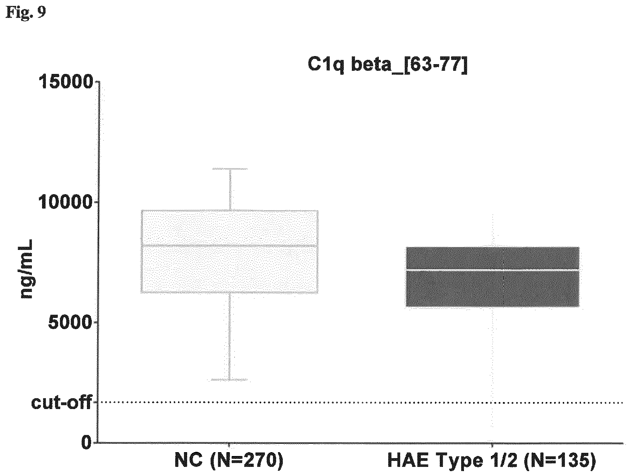

Figs. 1 to 9 are boxplots indicating levels of the indicated peptide; the y-axis demonstrates the logarithmised levels of said indicated peptide in ng/ml as determined from a dried blood spot on a filter card as described in the Example part; the x-axis depicts groups of subjects which have been grouped as described in the Example part. The boxplot represents the 25th and 75th percentile of each group of subjects by the bottom and top of the box, respectively; the band near the middle of the box represents the 50th percentile (i.e. the median) of each group; the whiskers represent one standard deviation above and below the mean of the data; any data not included between the whiskers is shown as an outlier with a small circle or star. The horizontal line represents the cut-off level of expressed as ng/ml for the indicated peptide.
Fig. 1 is a boxplot of peptide fragment C4Beta_[571-579] of protein C4 beta illustrating a cut-off of 500 ng/ml. Such cut-off allows to distinguish between healthy controls and patients suffering from HAE type 1 and HAE type 2.
Fig. 2 is a boxplot of peptide fragment SerpinGl_[242-249] of protein C1-INH illustrating a cut-off of 835 ng/ml. Such cut-off allows to distinguish between healthy controls and patients suffering from HAE type 1.
Fig. 3 is a boxplot of peptide fragment C1q Beta_[178-186] of protein C1q beta illustrating a cut-off of 800 ng/ml. This peptide fragment may be used as a control.
Fig. 4 is a boxplot of peptide fragment C4Alpha_[680-685] of protein C4alpha illustrating a cut-off of 260 ng/ml. Such cut-off allows to distinguish between healthy controls and patients suffering from either HAE type 1 or HAE type 2.
Fig. 5 is a boxplot of peptide fragment C4Alpha_[786-791] of protein C4alpha illustrating a cut-off of 100 ng/ml. Such cut-off allows to distinguish between healthy controls and patients suffering from either HAE type 1 or HAE type 2.
Fig. 6 is a boxplot of peptide fragment C4Beta_[294-297] of protein C4beta illustrating a cut-off of 201 ng/ml. Such cut-off allows to distinguish between healthy controls and patients suffering from either HAE type 1 or HAE type 2.
Fig. 7 is a boxplot of peptide fragment C4Gamma_[1638-1641] of protein C4gamma illustrating a cut-off of 920 ng/ml. Such cut-off allows to distinguish between healthy controls and patients suffering from either HAE type 1 or HAE type 2.
Fig. 8 is a boxplot of peptide fragment SerpinG1_[391-400] of protein C1-INH illustrating a cut-off of 392 ng/ml. Such cut-off allows to distinguish between healthy controls and patients suffering from HAE type 1.
Fig. 9 is a boxplot of peptide fragment C1q-Beta_[63-77] of protein C1q beta illustrating a cut-off of 1690 ng/ml. This peptide fragment may be used as a control.

### Examples

In the Examples described in the following a dried blood spot (abbr. DBS) on a filter card was used as a sample from a subject.

### Example 1: Method for HAE diagnostic based on fragmentation of C3, C1q, C4 and C1-INH into peptides and mass spectrometry thereof

To quantify the content/levels of C3, C1q, C4 and C1-INH in dried blood spots (DBS) extract a protocol as described was used. After extraction of blood components, the DBS extract was subjected in situ to reaction with dithiothreitol (DDT) to reduce the disulfide bridges in the proteins and to iodacetamide (IAA) to alkylate the free -SH groups. The reaction mixture was then digested in its entirety with trypsin. The tryptic mixture containing peptide fragments of the proteins to be analyzed was injected in LC/IM-high resolution mass spectrometry. For all proteins, peptides without post-transactional modifications could be identified in blood matrix (see Table 1 "C3, C1q, C4 and C1-INH peptides identified in tryptic mixture obtained after total tryptic digestion of DBS extract, selected peptides with +H adducts"). For all peptides fragmentation spectra were obtained and, based on the experimental fragmentation pattern, transitions to be used in multiple reaction monitoring mass spectrometry.

The tryptic peptides could be measured next by LC/MRM-MS. Below are example of tryptic peptides from C3, C1q, C4 and C1-INH detected and quantified using LC/MRM-MS.

### Equipment

For detecting the tryptic peptides of the proteins to be quantified in a biological sample of a donor, the following equipment was used:

| **Equipment** | **Model** | **Provider** |
|---|---|---|
| DBS Puncher | 1296-071 Delfia | Perkin Elmer |
| Pipettes | single and multichannel | Eppendorf |
| Vortexer | Mixer UZUSIO VTX-300L | LMS co. LTD |
| Sonicator | SW12H | Sonoswiss |
| Incubator | Titramax 1000 | Heidolph |
| Centrifuge | Benchtop | Eppendorf |
| UPLC | Acuity iclass | Waters |
| IM-qToF | Vion | Waters |
| TQ | TQS-micro | Waters |
| Data mining tool | Progenesis | Nonlinear |

### Reagents

For detecting the peptides of the proteins to be quantified in a sample from a subject the following reagents were used. To the extent that values depend on temperature (e.g., the pH value) such values were determined at a temperature of 25°C.

| **Name** | **Supplier** | **Purity** |
|---|---|---|
| 1,4-Dithiothreitol | Roche | > 97% |
| Acetonitrile, waterfreei (max. 0,003% H₂O) HiPerSolv CHROMANORM® | VWR | UPLC/UHPLC grade |
| AcroPrep™ Advance 96 well filter plates for aqueous filtration, 350 µl, 1,0 µm glass fibre | PALL | |
| Ammonia solution | Merck | 25% |
| Ammoniumbicarbonate | ACROS | 98% |
| Formic acid, ACS | VWR | >96% |
| Iodoacetamide, IAA | Sigma Aldrich | > 99% |
| Kinetex colums EVO C18 | VWR | |
| Leucine-Enkephalin | waters | |
| Methanol HiPerSolv CHROMANORM® | VWR | LC-MS grade |
| Mirco-Platte 96-wells, PP, F-GREINER (100 pieces) Natur | VWR | |
| SafeSeal vial 1,5 ml | Sarstedt | |
| Taurocholic acid sodium salt hydrate | Sigma Aldrich | >95% |
| Trypsin 20 µg/vial | Promega | sequencing grade |
| Verex™ Cap (pre-assembled), 8-425, Screw top, w/ PTFE/Silicone septa, black | Phenomenex | |
| Verex™ Insert, 5mm Dia., 175µL, Clear 51, Conical Bottom, w/ bottom spring | Phenomenex | |
| Verex™ Vial, 8mm Screw Top, 2mL, Clear 33, w/ Patch | Phenomenex | |
| Water HiPerSolv® CHROMANORM® | VWR | LC-MS grade |
| Pipette tips | Sarstedt | |

### Preparation of stock solution of Internal Standard

Internal Standard (IS 1) stock solution was used as internal standard and was prepared by dissolving 3 mg Leucine-Enkephaline (as provided by Waters, UK) in water to a concentration of 400 µg/mL.

### Storing of Samples and Solutions

Control samples and study samples (dried blood spots) were stored at RT. Internal Standard working solutions were stored at room temperature until use.

### Sample Preparation for Analysis

1 punch Ø of 3.2 mm was cut from the filter card with dried blood spots and subjected the following protocol:
First, for extraction 100 µL 0.1 M NH₄HCO₃ were added to the punches, whereby the material was sonicated for 10 min. at 60°C, incubated for 30 min on a shaker (at 700 rpm) at 37°C and sonicated 10 minutes at 60°C.
Second, to the solution 12.5 µl 1 M DTT was added and the reaction mixture was incubated for 3 h at 37°C on a shaker (700 rpm).
Third, 37.5 µl 1 M IAA was added and the solution was incubated for 1.5 hours on a shaker (700 rpm) in the dark.
Fourth, 10 µl 0.5 µg/µl trypsin was added and the solution was incubated for 3 to 16 hours on a shaker (700 rpm) in the dark.

The thus obtained solution containing a digest of blood extract was transferred to a PTFE (polytetrafluoroethylene) filter plate (Acroprep™, Pall, Germany) and then to a 96 well plate by centrifugation at 3.500 rpm. Afterwards, 100 µL of internal standard with a concentration of 200ng/mL was added.

### Methods

A person skilled in the art will acknowledge that methods for detecting the fragment peptides of the proteins to be analyzed in a sample from a subject using mass spectrometric analysis may also employ other tryptic peptides, specific transitions and specific fragments which allow for specific detection of and/or quantification of HAE relevant peptide fragments and their isoforms in said sample from a subject.

LC/IM-QToF- MS analyses of the peptide fragments of the proteins to be analyzed from DBS extracts were performed using a Waters Acquity iclass UPLC (Waters, UK) coupled with Vion mass spectrometer (Waters, UK) as follows.
1. Chromatographic run was performed on a Kinetex EVO C18 column (Phenomenex, Germany). 10 µL of the extract were injected onto the column and the compounds of the extract were eluted using a linear gradient from 0 % A (50 mM formic acid in water) to 100 % B (50 mM formic acid in acetonitrile:methanol vol. 1:1).
2. Internal standard was continuously injected at a concentration of 200ng/mL in water and the signal was used to normalize the sample signal across the batch.

IM-QToF MS analyses were performed in positive ion mode using the following parameters:
- Analyzer mode: sensitivity
- MS mode: High definition MSE
- Capillary voltage: 1.2 kV
- Source temperature: 150 °C
- Desolvation temperature: 600 °C
- Desolvation gas; 1000 L/h
- Cone gag: 50 L/h
- Low Collision Energy: 6 eV
- High Collision Energy Ramp: 20-40 eV
- Scan mass: 50-1000 m/z
- Scan time: 0.5 s

LC/MRM-MS analyses of the peptide fragments of the proteins to be analyzed for DBS extracts were performed using a Waters Acquity iclass UPLC (Waters, UK) coupled with a TQ-S micro mass spectrometer (Waters, UK).

For the examples the following parameters were used in the quantification of the peptide fragments:
1. Chromatographic run was performed on a Kinetex EVO C18 column (Phenomenex, Germany). The 10 µL extract were injected on the column and the compounds were eluted using a linear gradient from 0 % A (50 mM formic acid in water) to 100 % B (50 mM formic acid in acetonitrile:methanol vol. 1:1).
2. For the internal standard, MRM transition 556.24 →119.97 was monitored. For each peptide, specific transition was used as shown in Example 2.

MRM-MS analyses were performed in positive ion mode using the following parameters:
- Capillary voltage: 1.2 kV
- Cone voltage: 20 V
- Source temperature: 150 °C
- Desolvation temperature: 600 °C
- Desolvation gas: 1000 L/h
- Cone gag: 50 L/h
- Collision Energy: 20 V
- Collision Cell Entrance: 30 eV
- Collision Cell Exit: 30 eV.

### Example 2: Quantifying peptide fragments of proteins C1q, C4, C1-INH and C3 in dried blood spots of healthy donors

Using the methods outlined in Example 1, the different peptide fragments of proteins C1q, C4, C1-INH and C3 were quantified using DBS from a total of 270 healthy subjects.

For Complement C1q the following tryptic peptides could be quantified, whereby the numbers in the brackets represents the position of the first amino acid and the last amino acid of the peptide in amino acid sequence of C1q (with the sequences being indicated with the N-terminus being at the left side and the C-terminus being at the right side). Such peptides are shown in Table 1:

**Table 1:**

| **Peptide** | **Sequence (N-terminus -> C-terminus)** |
|---|---|
| C1q-A_[104-110] | GSPGNIK |
| C1q-A_[111-121] | DQPRPAFSAIR |
| C1q-A_[123-150] | NPPMGGNVVIFDTVITNQEEPYQNHSGR |
| C1q-A_[151-180] | FVCTVPGYYYFTFQVLSQWEICLSIVSSSR |
| C1q-A_[151-180]_Cys_CAM: 153, 172 | FVCTVPGYYYFTFQVLSQWEICLSIVSSSR |
| C1q-A_[186-195] | SLGFCDTTNK |
| C1q-A_[186-195]_Cys_CAM: 190 | SLGFCDTTNK |
| C1q-A_[196-219] | GLFQWSGGMVLQLQQGDQVWVEK |
| C1q-A_[224-245] | GHIYQGSEADSVFSGFLIFPSA |
| C1q-A_[23-27] | EDLCR |
| C1q-A_[28-32] | APDGK |
| C1q-A_[34-41] | GEAGRPGR |
| C1q-A_[49-60] | GEQGEPGAPGIR |
| C1q-A_[82-94] | VGYPGPSGPLGAR |
| C1q-B_[118-121] | ATQK |
| C1q-B_[137-141] | DQTIR |
| C1q-B_[160-163] | FTCK |
| C1q-B_[164-177] | VPGLYYFTYHASSR |
| C1q-B_[178-186] | GNLCVNLMR |
| C1q-B_[178-186]_Cys_CAM: 181 | GNLCVNLMR |
| C1q-B_[194-215] | VVTFCDYAYNTFQVTTGGMVLK |
| C1q-B_[194-215]_Cys_CAM: 198 | VVTFCDYAYNTFQVTTGGMVLK |
| C1q-B_[216-229] | LEQGENVFLQATDK |
| C1q-B_[230-253] | NSLLGMEGANSIFSGFLLFPDMEA |
| C1q-B_[28-59] | QLSCTGPPAIPGIPGIPGTPGPDGQPGTPGIK |
| C1q-B_[28-59]_Cys_CAM: 31 | QLSCTGPPAIPGIPGIPGTPGPDGQPGTPGIK |
| C1q-B_[63-77] | GLPGLAGDHGEFGEK |
| C1q-B_[78-88] | GDPGIPGNPGK |
| C1q-B_[93-98] | GPMGPK |
| C1q-B_[99-110] | GGPGAPGAPGPK |
| C1q-C_[118-126] | FQSVFTVTR |
| C1q-C_[127-139] | QTHQPPAPNSLIR |
| C1q-C_[140-157] | FNAVLTNPQGDYDTSTGK |
| C1q-C_[162-184] | VPGLYYFVYHASHTANLCVLLYR |
| C1q-C_[162-184]_Cys_CAM: 179 | VPGLYYFVYHASHTANLCVLLYR |
| C1q-C_[189-198] | VVTFCGHTSK |
| C1q-C_[189-198]_Cys_CAM: 193 | VVTFCGHTSK |
| C1q-C_[199-210] | TNQVNSGGVLLR |
| C1q-C_[211-245] | LQVGEEVWLAVNDYYDMVGIQGSDSVFSGFLLFPD |
| C1q-C_[29-47] | NTGCYGIPGMPGLPGAPGK |
| C1q-C_[29-47]_Cys_CAM: 32 | NTGCYGIPGMPGLPGAPGK |
| C1q-C_[48-57] | DGYDGLPGPK |
| C1q-C_[58-69] | GEPGIPAIPGIR |
| C1q-C_[76-86] | GEPGLPGHPGK |
| C1q-C_[87-113] | NGPMGPPGMPGVPGPMGIPGEPGEEGR |

All of the Complement C1q tryptic peptides can be used to differentiate between healthy subjects and hereditary angioedema patients. Two of said peptides, namely C1q-B_[[178-186] with MRM transition 510.26 → 254.58 and C1q-B_[63-77] with MRM transition 495.25→774.5 were used as representative examples(see Example 3).

For complement C3 the following tryptic peptides could be quantified, whereby the numbers in the brackets represents the position of the first amino acid and the last amino acid of the peptide in amino acid sequence of C3 (with the sequences being indicated with the N-terminus being at the left side and the C-terminus being at the right side). Such peptides are shown in Table 2:

**Table 2:**

| **Peptide** | **Sequence (N-terminus -> C-terminus)** |
|---|---|
| C3Beta_[105-119] | FVTVQATFGTQVVEK |
| C3Beta_[120-136] | VVLVSLQSGYLFIQTDK |
| C3Beta_[137-148] | TIYTPGSTVLYR |
| C3Beta_[149-155] | IFTVNHK |
| C3Beta_[156-161] | LLPVGR |
| C3Beta_[162-176] | TVMVNIENPEGIPVK |
| C3Beta_[177-205] | QDSLSSQNQLGVLPLSWDIPELVNMGQWK |
| C3Beta_[208-225] | AYYENSPQQVFSTEFEVK |
| C3Beta_[226-241] | EYVLPSFEVIVEPTEK |
| C3Beta_[23-35] | SPMYSIITPNILR |
| C3Beta_[242-249] | FYYIYNEK |
| C3Beta_[250-258] | GLEVTITAR |
| C3Beta_[259-263] | FLYGK |
| C3Beta_[265-281] | VEGTAFVIFGIQDGEQR |
| C3Beta_[291-304] | IPIEDGSGEVVLSR |
| C3Beta_[306-315] | VLLDGVQNPR |
| C3Beta_[316-322] | AEDLVGK |
| C3Beta_[323-343] | SLYVSATVILHSGSDMVQAER |
| C3Beta_[344-359] | SGIPIVTSPYQIHFTK |
| C3Beta_[363-386] | YFKPGMPFDLMVFVTNPDGSPAYR |
| C3Beta_[36-65] | LESEETMVLEAHDAQGDVPVTVTVHDFPGK |
| C3Beta_[387-408] | VPVAVQGEDTVQSLTQGDGVAK |
| C3Beta_[409-425] | LSINTHPSQKPLSITVR |
| C3Beta_[429-439] | QELSEAEQATR |
| C3Beta_[440-462] | TMQALPYSTVGNSNNYLHLSVLR |
| C3Beta_[463-478] | TELRPGETLNVNFLLR |
| C3Beta_[479-481] | MDR |
| C3Beta_[482-486] | AHEAK |
| C3Beta_[489-497] | YYTYLIMNK |
| C3Beta_[500-502] | LLK |
| C3Beta_[503-505] | AGR |
| C3Beta_[506-508] | QVR |
| C3Beta_[509-530] | EPGQDLVVLPLSITTDFIPSFR |
| C3Beta_[531-544] | LVAYYTLIGASGQR |
| C3Beta_[545-556] | EVVADSVWVDVK |
| C3Beta_[557-566] | DSCVGSLVVK |
| C3Beta_[557-566]_ Cys_CAM: 559 | DSCVGSLVVK |
| C3Beta_[574-584] | QPVPGQQMTLK |
| C3Beta_[585-592] | IEGDHGAR |
| C3Beta_[616-622] | IWDVVEK |
| C3Beta_[623-633] | ADIGCTPGSGK |
| C3Beta_[634-657] | DYAGVFSDAGLTFTSSSGQQTAQR |
| C3Beta_[658-667] | AELQCPQPAA |
| C3Beta_[658-667]_Cys_CAM: 662 | AELQCPQPAA |
| C3Beta_[67-73] | LVLSSEK |
| C3Beta_[74-94] | TVLTPATNHMGNVTFTIPANR |
| C3Beta_[95-97] | EFK |
| C3Beta_[98-100] | SEK |
| C3cAlpha1_[749-764] | SNLDEDIIAEENIVSR |
| C3cAlpha1_[765-779] | SEFPESWLWNVEDLK |
| C3cAlpha1_[780-783] | EPPK |
| C3cAlpha1_[784-789] | NGISTK |
| C3cAlpha1_[797-812] | DSITTWEILA VSMSDK |
| C3cAlpha1_[814-834] | GICVADPFEVTVMQDFFIDLR |
| C3cAlpha1_[814-834]_Cys_CAM: 816 | GICVADPFEVTVMQDFFIDLR |
| C3cAlpha1_[835-841] | LPYSVVR |
| C3cAlpha1_[842-848] | NEQVEIR |
| C3cAlpha1_[849-855] | AVLYNYR |
| C3cAlpha1_[856-861] | QNQELK |
| C3cAlpha1_[864-879] | VELLHNPAFCSLATTK |
| C3cAlpha1_[864-879]_Cys_CAM: 873 | VELLHNPAFCSLATTK |
| C3cAlpha1_[905-913] | TGLQEVEVK |
| C3cAlpha1_[914-926] | AAVYHHFISDGVR |
| C3cAlpha1_[938-940] | MNK |
| C3cAlpha1_[941-945] | TVAVR |
| C3cAlpha1_[946-951] | TLDPER |
| C3cAlpha1_[952-954] | LGR |
| C3cAlpha2_[1321-1325] | SEETK |
| C3cAlpha2_[1326-1337] | ENEGFTVTAEGK |
| C3cAlpha2_[1338-1351] | GQGTLSVVTMYHAK |
| C3cAlpha2_[1354-1360] | DQLTCNK |
| C3cAlpha2_[1354-1360]_Cys_CAM: 1358 | DQLTCNK |
| C3cAlpha2_[1361-1364] | FDLK |
| C3cAlpha2_[1365-1375] | VTIKPAPETEK |
| C3cAlpha2_[1376-1381] | RPQDAK |
| C3cAlpha2_[1382-1391] | NTMILEICTR |
| C3cAlpha2_[1382-1391]_Cys_CAM: 1389 | NTMILEICTR |
| C3cAlpha2_[1394-1419] | GDQDATMSILDISMMTGFAPDTDDLK |
| C3cAlpha2_[1420-1427] | QLANGVDR |
| C3cAlpha2_[1428-1431] | YISK |
| C3cAlpha2_[1432-1436] | YELDK |
| C3cAlpha2_[1437-1441] | AFSDR |
| C3cAlpha2_[1442-1450] | NTLIIYLDK |
| C3cAlpha2_[1451-1462] | VSHSEDDCLAFK |
| C3cAlpha2_[1451-1462]_Cys_CAM: 1458 | VSHSEDDCLAFK |
| C3cAlpha2_[1463-1478] | VHQYFNVELIQPGAVK |
| C3cAlpha2_[1479-1491] | VYAYYNLEESCTR |
| C3cAlpha2_[1479-1491]_Cys_CAM: 1489 | VYAYYNLEESCTR |
| C3cAlpha2_[1492-1497] | FYHPEK |
| C3cAlpha2_[1502-1504] | LNK |
| C3cAlpha2_[1505-1507] | LCR |
| C3cAlpha2_[1505-1507]_Cys_CAM: 1506 | LCR |
| C3cAlpha2_[1527-1532] | VTLEER |
| C3cAlpha2_[1533-1535] | LDK |
| C3cAlpha2_[1536-1546] | ACEPGVDYVYK |
| C3cAlpha2_[1536-1546]_Cys_CAM: 1537 | ACEPGVDYVYK |
| C3cAlpha2_[1552-1570] | VQLSNDFDEYIMAIEQTIK |
| C3cAlpha2_[1571-1582] | SGSDEVQVGQQR |
| C3cAlpha2_[1583-1589] | TFISPIK |
| C3cAlpha2_[1592-1595] | EALK |
| C3cAlpha2_[1596-1599] | LEEK |
| C3cAlpha2_[1601-1624] | HYLMWGLSSDFWGEKPNLSYIIGK |
| C3cAlpha2_[1625-1644] | DTWVEHWPEEDECQDEENQK |
| C3cAlpha2_[1625-1644]_Cys_CAM: 1637 | DTWVEHWPEEDECQDEENQK |

All of the Complement C3 tryptic peptides can be used in the assay. Two of said peptides, namely C3Beta_[489-497] with MRM transition 604.8 → 327.22 and C3cAlpha1_[814-834]_Cys_CAM: 816 with MRM transition 824.74 → 798.44 were used as illustrative examples (see Example 3).

For complement C4 the following tryptic peptides could be quantified, whereby the numbers in the brackets represents the position of the first amino acid and the last amino acid of the peptide in amino acid sequence of C4 (with the sequences being indicated with the N-terminus being at the left side and the C-terminus being at the right side). Such peptides are shown in Table 3.

**Table 3:**

| **Peptide** | **Sequence (N-terminus -> C-terminus)** |
|---|---|
| C4Alpha_[1006-1008] | LPR |
| C4Alpha_[1009-1026] | GCGEQTMIYLAPTLAASR |
| C4Alpha_[1009-1026]_Cys_CAM: 1010 | GCGEQTMIYLAPTLAASR |
| C4Alpha_[1027-1030] | YLDK |
| C4Alpha_[1031-1042] | TEQWSTLPPETK |
| C4Alpha_[1043-1051] | DHAVDLIQK |
| C4Alpha_[1052-1055] | GYMR |
| C4Alpha_[1062-1072] | ADGSYAAWLSR |
| C4Alpha_[1073-1084] | GSSTWLTAFVLK |
| C4Alpha_[1085-1099] | VLSLAQEQVGGSPEK |
| C4Alpha_[1100-1126] | LQETSNWLLSQQQADGSFQDLSPVIHR |
| C4Alpha_[1168-1174] | VEASISK |
| C4Alpha_[1175-1182] | ASSFLGEK |
| C4Alpha_[1183-1204] | ASAGLLGAHAAAITAYALTLTK |
| C4Alpha_[1211-1248] | GVAHNNLMAMAQETGDNLYWGSVTGSQSNAV SPTPAPR |
| C4Alpha_[1249-1278] | NPSDPMPQAPALWIETTAYALLHLLLHEGK |
| C4Alpha_[1279-1291] | AEMADQAAAWLTR |
| C4Alpha_[1292-1300] | QGSFQGGFR |
| C4Alpha_[1301-1325] | STQDTVIALDALSAYWIASHTTEER |
| C4Alpha_[1326-1336] | GLNVTLSSTGR |
| C4Alpha_[1337-1340] | NGFK |
| C4Alpha_[1341-1349] | SHALQLNNR |
| C4Alpha_[1350-1352] | QIR |
| C4Alpha_[1353-1365] | GLEEELQFSLGSK |
| C4Alpha_[13 70-13 75] | VGGNSK |
| C4Alpha_[1383-1390] | TYNVLDMK |
| C4Alpha_[1391-1404] | NTTCQDLQIEVTVK |
| C4Alpha_[1391-1404]_Cys_CAM: 1394 | NTTCQDLQIEVTVK |
| C4Alpha_[1405-1428] | GHVEYTMEANEDYEDYEYDELPAK |
| C4Alpha_[1429-1446] | DDPDAPLQPVTPLQLFEG |
| C4Alpha_[680-685] | NVNFQK |
| C4Alpha_[686-690] | AINEK |
| C4Alpha_[691-700] | LGQYASPTAK |
| C4Alpha_[702-709]_Cys_CAM: 702, 703 | CCQDGVTR |
| C4Alpha_[71 0-714] | LPMMR |
| C4Alpha_[715-719]_Cys_CAM: 716 | SCEQR |
| C4Alpha_[723-729] | VQQPDCR |
| C4Alpha_[723-729]_Cys_CAM: 728 | VQQPDCR |
| C4Alpha_[730-743]_Cys_CAM: 735, 736 | EPFLSCCQFAESLR |
| C4Alpha_[750-756] | GQAGLQR |
| C4Alpha_[757-775] | ALEILQEEDLIDEDDIPVR |
| C4Alpha_[776-785] | SFFPENWLWR |
| C4Alpha_[786-791] | VETVDR |
| C4Alpha_[792-815] | FQILTLWLPDSLTTWEIHGLSLSK |
| C4Alpha_[818-828] | GLCVATPVQLR |
| C4Alpha_[818-828]_Cys_CAM: 820 | GLCVATPVQLR |
| C4Alpha_[832-838] | EFHLHLR |
| C4Alpha_[846-861] | FEQLELRPVLYNYLDK |
| C4Alpha_[862-912] | NLTVSVHVSPVEGLCLAGGGGLAQQVLVPAGSA RPVAFSVVPTAATAVSLK |
| C4Alpha_[862-912]_Cys_CAM: 876 | NLTVSVHVSPVEGLCLAGGGGLAQQVLVPAGSA RPVAFSVVPTAATAVSLK |
| C4Alpha_[913-916] | VVAR |
| C4Alpha_[917-929] | GSFEFPVGDAVSK |
| C4Alpha_[936-941] | EGAIHR |
| C4Alpha_[942-954] | EELVYELNPLDHR |
| C4Alpha_[957-979] | TLEIPGNSDPNMIPDGDFNSYVR |
| C4Alpha_[980-1005] | VTASDPLDTLGSEGALSPGGVASLLR |
| C4Beta_[105-118] | GPEVQLVAHSPWLK |
| C4Beta-[119-123] | DSLSR |
| C4Beta_[124-137] | TTNIQGINLLFSSR |
| C4Beta_[139-155] | GHLFLQTDQPIYNPGQR |
| C4Beta_[158-159] | YR |
| C4Beta_[160-166] | VFALDQK |
| C4Beta_[167-185] | MRPSTDTITVMVENSHGLR |
| C4Beta_[190-214] | EVYMPSSIFQDDFVIPDISEPGTWK |
| C4Beta_[219-234] | FSDGLESNSSTQFEVK |
| C4Beta_[23-48] | LLLFSPSVVHLGVPLSVGVQLQDVPR |
| C4Beta_[236-244] | YVLPNFEVK |
| C4Beta_[245-269] | ITPGKPYILTVPGHLDEMQLDIQAR |
| C4Beta_[270-283] | YIYGKPVQGVAYVR |
| C4Beta_[284-292] | FGLLDEDGK |
| C4Beta_[294-297] | TFFR |
| C4Beta_[298-304] | GLESQTK |
| C4Beta_[305-316] | LVNGQSHISLSK |
| C4Beta_[326-337] | LNMGITDLQGLR |
| C4Beta_[338-373] | LYVAAAIIESPGGEMEEAELTSWYFVSSPFSLDLS K |
| C4Beta_[392-404] | EMSGSP ASGIPVK |
| C4Beta_[405-459] | VSATVSSPGSVPEVQDIQQNTDGSGQVSIPIIIPQTI |
| | SELQLSVSAGSPHPAIAR |
| C4Beta_[460-484] | LTVAAPPSGGPGFLSIERPDSRPPR |
| C4Beta_[485-494] | VGDTLNLNLR |
| C4Beta_[49-53] | GQVVK |
| C4Beta_[495-512] | AVGSGATFSHYYYMILSR |
| C4Beta_[513-520] | GQIVFMNR |
| C4Beta_[521-523] | EPK |
| C4Beta_[525-559] | TLTSVSVFVDHHLAPSFYFVAFYYHGDHPVANS LR |
| C4Beta_[560-570] | VDVQAGACEGK |
| C4Beta_[560-570]_Cys_CAM: 567 | VDVQAGACEGK |
| C4Beta_[571-579] | LELSVDGAK |
| C4Beta_[580-582] | QYR |
| C4Beta_[583-588] | NGESVK |
| C4Beta_[589-614] | LHLETDSLALVALGALDTALYAAGSK |
| C4Beta_[60-63] | NPSR |
| C4Beta_[615-623] | SHKPLNMGK |
| C4Beta_[624-664] | VFEAMNSYDLGCGPGGGDSALQVFQAAGLAFS DGDQWTLSR |
| C4Beta_[624-664]_Cys_CAM: 635 | VFEAMNSYDLGCGPGGGDSALQVFQAAGLAFS DGDQWTLSR |
| C4Beta_[64-71] | NNVPCSPK |
| C4Beta_[64-71]_Cys_CAM: 68 | NNVPCSPK |
| C4Beta_[667-671] | LSCPK |
| C4Beta_[667-671]_Cys_CAM: 669 | LSCPK |
| C4Beta_[72-80] | VDFTLSSER |
| C4Beta_[81-92] | DFALLSLQVPLK |
| C4Beta_[93-95] | DAK |
| C4Beta_[96-104] | SCGLHQLLR |
| C4Beta_[96-104]_Cys_CAM: 97 | SCGLHQLLR |
| C4Gamma_[1458-1465] | VVEEQESR |
| C4Gamma_[1466-1474] | VHYTVCIWR |
| C4Gamma_[1466-1474]_Cys_CAM: 1471 | VHYTVCIWR |
| C4Gamma_[1475-1477] | NGK |
| C4Gamma_[1478-1498] | VGLSGMAIADVTLLSGFHALR |
| C4Gamma_[1499-1503] | ADLEK |
| C4Gamma_[1504-1510] | LTSLSDR |
| C4Gamma_[1511-1533] | YVSHFETEGPHVLLYFDSVPTSR |
| C4Gamma_[1534-1564] | ECVGFEAVQEVPVGLVQPASATLYDYYNPER |
| C4Gamma_[1534-1564]_Cys_CAM: 1535 | ECVGFEAVQEVPVGLVQPASATLYDYYNPER |
| C4Gamma_[1566-1575] | CSVFYGAPSK |
| C4Gamma_[1566-1575]_Cys_CAM: 1566 | CSVFYGAPSK |
| C4Gamma_[1578-1594] | LLATLCSAEVCQCAEGK |
| C4Gamma_[1578-1594]_Cys_CAM: 1583, 1588,159 0 | LLATLCSAEVCQCAEGK |
| C4Gamma_[1595-1597] | CPR |
| C4Gamma_[1595-1597]_Cys_CAM: 1595 | CPR |
| C4Gamma_[1601-1604] | ALER |
| C4Gamma_[1616-1622] | FACYYPR |
| C4Gamma_[1616-1622]_Cys_CAM: 1618 | FACYYPR |
| C4Gamma_[1623-1630] | VEYGFQVK |
| C4Gamma_[1631-1633] | VLR |
| C4Gamma_[1638-1641] | AAFR |
| C4Gamma_[1642-1646] | LFETK |
| C4Gamma_[1656-1658] | DVK |
| C4Gamma_[1659-1665] | AAANQMR |
| C4Gamma_[1671-1674] | ASCR |
| C4Gamma_[1677-1681] | LEPGK |
| C4Gamma_[1682-1716] | EYLIMGLDGATYDLEGHPQYLLDSNSWIEEMPSE R |
| C4Gamma_[1720-1722] | STR |
| C4Gamma_[1725-1744] | AACAQLNDFLQEYGTQGCQV |
| C4Gamma_[1725-1744]_Cys_CAM: 1727, 1742 | AACAQLNDFLQEYGTQGCQV |

All of the Complement C4 tryptic peptides can be used to differentiate between healthy subjects and hereditary angioedema patients. for of the peptides, namely C4Alpha_[680-685] with MRM transition 375.2 → 536.28, C4Alpha_[786-791] with MRM transition 359.69→ 490.26, C4Beta_[294-297] with MRM transition 285.66 → 322.19, C4Beta_[571-579] with MRM transition 466.26 → 243.13, and C4 gamma_[1638-1641] with MRM transition 232.64→ 322.19] were used as representative examples (see Example 3).

For complement C1-INH (also referred to as SerpinG1), the following tryptic peptides could be quantified, whereby the numbers in the brackets represents the position of the first amino acid and the last amino acid of the peptide in amino acid sequence of C1-INH (with the sequences being indicated with the N-terminus being at the left side and the C-terminus being at the right side). Such peptides are shown in Table 4.

**Table 4:**

| **Peptide** | **Sequence (N-terminus -> C-terminus)** |
|---|---|
| SerpinG1_[202-211] | DFTCVHQALK |
| SerpinG1_[202-211]_Cys_CAM: 205 | DFTCVHQALK |
| SerpinG1_[212-216] | GFTTK |
| SerpinG1_[217-233] | GVTSVSQIFHSPDLAIR |
| SerpinG1_[23-40] | NPNATSSSSQDPESLQDR |
| SerpinG1_[234-241] | DTFVNASR |
| SerpinG1_[242-249] | TLYSSSPR |
| SerpinG1_[250-268] | VLSNNSDANLELINTWVAK |
| SerpinG1_[269-273] | NTNNK |
| SerpinG1_[274-276] | ISR |
| SerpinG1_[277-286] | LLDSLPSDTR |
| SerpinG1_[301-306] | TTFDPK |
| SerpinG1_[310-316] | MEPFHFK |
| SerpinG1_[322-328] | VPMMNSK |
| SerpinG1_[330-341] | YPVAHFIDQTLK |
| SerpinG1_[344-364] | VGQLQLSHNLSLVILVPQNLK |
| SerpinG1_[367-380] | LEDMEQALSPSVFK |
| SerpinG1_[381-385] | AIMEK |
| SerpinG1_[386-390] | LEMSK |
| SerpinG1_[391-400] | FQPTLLTLPR |
| SerpinG1_[403-415] | VTTSQDMLSIMEK |
| SerpinG1_[41-44] | GEGK |
| SerpinG1_[416-466] | LEFFDFSYDLNLCGLTEDPDLQVSAMQHQTVLELTETG VEAAAASAISVAR |
| SerpinG1_[467-487] | TLLVFEVQQPFLFVLWDQQHK |
| SerpinG1_[488-494] | FPVFMGR |
| SerpinG1_[495-499] | VYDPR |
| SerpinGl_[53-77] | MLFVEPILEVSSLPTTNSTTNSATK |

All the complement C1-INH tryptic peptides can be used to differentiate between healthy subjects and hereditary angioedema patients. Two peptides, namely SerpinG1_[242-249] with MRM transition 455.74 → 696.33 and SerpinG1[391-400] with MRM transition 593.35→ 455.79], were used as representative example (see Example 3).

### Example 3: Quantifying tryptic peptide fragments of the proteins C3, Clq, C4 and C1-INH in DBS extract from healthy subjects and hereditary angioedema patients with known pathogenic variants in Serping1 gene.

### HAE patients

All patients with hereditary angioedema disease type 1/2 or of whom it was strongly assumed that they were suffering from hereditary angioedema disease type 1/2 sent to the participating centers were included into the study. SerpinG1 mutations were confirmed in all the patients taken in consideration for this study using techniques such as next generation sequencing, Sanger sequencing and / or multiplexed ligation dependent probe amplification.

### Protein C4

Using the methods outlined in Example 1, the content of peptide fragments C4Alpha_[680-685], C4Alpha_[786-791], C4Beta_[294-297], C4Beta_[571-579] and C4 gamma_[1638-1641] of protein C4 was quantified in DBS from a total of270 healthy subjects. Similarly, the content of peptide fragments C4Alpha_[680-685], C4Alpha_[786-791], C4Beta_[294-297], C4Beta_[571-579] and C4 gamma_[1638-1641] of the protein C4 was quantified in DBS from a total of 135 previously genetically diagnosed hereditary angioedema patients.

For this assay, pure synthetic peptides were obtained and used to obtain a standard curve used to quantify the peptides originating from blood samples. The linearity of the standard curve is reflected in R² values in the following Table 5.

**Table 5:**

| **Protein** | **Sequence** | **Molecular weight** | **Parent ion charge state** | **Transition** | **Retention time** | **R² Linearity** |
|---|---|---|---|---|---|---|
| C4Beta [571-579] | LELSVDGAK | 930.50 | 2+ | 466.26/243.13 | 3.5 | 0.998109 |
| C4Alpha [680-685] | NVNFQK | 748.39 | 2+ | 375.2/536.28 | 2.4 | 0.999882 |
| C4Alpha [786-791] | VETVDR | 717.37 | 2+ | 359.69/490.26 | 2 | 0.999694 |
| C4Beta [294-297] | TFFR | 569.30 | 2+ | 285.66/322.19 | 3.1 | 0.987227 |
| C4Gamma [1638-1641] | AAFR | 463.25 | 2+ | 232.64/322.19 | 2 | 0.999615 |

As shown in Table 6 below, peptides C4Alpha[680-685], C4Alpha_[786-791], C4Beta_[294-297], C4Beta_[571-579] and C4Gamma_[1638-1641] of protein C4 were reduced in a statistically significant manner in hereditary angioedema patients compared to healthy subjects (p<0,0001). The values for the various peptides are in ng/ml.

**Table 6:**

| **Peptide** | | **Controls** | **HAE Type 1** | **HAE Type 2** |
|---|---|---|---|---|
| | **Number of values (N)** | **270** | **118** | **17** |
| | Minimum | 500 | 6.25 | 25 |
| | 25% Percentile | 1075 | 6.25 | 56.25 |
| | Median | 1572 | 37.5 | 165.6 |
| | 75% Percentile | 2408 | 228.1 | 298.4 |
| **C4Beta**_**[571-579]** | Maximum | 3400 | 475 | 375 |
| | Mean | 1760 | 114.3 | 173 |
| | Std. Deviation | 770.2 | 131.3 | 122.5 |
| | Std. Error of Mean | 47.22 | 12.3 | 30.62 |
| | Lower 95% CI of mean | 1667 | 89.94 | 107.8 |
| | Upper 95% CI of mean | 1853 | 138.7 | 238.3 |
| | Minimum | 268.8 | 0 | 0 |
| | 25% Percentile | 462.5 | 0 | 6.25 |
| | Median | 687.5 | 12.5 | 43.75 |
| | 75% Percentile | 943.8 | 50 | 100 |
| **C4Alpha_[680-685]** | Maximum | 2131 | 237.5 | 137.5 |
| | Mean | 723.5 | 36.74 | 52.21 |
| | Std. Deviation | 327.7 | 50.34 | 47.18 |
| | Std. Error of Mean | 19.98 | 4.694 | 11.44 |
| | Lower 95% CI of mean | 684.1 | 27.44 | 27.95 |
| | Upper 95% CI of mean | 762.8 | 46.04 | 76.47 |
| | Minimum | 100 | 0 | 0 |
| | 25% Percentile | 350 | 0 | 7.813 |
| | Median | 531.3 | 12.5 | 18.75 |
| | 75% Percentile | 775 | 31.25 | 31.25 |
| **C4Alpha_[786-791]** | Maximum | 1444 | 75 | 43.75 |
| | Mean | 590.9 | 16.39 | 19.14 |
| | Std. Deviation | 329.7 | 19.96 | 13 |
| | Std. Error of Mean | 20.49 | 1.87 | 3.251 |
| | Lower 95% CI of mean | 550.5 | 12.69 | 12.21 |
| | Upper 95% CI of mean | 631.2 | 20.1 | 26.07 |
| | Minimum | 218.8 | 0 | 25 |
| | 25% Percentile | 437.5 | 0 | 43.75 |
| | Median | 634.4 | 18.75 | 75 |
| | 75% Percentile | 831.3 | 62.5 | 162.5 |
| **C4Beta_[294-297]** | Maximum | 1488 | 200 | 187.5 |
| | Mean | 657.5 | 35.84 | 95.31 |
| | Std. Deviation | 259 | 49.89 | 58.96 |
| | Std. Error of Mean | 15.76 | 4.613 | 14.74 |
| | Lower 95% CI of mean | 626.4 | 26.71 | 63.89 |
| | Upper 95% CI of mean | 688.5 | 44.98 | 126.7 |
| | Minimum | 975 | 0 | 218.8 |
| | 25% Percentile | 1980 | 0 | 309.4 |
| | Median | 2659 | 181.3 | 418.8 |
| **C4Gamma_[1638-1641]** | 75% Percentile | 3381 | 318.8 | 715.6 |
| | Maximum | 5550 | 912.5 | 962.5 |
| | Mean | 2704 | 187.7 | 500.8 |
| | Std. Deviation | 969.8 | 214.9 | 233 |
| | Std. Error of Mean | 59.02 | 19.7 | 58.25 |
| | Lower 95% CI of mean | 2588 | 148.6 | 376.6 |
| | Upper 95% CI of mean | 2820 | 226.7 | 624.9 |

### Protein C1-INH

Using the methods outlined in Example 1, the content of peptide fragments SerpinG1_[242-249] and SerpinG1_[391-400] of protein C1-INH were quantified in DBS from a total of 270 healthy subjects. Similarly, the content of peptide fragments SerpinG_[242-249] and SerpinG1_[391-400] of protein C1-INH was quantified in DBS from a total of 135 previously genetically diagnosed hereditary angioedema patients.

For this assay, pure synthetic peptides were obtained and used to obtain a standard curve used to quantify the peptides originating from blood samples. The linearity of the standard curve is reflected in R² values in the following Table 7:

**Table 7:**

| **Protein** | **Sequence** | **Molecular weight** | **Parent ion charge state** | **Transition** | **Retention time** | **R² Linearity** |
|---|---|---|---|---|---|---|
| SerpinG1[242-249] | TLYSSSPR | 909.46 | 2+ | 455.74/696.33 | 2.6 | 0.999621 |
| SerpinG1[391-400] | FQPTLLTLPR | 1184.69 | 2+ | 593.35/455.79 | 4.8 | 0.972887 |

As shown in Table 8 below, SerpinG_[242-249] and SerpinG1_[391-400] of protein C1-IHN were reduced in a statistically significant manner in hereditary angioedema patients type 1 in comparison to healthy subjects (p<0,0001). The values for the various peptides are in ng/ml.

**Table 8:**

| **Peptide** | | **Controls** | **HAE Type 1** | **HA E Type 2** |
|---|---|---|---|---|
| | **Number of values (N)** | **270** | **118** | **17** |
| **SerpinG1_[242-249]** | Minimum | 1113 | 0 | 837.5 |
| | 25% Percentile | 2606 | 0 | 884.4 |
| | Median | 3291 | 190.6 | 2463 |
| | 75% Percentile | 4081 | 381.3 | 6156 |
| | Maximum | 6475 | 831.3 | 6394 |
| | Mean | 3412 | 223.1 | 2956 |
| | Std. Deviation | 1174 | 228.9 | 2319 |
| | Std. Error of Mean | 71.43 | 21.07 | 562.4 |
| | Lower 95% CI of mean | 3272 | 181.4 | 1764 |
| | Upper 95% CI of mean | 3553 | 264.8 | 4148 |
| **SerpinG1_[391-400]** | Minimum | 675 | 0 | 418.8 |
| | 25% Percentile | 1566 | 0 | 640.6 |
| | Median | 2169 | 81.25 | 1500 |
| | 75% Percentile | 2930 | 189.1 | 5406 |
| | Maximum | 5069 | 393.8 | 6169 |
| | Mean | 2322 | 107.4 | 2384 |
| | Std. Deviation | 985.7 | 113.8 | 2284 |
| | Std. Error of Mean | 59.99 | 10.47 | 553.9 |
| | Lower 95% CI of mean | 2204 | 86.62 | 1210 |
| | Upper 95% CI of mean | 2440 | 128.1 | 3558 |

For all samples included in the study, the HAE type 1 patients could be distinguished from healthy controls (non-HAE).

### Protein C1q

Using the methods outlined in Example 1, the content of peptide fragments C1q-B_[178-186] and C1q-B_[63-77] of protein C1q were quantified in DBS from a total of 270 healthy subjects. Similarly, the content of peptide fragments C1q-B_[178-186] and Clq-B_[63-77] of the protein C1q were determined in DBS from a total of 135 previously genetically diagnosed hereditary angioedema patients.

For this assay, pure synthetic peptides were obtained and used to obtain a standard curve used to quantify the peptides originating from blood samples. The linearity of the standard curve is reflected in R² values in the following Table 9.

**Table 9:**

| **Protein** | **Sequence** | **Molecular weight** | **Parent ion charge state** | **Transition** | **Retention time** | **R² Linearity** |
|---|---|---|---|---|---|---|
| C1qBeta[178-186] | GNLCVNLMR | 1018.51 | 2+ | 510.26/254.58 | 3.9 | 0.983744 |
| C1q-B[63-77] | GLPGLAGD HGEFGEK | 1482.7104 | 3+ | 495.25/774.5 | 4.1 | 0.973842 |

As shown in Table 10 below, peptide fragments C1q-B_[178-186] and C1q-B_[63-77] of protein C1q were not significantly reduced in a statistically significant manner in hereditary angioedema patients type 1 compared to healthy subjects. The values for the various peptides are in ng/ml.

**Table 10:**

| **Peptide** | | **Controls** | **HAE Type 1** | **HAE Type 2** |
|---|---|---|---|---|
| | **Number of values (N)** | **270** | **118** | **17** |
| **C1q-B_[178-186]** | Minimum | 1000 | 247 | |
| | 25% Percentile | 2982 | 3857 | |
| | Median | 4790 | 4750 | |
| | 75% Percentile | 6240 | 5355 | |
| | Maximum | 9365 | 5916 | |
| | Mean | 4698 | 4250 | |
| | Std. Deviation | 2062 | 1647 | |
| | Std. Error of Mean | 291.6 | 425.3 | |
| | Lower 95% CI of mean | 4112 | 3338 | |
| | Upper 95% CI of mean | 5284 | 5162 | |
| **C1q-B_[63-77]** | Minimum | 1783 | 552.6 | |
| | 25% Percentile | 6262 | 5635 | |
| | Median | 8212 | 7196 | |
| | 75% Percentile | 9666 | 8200 | |
| | Maximum | 11821 | 9729 | |
| | Mean | 7651 | 6684 | |
| | Std. Deviation | 2652 | 2183 | |
| | Std. Error of Mean | 364.2 | 563.7 | |
| | Lower 95% CI of mean | 6920 | 5475 | |
| | Upper 95% CI of mean | 8382 | 7893 | |

### Protein C3

Using the methods outlined in Example 1, the content of peptide fragment C3Beta_[250-258] of protein C3 was quantified in DBS from a total of 270 healthy subjects. Similarly, the content of peptide fragment C3Beta_[250-258] of protein C3 was quantified in DBS from a total of 135 previously genetically diagnosed hereditary angioedema patients.

For this assay, pure synthetic peptides were obtained and used to obtain a standard curve used to quantify the peptides originating from blood samples. The linearity of the standard curve is reflected in R² values in the following Table 11.

**Table 11:**

| **Protein** | **Sequence** | **Molecular weight** | **Parent ion charge state** | **Transition** | **Retention time** | **R² Linearity** |
|---|---|---|---|---|---|---|
| C3Beta[250-258] | GLEVTITAR | 958.54 | 2+ | 480.28/ 660.4 | 3.7 | 0.943337 |

As shown in Table 12 below, C3Beta_[250-258] of the protein C1q is not reduced in a statistically significant matter in hereditary angioedema patients type 1 compared to healthy subjects. The values for the various peptides are in ng/ml.

**Table 12:**

| **Peptide** | | **Controls** | **HAE Type 1** | **HAE Type 2** |
|---|---|---|---|---|
| | **Number of values (N)** | **270** | **118** | **17** |
| **C3Beta_[250-258]** | Minimum | 1 | 0.0 | |
| | 25% Percentile | 3.785 | 0.0300 | |
| | Median | 6.73 | 2.840 | |
| | 75% Percentile | 10.36 | 3.960 | |
| | Maximum | 15.78 | 8.710 | |
| | Mean | 7.07 | 2.339 | |
| | Std. Deviation | 3.923 | 2.347 | |
| | Std. Error of Mean | 0.2466 | 0.2020 | |
| | Lower 95% CI of mean | 6.584 | 1.940 | |
| | Upper 95% CI of mean | 7.555 | 2.739 | |

### Example 4: Determination of biomarker cut-off levels in DBS extract from healthy subjects and hereditary angioedema patients with known pathogenic variants in Serping1 gene.

Based on the data and results of Example 3 and using synthetic peptides as calibration standards for SerpinG_[242-249], C4Alpha_[680-685], C4Alpha_[786-791], C4Beta_[294-297], C4Beta_[571-579], C1q-Beta_[178-186], C4Gamma_[1638-1641], SerpinGl_[391-400] and C1q-Beta_[63-77] a cut-off level was determined empirically for each peptide.

| **Peptide** | **Cut-off** |
|---|---|
| C4Beta_[571-579] | 500 ng/mL |
| SerpinG1_[242-249] | 835 ng/mL |
| C1q-Beta_[178-186] | 800 ng/mL |
| C4Alpha_[680-685] | 260 ng/mL |
| C4Alpha_[786-791] | 100 ng/mL |
| C4Beta_[294-297] | 201 ng/mL |
| C4Gamma_[1638-1641] | 920 ng/mL |
| SerpinG1_[391-400] | 392 ng/mL |
| C1q-Beta_[63-77] | 1690 ng/mL |

The results are shown in Figs. 1 to 9.

For all samples included in the study, the HAE patients could be distinguished from the healthy controls (non-HAE, NC).

The features of the present invention disclosed in the specification, the claims, the sequence listing and/or the drawings may both separately and in any combination thereof be material for realizing the invention in various forms thereof.

## Claims

1. A method for differential diagnosis of hereditary angioedema, wherein the method comprises determining the level of C4 protein, C1-INH protein and C1q protein in a sample from a subject, wherein the sample is a dried blood spot sample and wherein the level is determined by mass spectrometry.

2. The method of claim 1, wherein determining the level of C4 protein comprises detecting and quantifying the level of a C4 fragment peptide, wherein determining the level of C1-INH protein comprises detecting and quantifying the level of a C1-INH fragment peptide, and wherein determining the level of C1q protein comprises detecting and quantifying the level of a C1 q fragment peptide.

3. The method of any one of claims 1 to 2, wherein if the sample tests negative for C4 protein and tests positive for C1-INH protein and C1q protein, the subject is suffering from hereditary angioedema type II.

4. The method of any one of claims 1 to 2, wherein if the sample tests negative for C4 protein and C1-INH protein and tests positive for C1q protein, the subject is suffering from hereditary angioedema type I.

5. The method of any one of claims 1 to 4, wherein the method is a method for differentiating between hereditary angioedema type I and hereditary angioedema type II.

6. The method of any one of claims 1 to 5, wherein a sample tests positive for C4 protein if the level of C4 protein or of a C4 fragment peptide is higher than a cut-off value, and tests negative if the level ofC4 protein or of a C4 fragment peptide is lower than a cut-off value.

7. The method of any one of claims 1 to 6, wherein a sample tests positive for C1-INH protein if the level of C1-INH protein or of a C1-INH fragment peptide is higher than a cut-off value, and tests negative if the level of C1-INH protein or of a C1-INH fragment peptide is lower than a cut-off value.

8. The method of any one of claims 1 to 7, wherein a sample tests positive for C1q protein if the level of C1q protein or of a C1q fragment peptide is higher than a cut-off value, and tests negative if the level of C1q protein or of a C1q fragment peptide is lower than a cut-off value.

9. The method of any one of claims 1 to 8, wherein the C4 fragment peptide, the C1-INH fragment peptide, the C1q fragment peptide and/or the C3 fragment peptide is prepared from the sample by a protease digest or a peptidase digest.

10. The method of claim 9, wherein the protease is selected from the group comprising Arg-C, Asp-N, Asp-N (N-terminal Glu), BNPS or NCS/urea, Caspase-1, Caspase-10, Caspase-2, Caspase-3, Caspase-4, Caspase-5, Caspase-6, Caspase-7, Caspase-8, Caspase-9, Chymotrypsin, Chymotrypsin (low specificity), Clostripain, CNBr, CNBr (methyl-Cys), CNBr (with acids), Enterokinase, Factor Xa, Formic acid, Glu-C (AmAc buffer, Glu-C (Phos buffer), Granzyme B, HRV3C protease, Hydroxylamine, Iodosobenzoic acid, Lys-C, Lys-N, Lys-N (Cys modified), Mild acid hydrolysis, NBS (long exposure), NBS (short exposure), NTCB, Pancreatic elastase, Pepsin A, Pepsin A (low specificity), Prolyl endopeptidase, Proteinase K, TEV protease, Thermolysin, Thrombin.

11. The method of claim 10, wherein the protease is trypsin or pepsin, preferably trypsin.

12. The method of any one of claims 2 to 11, wherein the C4 fragment peptide is selected from the group consisting of
| **Peptide** | **Sequence (N-terminus -> C-terminus)** |
|---|---|
| C4Alpha_[1006-1008] | LPR |
| C4Alpha_[1009-1026] | GCGEQTMIYLAPTLAASR |
| C4Alpha_[1009-1026]_Cys_CAM: 1010 | GCGEQTMIYLAPTLAASR |
| C4Alpha_[1027-1030] | YLDK |
| C4Alpha_[1031-1042] | TEQWSTLPPETK |
| C4Alpha_[1043-1051] | DHAVDLIQK |
| C4Alpha_[1052-1055] | GYMR |
| C4Alpha_[1062-1072] | ADGSYAAWLSR |
| C4Alpha_[1073-1084] | GSSTWLTAFVLK |
| C4Alpha_[1085-1099] | VLSLAQEQVGGSPEK |
| C4Alpha_[1100-1126] | LQETSNWLLSQQQADGSFQDLSPVIHR |
| C4Alpha_[1168-1174] | VEASISK |
| C4Alpha_[1175-1182] | ASSFLGEK |
| C4Alpha_[1183-1204] | ASAGLLGAHAAAITAYALTLTK |
| C4Alpha_[121 1-1248] | GVAHNNLMAMAQETGDNLYWGSVTGSQSNAVSPTPAPR |
| C4Alpha_[1249-1278] | NPSDPMPQAPALWIETTAYALLHLLLHEGK |
| C4Alpha_[1279-1291] | AEMADQAAAWLTR |
| C4Alpha_[1292-1300] | QGSFQGGFR |
| C4Alpha_[1301-1325] | STQDTVIALDALSAYWIASHTTEER |
| C4Alpha_[1326-1336] | GLNVTLSSTGR |
| C4Alpha_[1337-1340] | NGFK |
| C4Alpha_[1341-1349] | SHALQLNNR |
| C4Alpha_[1350-1352] | QIR |
| C4Alpha_[1353-1365] | GLEEELQFSLGSK |
| C4Alpha_[1370-1375] | VGGNSK |
| C4Alpha_[1383-1390] | TYNVLDMK |
| C4Alpha_[1391-1404] | NTTCQDLQIEVTVK |
| C4Alpha_[1391-1404]_Cys_CAM: 1394 | NTTCQDLQIEVTVK |
| C4Alpha_[1405-1428] | GHVEYTMEANEDYEDYEYDELPAK |
| C4Alpha_[1429-1446] | DDPDAPLQPVTPLQLFEG |
| C4Alpha_[680-685] | NVNFQK |
| C4Alpha_[686-690] | AINEK |
| C4Alpha_[691-700] | LGQYASPTAK |
| C4Alpha_[702-709]_Cys_CAM: 702, 703 | CCQDGVTR |
| C4Alpha_[710-714] | LPMMR |
| C4Alpha_[715-719]_Cys_CAM: 716 | SCEQR |
| C4Alpha_[723-729] | VQQPDCR |
| C4Alpha_[723-729]_Cys_CAM: 728 | VQQPDCR |
| C4Alpha_[730-743]_Cys_CAM: 735, 736 | EPFLSCCQFAESLR |
| C4Alpha_[750-756] | GQAGLQR |
| C4Alpha_[757-775] | ALEILQEEDLIDEDDIPVR |
| C4Alpha_[776-785] | SFFPENWLWR |
| C4Alpha_[786-791] | VETVDR |
| C4Alpha_[792-815] | FQILTLWLPDSLTTWEIHGLSLSK |
| C4Alpha_[818-828] | GLCVATPVQLR |
| C4Alpha_[818-828]_Cys_CAM: 820 | GLCVATPVQLR |
| C4Alpha_[832-838] | EFHLHLR |
| C4Alpha_[846-861] | FEQLELRPVLYNYLDK |
| C4Alpha_[862-912] | NLTVSVHVSPVEGLCLAGGGGLAQQVLVPAGSA RPVAFSVVPTAATAVSLK |
| C4Alpha_[862-912]_Cys_CAM: 876 | NLTVSVHVSPVEGLCLAGGGGLAQQVLVPAGSA RPVAFSVVPTAATAVSLK |
| C4Alpha_[913-916] | VVAR |
| C4Alpha_[917-929] | GSFEFPVGDAVSK |
| C4Alpha_[936-941] | EGAIHR |
| C4Alpha_[942-954] | EELVYELNPLDHR |
| C4Alpha_[957-979] | TLEIPGNSDPNMIPDGDFNSYVR |
| C4Alpha_[980-1005] | VTASDPLDTLGSEGALSPGGVASLLR |
| C4Beta_[105-118] | GPEVQLVAHSPWLK |
| C4Beta_[119-123] | DSLSR |
| C4Beta_[124-137] | TTNIQGINLLFSSR |
| C4Beta_[139-155] | GHLFLQTDQPIYNPGQR |
| C4Beta_[158-159] | YR |
| C4Beta_[160-166] | VFALDQK |
| C4Beta_[167-185] | MRPSTDTITVMVENSHGLR |
| C4Beta_[190-214] | EVYMPSSIFQDDFVIPDISEPGTWK |
| C4Beta_[219-234] | FSDGLESNSSTQFEVK |
| C4Beta_[23-48] | LLLFSPSWHLGVPLSVGVQLQDVPR |
| C4Beta_[236-244] | YVLPNFEVK |
| C4Beta_[245-269] | ITPGKPYILTVPGHLDEMQLDIQAR |
| C4Beta_[270-283] | YIYGKPVQGVAYVR |
| C4Beta_[284-292] | FGLLDEDGK |
| C4Beta_[294-297] | TFFR |
| C4Beta_[298-304] | GLESQTK |
| C4Beta_[305-316] | LVNGQSHISLSK |
| C4Beta_[326-337] | LNMGITDLQGLR |
| C4Beta_[338-373] | LYVAAAIIESPGGEMEEAELTSWYFVSSPFSLDLSK |
| C4Beta_[392-404] | EMSGSPASGIPVK |
| C4Beta_[405-459] | VSATVSSPGSVPEVQDIQQNTDGSGQVSIPIIIPQTI SELQLSVSAGSPHPAIAR |
| C4Beta_[460-484] | LTVAAPPSGGPGFLSIERPDSRPPR |
| C4Beta_[485-494] | VGDTLNLNLR |
| C4Beta_[49-53] | GQVVK |
| C4Beta_[495-512] | AVGSGATFSHYYYMILSR |
| C4Beta_[513-520] | GQIVFMNR |
| C4Beta_[521-523] | EPK |
| C4Beta_[525-559] | TLTSVSVFVDHHLAPSFYFVAFYYHGDHPVANSLR |
| C4Beta_[560-570] | VDVQAGACEGK |
| C4Beta_[560-570]_Cys_CAM: 567 | VDVQAGACEGK |
| C4Beta_[571-579] | LELSVDGAK |
| C4Beta_[580-582] | QYR |
| C4Beta_[583-588] | NGESVK |
| C4Beta_[589-614] | LHLETDSLALVALGALDTALYAAGSK |
| C4Beta_[60-63] | NPSR |
| C4Beta_[615-623] | SHKPLNMGK |
| C4Beta_[624-664] | VFEAMNSYDLGCGPGGGDSALQVFQAAGLAFSDGDQWTLSR |
| C4Beta_[624-664]_Cys_CAM: 635 | VFEAMNSYDLGCGPGGGDSALQVFQAAGLAFS DGDQWTLSR |
| C4Beta_[64-71] | NNVPCSPK |
| C4Beta_[64-71]_Cys_CAM: 68 | NNVPCSPK |
| C4Beta_[667-671] | LSCPK |
| C4Beta_[667-671]_Cys_CAM: 669 | LSCPK |
| C4Beta_[72-80] | VDFTLSSER |
| C4Beta_[81-92] | DFALLSLQVPLK |
| C4Beta_[93-95] | DAK |
| C4Beta_[96-104] | SCGLHQLLR |
| C4Beta_[96-104]_Cys_CAM: 97 | SCGLHQLLR |
| C4Gamma_[1458-1465] | VVEEQESR |
| C4Gamma_[1466-1474] | VHYTVCIWR |
| C4Gamma_[1466-1474]_Cys_CAM: 1471 | VHYTVCIWR |
| C4Gamma_[1475-1477] | NGK |
| C4Gamma_[1478-1498] | VGLSGMAIADVTLLSGFHALR |
| C4Gamma_[1499-1503] | ADLEK |
| C4Gamma_[1504-1510] | LTSLSDR |
| C4Gamma_[1511-1533] | YVSHFETEGPHVLLYFDSVPTSR |
| C4Gamma_[1534-1564] | ECVGFEAVQEVPVGLVQPASATLYDYYNPER |
| C4Gamma_[1534-1564]_Cys_CAM: 1535 | ECVGFEAVQEVPVGLVQPASATLYDYYNPER |
| C4Gamma_[1566-1575] | CSVFYGAPSK |
| C4Gamma_[1566-1575]_Cys_CAM: 1566 | CSVFYGAPSK |
| C4Gamma_[1578-1594] | LLATLCSAEVCQCAEGK |
| C4Gamma_[1578-1594]_Cys_CAM: 1583, 1588, 159 0 | LLATLCSAEVCQCAEGK |
| C4Gamma_[1595-1597] | CPR |
| C4Gamma_[1595-1597]_Cys_CAM: 1595 | CPR |
| C4Gamma_[1601-1604] | ALER |
| C4Gamma_[1616-1622] | FACYYPR |
| C4Gamma_[1616-1622]_Cys_CAM: 1618 | FACYYPR |
| C4Gamma_[1623-1630] | VEYGFQVK |
| C4Gamma_[1631-1633] | VLR |
| C4Gamma_[1638-1641] | AAFR |
| C4Gamma_[1642-1646] | LFETK |
| C4Gamma_[1656-1658] | DVK |
| C4Gamma_[1659-1665] | AAANQMR |
| C4Gamma_[1671-1674] | ASCR |
| C4Gamma_[1677-1681] | LEPGK |
| C4Gamma_[1682-1716] | EYLIMGLDGATYDLEGHPQYLLDSNSWIEEMPSER |
| C4Gamma_[1720-1722] | STR |
| C4Gamma_[1725-1744] | AACAQLNDFLQEYGTQGCQV |
| C4Gamma_[1725-1744]_Cys_CAM: 1727, 1742 | AACAQLNDFLQEYGTQGCQV |

13. The method of claim 12, wherein the C4 fragment is selected from the group consisting of C4Beta[571-579], C4Alpha[680-685], C4Alpha[786-791], C4Beta[294-297] and C4Gamma[1638-1641], preferably the C4 fragment is C4Beta[571-579].

14. The method of any one of claims 2 to 13, wherein the C1-INH fragment peptide is selected from the group consisting of
| Peptide | Sequence (N-terminus -> C-terminus) |
|---|---|
| SerpinG1_[202-211] | DFTCVHQALK |
| SerpinG1_[202-211]_Cys_CAM: 205 | DFTCVHQALK |
| SerpinG1_[212-216] | GFTTK |
| SerpinG1_[217-233] | GVTSVSQIFHSPDLAIR |
| SerpinG1_[23-40] | NPNATSSSSQDPESLQDR |
| SerpinG1_[234-241] | DTFVNASR |
| SerpinG1_[242-249] | TLYSSSPR |
| SerpinG1_[250-268] | VLSNNSDANLELINTWVAK |
| SerpinG1_[269-273] | NTNNK |
| SerpinG1_[274-276] | ISR |
| SerpinG1_[277-286] | LLDSLPSDTR |
| SerpinG1_[301-306] | TTFDPK |
| SerpinG1_[310-316] | MEPFHFK |
| SerpinG1_[322-328] | VPMMNSK |
| SerpinG1_[330-341] | YPVAHFIDQTLK |
| SerpinG1_[344-364] | VGQLQLSHNLSLVILVPQNLK |
| SerpinG1_[367-380] | LEDMEQALSPSVFK |
| SerpinG1_[381-385] | AIMEK |
| SerpinG1_[386-390] | LEMSK |
| SerpinG1_[391-400] | FQPTLLTLPR |
| SerpinG1_[403-415] | VTTSQDMLSIMEK |
| SerpinG1_[41-44] | GEGK |
| SerpinG1_[416-466] | LEFFDFSYDLNLCGLTEDPDLQVSAMQHQTVLELTETGVEAAAASAISVAR |
| SerpinG1_[467-487] | TLLVFEVQQPFLFVLWDQQHK |
| SerpinG1_[488-494] | FPVFMGR |
| SerpinG1_[495-499] | VYDPR |
| SerpinG1_[53-77] | MLFVEPILEVSSLPTTNSTTNSATK |

15. The method of claim 14, wherein the C1-INH fragment peptide is selected from the group consisting of SerpinG1 [242-249] and SerpinG1 [391-400], preferably the C1-INH fragment is SerpinG1 [242-249].

16. The method of any one of claims 2 to 15, wherein the C1q fragment peptide is selected from the group consisting of
| **Peptide** | **Sequence (N-terminus -> C-terminus)** |
|---|---|
| C1q-A_[104-110] | GSPGNIK |
| C1q-A_[111-121] | DQPRP AFSAIR |
| C1q-A_[123-150] | NPPMGGNVVIFDTVITNQEEPYQNHSGR |
| C1q-A_[151-180] | FVCTVPGYYYFTFQVLSQWEICLSIVSSSR |
| C1q-A_[151-180_Cys_CAM: 153, 172 | FVCTVPGYYYFTFQVLSQWEICLSIVSSSR |
| C1q-A_[186-195] | SLGFCDTTNK |
| C1q-A_[186-195]_Cys_CAM: 190 | SLGFCDTTNK |
| C1q-A_[196-219] | GLFQVVSGGMVLQLQQGDQVWVEK |
| C1q-A_[224-245] | GHIYQGSEADSVFSGFLIFPSA |
| C1q-A_[23-27] | EDLCR |
| C1q-A_[28-32] | APDGK |
| C1q-A_[34-41] | GEAGRPGR |
| C1q-A_[49-60] | GEQGEPGAPGIR |
| C1q-A_[82-94] | VGYPGPSGPLGAR |
| C1q-B_[118-121] | ATQK |
| C1q-B_[137-141] | DQTIR |
| C1q-B_[160-163] | FTCK |
| C1q-B_[164-177] | VPGLYYFTYHASSR |
| C1q-B_[178-186] | GNLCVNLMR |
| C1q-B_[178-186]_Cys_CAM: 181 | GNLCVNLMR |
| C1q-B_[194-215] | VVTFCDYAYNTFQVTTGGMVLK |
| C1q-B_[194-215] Cys CAM: 198 | VVTFCDYAYNTFQVTTGGMVLK |
| C1q-B_[216-229] | LEQGENVFLQATDK |
| C1q-B_[230-253] | NSLLGMEGANSIFSGFLLFPDMEA |
| C1q-B_[28-59] | QLSCTGPPAIPGIPGIPGTPGPDGQPGTPGIK |
| C1q_B_[28-59]_Cys_CAM: 31 | QLSCTGPPAIPGIPGIPGTPGPDGQPGTPGIK |
| C1q-B_[63-77] | GLPGLAGDHGEFGEK |
| C1q-B_[78-88] | GDPGIPGNPGK |
| C1q-B_[93-98] | GPMGPK |
| C1q-B_[99-110] | GGPGAPGAPGPK |
| C1q-C_[118-126] | FQSVFTVTR |
| C1q-C_[127-139] | QTHQPPAPNSLIR |
| C1q-C_[140-157] | FNAVLTNPQGDYDTSTGK |
| C1q-C _[162-184] | VPGLYYFVYHASHTANLCVLLYR |
| C1q-C_[162-1841 Cvs CAM: 179 | VPGLYYFVYHASHTANLCVLLYR |
| C1q-C_[189-198] | VVTFCGHTSK |
| C1q-C_[189-198]_Cys_CAM: 193 | VVTFCGHTSK |
| C1q-C_[199-210] | TNQVNSGGVLLR |
| C1q-C_[211-245] | LQVGEEVWLAVNDYYDMVGIQGSDSVFSGFLLFPD |
| C1q-C_[29-47] | NTGCYGIPGMPGLPGAPGK |
| C1q_C_[29-47]_Cys_CAM: 32 | NTGCYGIPGMPGLPGAPGK |
| C1q-C_[48-57] | DGYDGLPGPK |
| C1q-C_[58-69] | GEPGIP AIPGIR |
| C1q-C_[76-86] | GEPGLPGHPGK |
| C1q-C_[87-113] | NGPMGPPGMPGVPGPMGIPGEPGEEGR |

17. The method of claim 16, wherein the C1q fragment peptide is selected from the group consisting of C1qBeta[178-186] and ClqBeta[63-77], preferably the C1qfragment is C1qBeta[178-186].

18. The method of any one of claims 6 to 17, wherein
the cut-off value for C4 fragment peptide C4Beta[571-579] is 500 ng/ml;
the cut-off value for C4 fragment peptide C4Alpha[680-685] is 260 ng/ml;
the cut-off value for C4 fragment peptide C4Alpha[786-791] is 100 ng/ml;
the cut-off value for C4 fragment peptide C4Beta[294-297] is 201 ng/ml ; and
the cut-off value for C4 fragment peptide C4Gamma[1638-1641] is 920 ng/ml.

19. The method of any one of claims 7 to 18, wherein
the cut-off value for C1-INH fragment peptide SerpinG1[242-249] is 835 ng/ml, and
the cut-off value for C1-INH fragment peptide SerpinG1[391-400] is 392 ng/ml.

20. The method of any one of claims 8 to 19, wherein
the cut-off value for C1q fragment peptide C1qBeta[178-186] is 800 ng/ml, and
the cut-off value for C1q fragment peptide and C1qBeta[63-77] is 1690 ng/ml.

21. The method of any one of claims 1 to 20, wherein the sensitivity of the method is 100 % for the detection of subjects suffering from hereditary angioedema type I or hereditary angioedema type II.

22. The method of any one of claims 1 to 21, wherein mass spectrometry is selected from the group comprising SELDI MS, MALDI MS, ESI MS, DESI MS and ion mobility MS.

23. The method of any one of claims 1 to 22, wherein mass spectrometry uses an analyzer selected from the group comprising Triple Quad, ToF, QToF, ion trap, orbitrap, ion mobility and any combination thereof.

24. The method of any one of claims 1 to 23, wherein spectrometric analysis comprises or uses MS/MS, MRM, SRM or any combination thereof, preferably MS/MS, MS^{e}, MSⁿ, MS/MRM or MS/SRM.

25. A kit suitable for use in a method for differential diagnosis of hereditary angioedema, preferably a method for differential diagnosis of hereditary angioedema according to any one of claims 1 to 24, wherein the kit comprises at least one element selected from the group comprising an interaction partner of one biomarker, one biomarker, instructions of use for the kit, and one or more container, wherein the biomarker is selected from the group comprising C4 protein, a fragment peptide of C4 protein, C1-INH protein, a fragment peptide of C1-INH protein, C1q protein and a fragment peptide of C1q.
